(19) Europäisches Patentamt
European Patent Office
Office européen des brevets

(11) Publication number: **0 127 902 B1**

(12) # EUROPEAN PATENT SPECIFICATION

(45) Date of publication of patent specification: **16.10.91**

(51) Int. Cl.5: **C07D 263/20**, C07D 263/24, A61K 31/42

(21) Application number: **84106400.9**

(22) Date of filing: **05.06.84**

The file contains technical information submitted after the application was filed and not included in this specification

(54) **Aminomethyl oxooxazolidinyl benzene derivatives useful as antibacterial agents.**

(30) Priority: **07.06.83 US 501897**
**14.02.84 US 578332**

(43) Date of publication of application:
**12.12.84 Bulletin 84/50**

(45) Publication of the grant of the patent:
**16.10.91 Bulletin 91/42**

(84) Designated Contracting States:
**AT BE CH DE FR GB IT LI LU NL SE**

(56) References cited:
**EP-A- 0 050 827**
**EP-A- 0 081 200**
**FR-A- 2 500 450**
**US-A- 3 687 965**
**US-A- 4 287 351**

**Chem. Soc. Rev. (1979) 18, 563-580**

(73) Proprietor: **E.I. DU PONT DE NEMOURS AND COMPANY**
**1007 Market Street**
**Wilmington Delaware 19898(US)**

(72) Inventor: **Gregory, Walter Adelman**
**104 Rockingham Drive**
**Wilmington, DE 19803(US)**

(74) Representative: **von Kreisler, Alek et al**
**Patentanwälte Von Kreisler-Schönwald-Fues-Keller Selting-Werner**
**Deichmannhaus am Hauptbahnhof**
**W-5000 Köln 1(DE)**

**EP 0 127 902 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

This invention relates to novel aminomethyl oxooxazolidinyl benzene derivatives, including the sulfides, sulfoxides, sulfones and sulfonamides, to pharmaceutical compositions containing them, and to methods of using them to alleviate bacterial infections.

### Background of the Invention

At the present time, no existing antibacterial product provides all features deemed advantageous. There is continual development of resistance by bacterial strains. A reduction of allergic reactions and of irritation at the site of injection, and greater biological half-life (i.e., longer in vivo activity) are currently desirable features for antibacterial products.

U.S. Patent 4,128,654 discloses, among others, compounds of the formula:

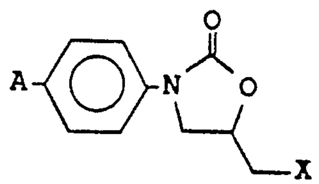

where
A = $RS(O)_n$;
X = Cl, Br or F;
R = $C_1$-$C_3$ alkyl; and
n = 0, 1 or 2.

The compounds are disclosed as being useful in controlling fungal and bacterial diseases of plants.

U.S. Reissue Patent 29,607 reissued April 11, 1978 discloses derivatives of 5-hydroxymethyl-3-substituted-2-oxazolidinones of the formula:

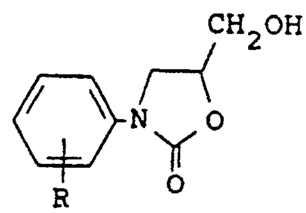

where R is H, F, $CH_3$, or $CF_3$. Such compounds are described as having antidepressive, tranquilizing, sedative, and antiinflammatory properties.

U.S. Patent 4,250,318 discloses antidepressant compounds of the formula:

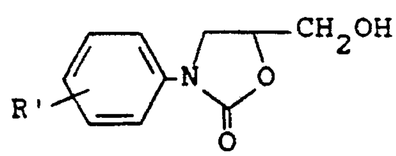

where R' can be, among others, a para-n-pentylamino group, an $SR_1$ group where $R_1$ is $C_1$-$C_5$ alkyl, or an acetylmethylthio group.

U.S. Patent 4,340,606, being equivalent to EP-A-0050 827 discloses antibacterial agents of the general formula:

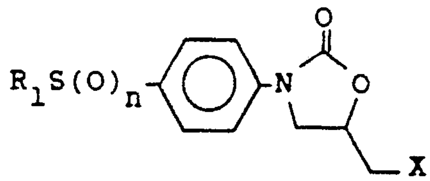

where

$R_1$ = $CH_3$, $C_2H_5$, $CF_2H$, $CF_3$ or $CF_2CF_2H$; and

X = $OR_2$ ($R_2$ = H or various acyl moieties).

U.S. Patent 3,687,965 discloses compounds of the formula:

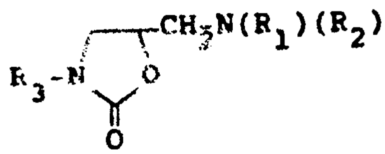

where

-N($R_1$)($R_2$) represents either dialkylamino radical in which the alkyl portions have one to five carbon atoms, or a heterocyclic amino radical which may be substituted by an alkyl radical having one to five carbon atoms or by a pyrrolidinocarbonylmethyl radical, and

$R_3$ represents a phenyl radical which may be substituted by one or more of the following radicals:

an alkoxy radical having one to five carbon atoms;

a halogen atom;

a trifluoromethyl radical, or

a carboxyl radical which may be esterified.

The patent states that these compounds possess hypotensive, vasodilatatory, spasmolytic, sedative, myorelaxant, analgesic and antiinflammatory properties. There is no mention of antibacterial properties.

Belgian Patent 892,270, published August 25, 1982, discloses monoamine oxidase inhibitors of the formula

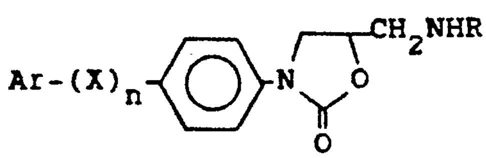

where

R is H, $C_1$-$C_4$ alkyl or propargyl;

Ar is phenyl, optionally substituted by halo or trifluoromethyl;

n is 0 or 1; and

X is $-CH_2CH_2-$, -CH=CH-, an acetylene group or $-CH_2O-$.

US-A-4 461 733 being equivalent to EP-A-0 081 200 discloses antibacterial agents of the formula

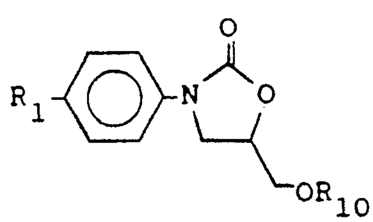

(I)

wherein, for the ℓ, and mixtures of the d and ℓ stereoisomersof the compound.

$R_1$ is $R_2SO_2$,

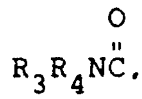, or 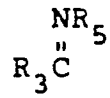 ;

$R_2$ is $-NR_3R_4$, $-N(OR_3)R_4$, $-N_3$, $-NHNH_2$, $-NX_2$, $-NR_6X$, $-NXZ$,

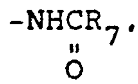, 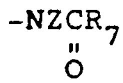

or $-N=S(O)_nR_8R_9$;

$R_3$ and $R_4$ are independently H, alkyl of 1-4 carbons or cycloalkyl of 3-8 carbons;

$R_5$ is $NR_3R_4$ or $OR_3$;

$R_6$ is alkyl of 1-4 carbons;

$R_7$ is alkyl of 1-4 carbons, optionally substituted with one or more halogens;

$R_8$ and $R_9$ are independently alkyl of 1-4 carbons or, taken together are $-(CH_2)_p$;

$R_{10}$ is H, alkyl of 1-3 carbons,

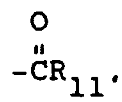,

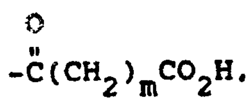, 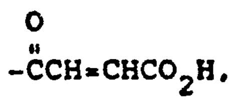, 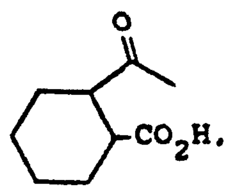,

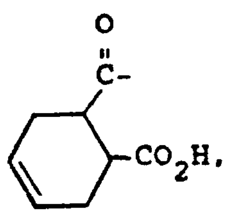, 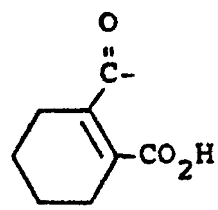 or 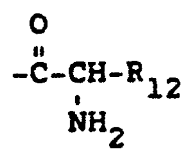 ;

$R_{11}$ is alkyl of 1-12 carbons;

$R_{12}$ is H, alkyl of 1-5 carbons, $CH_2OH$ or $CH_2SH$;

X is Cl, Br or I;

Z is a physiologically acceptable cation;

m is 2 or 3;

n is 0 or 1; and

p is 3, 4 or 5;

and when $R_{10}$ is alkyl of 1-3 carbons, $R_1$ can also be $CH_3S(O)_q$ where q is 0, 1 or 2; or a pharmaceutically acceptable salt thereof.

None of the cited references nor any known references suggest the novel antibacterial compounds of this invention.

## Summary of the Invention

The novel compounds of the instant invention possess useful antibacterial activity in both *in vitro* and *in vivo* tests. Specifically, one aspect of this invention relates to compounds having the formula:

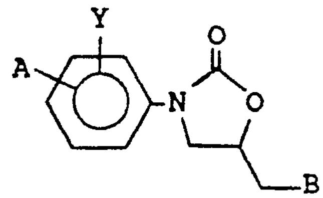

(I)

wherein, for the ℓ, and mixtures of the d and ℓ stereoisomers of the compound,

| | |
|---|---|
| A | is $-NO_2$, $-S(O)_nR_1$, -SH, |
| | 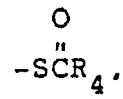 |
| | $-COR_5$, |
| | 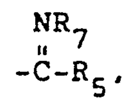 |
| | -CN, $-OR_5$, $-NR_5R_6$, |
| | 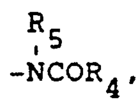, 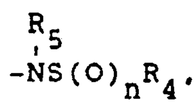 |
| | alkyl of 1 to 5 carbons, optionally substituted with one or more halogen atoms or alkenyl of 2-5 carbons; |
| $R_1$ | is $C_1$-$C_4$ alkyl, optionally substituted with one or more halogen atoms, CN; $-NR_9R_{10}$; $-N_3$; |
| $R_4$ | is alkyl of 1-4 carbons, |
| $R_5$ and $R_6$ | are independently H, alkyl of 1-4 carbons |
| $R_7$ | is $-OR_5$; |
| $R_8$ | is H or alkyl of 1-4 carbons; |
| $R_9$ | is H, $C_1$-$C_4$ alkyl or $C_3$-$C_8$ cycloalkyl; |
| $R_{10}$ | is H, $-OR_8$ or $-NR_{11}R_{11a}$ |
| $R_{11}$ and $R_{11a}$ | are independently H or $C_1$-$C_4$ alkyl; |
| Y | is H, or $NO_2$, or A and Y taken together can be $-O(CH_2)_tO-$; |
| n | is 0, 1 or 2; |
| t | is 1, 2 or 3; |
| B | is |
| | 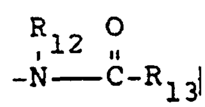 |
| | or $N_3$; |
| $R_{12}$ | is H, $C_1$-$C_{10}$ alkyl or $C_3$-$C_8$ cycloalkyl; |

| | |
|---|---|
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$; $-SR_{14}$; $-CH_2N_3$; $-NR_{19}R_{20}$; or $C(NH_2)R_{21}R_{22}$; |
| $R_{14}$ | is $C_1$-$C_4$ alkyl, optionally substituted with one or more halogen atoms; |
| $R_{15}$ | is $C_1$-$C_4$ alkyl; |
| $R_{16}$ and $R_{17}$ | are independently $C_1$-$C_4$ alkyl; |
| $R_{18}$ | is $C_1$-$C_4$ alkyl; |
| $R_{19}$ and $R_{20}$ | are independently H or $C_1$-$C_4$ alkyl; |
| $R_{21}$ and $R_{22}$ | are independently H, phenyl; |
| u | is 1 or 2; |
| v | is 0, 1 or 2; and |

or a pharmaceutically suitable salt thereof; provided that:

1) when A is $CH_3S$-, then B is not

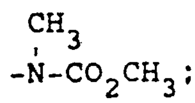

2) when A is $CH_3SO_2$-, then B is not

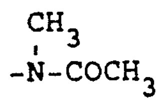 or 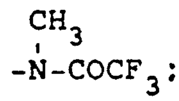

3) when A is $H_2NSO_2$- and B is

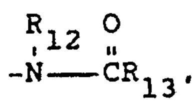

then $R_{12}$ is H;

4) when A is -CN, B is not $-N_3$;

5) when A is $(CH_3)_2CH$, B is not $NHCOCH_2Cl$.

Preferred, for their high antibacterial activity or ease of synthesis, or both, are compounds of formula I where:

(1)

| | |
|---|---|
| Y | is H; |
| A, | substituted in the para position, is $-S(O)_nR_1$, $NO_2$, 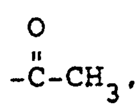 or $-CH(CH_3)_2$; |
| $R_1$ | is $C_1$-$C_2$ alkyl optionally substituted with one or more halogen atoms or $-NR_9R_{10}$; |
| n | is 0, 1 or 2 when $R_1$ is alkyl or substituted alkyl; or |

(2)

B is

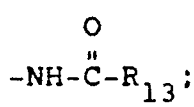

$R_{13}$ is H, $CH_3$, $OR_{18}$, $CHCl_2$, $CH_2Cl$ or $CH_2OR_{15}$;
$R_{15}$ is $C_1$-$C_4$ alkyl; and
$R_{18}$ is $C_1$-$C_4$ alkyl.

Preferred because of high antibacterial activity are compounds of formula I having the absolute configuration depicted:

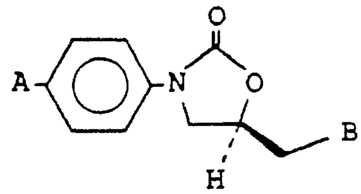

More preferred because of high antibacterial activity are compounds of formula I having the absolute configuration depicted:

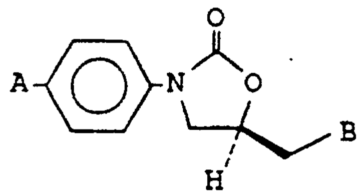

and where A is $S(O)CH_3$, $SCH_3$, $S(O)_2CH_3$, $SO_2NH_2$, $COCH_3$ or $CH(CH_3)_2$; and
where B is $-NHCOCH_3$, $-NHCO_2CH_3$ or $-NHCOCHCl_2$.

Specifically preferred for their high antibacterial activity are the following compounds:

- (ℓ)-N-[3-[4-(methylsulfonyl)phenyl]-2-oxooxazolidin-5-ylmethyl]carbamic acid, methyl ester;
- (ℓ)-N-[3-[4-(methylthio)phenyl]-2-oxooxazolidin-5-ylmethyl]carbamic acid, methyl ester;
- (ℓ)-N-[3-[4-(methylsulfonyl)phenyl]-2-oxooxazolidin-5-ylmethyl]formamide;
- (ℓ)-N-[3-[4-(methylsulfonyl)phenyl]-2-oxooxazolidin-5-ylmethyl]acetamide;
- (ℓ)-N-[3-[4-(methylthio)phenyl]-2-oxooxazolidin-5-ylmethyl]acetamide;
- (ℓ)-N-[3-[4-(aminosulfonyl)phenyl]-2-oxooxazolidin-5-ylmethyl]acetamide;
- (ℓ)-N-[3-[4-(methylsulfinyl)phenyl]-2-oxooxazolidin-5-ylmethyl]acetamide;
- (ℓ)-2,2-dichloro-N-[3-[4-(methylsulfonyl)phenyl]-2-oxooxazolidin-5-ylmethyl]acetamide;
- (ℓ)-N-[3-(4-isopropylphenyl)-2-oxooxazolidin-5-ylmethyl]acetamide; and
- (ℓ)-N-[3-(4-acetylphenyl)-2-oxooxazolidin-5-ylmethyl]acetamide;

Another aspect of this invention relates to novel intermediates having the formula:

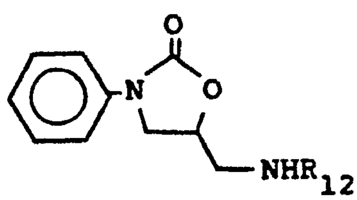

**(Ia)**

wherein, for the ℓ, and mixtures of the d and ℓ stereoisomers of the compound,
$R_{12}$ is H, $C_1$-$C_{10}$ alkyl or $C_3$-$C_8$ cycloalkyl.

Another aspect of this invention relates to novel intermediates having the formula:

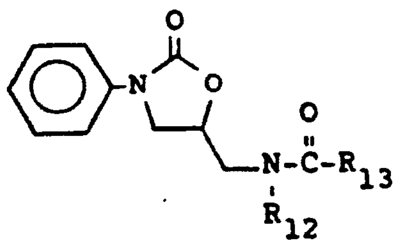

(Ib)

wherein, for the ℓ, and mixtures of the d and ℓ stereoisomers of the compound,

| | |
|---|---|
| $R_{12}$ | is H, $C_1$-$C_{10}$ alkyl or $C_3$-$C_8$ cycloalkyl; |
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$; $-SR_{14}$; $-NR_{19}R_{20}$; or $C(NH_2)R_{21}R_{22}$; |
| $R_{14}$ | is $C_1$-$C_4$ alkyl, optionally substituted with one or more halogen atoms; |
| $R_{15}$ | is $C_1$-$C_4$ alkyl; |
| $R_{16}$ and $R_{17}$ | are independently $C_1$-$C_4$ alkyl; |
| $R_{18}$ | is $C_1$-$C_4$ alkyl; |
| $R_{19}$ and $R_{20}$ | are independently H or $C_1$-$C_4$ alkyl; |
| $R_{21}$ and $R_{22}$ | are independently H, phenyl; and |
| v | is 0, 1 or 2. |

Another aspect of this invention relates to a pharmaceutical composition comprising a suitable pharmaceutical carrier and an antibacterially effective amount of a compound of formula I. Yet another aspect of the invention relates to a method for alleviating bacterial infection in a mammal which comprises administering to the mammal an antibacterially effective amount of a compound of formula I.

## Detailed Description

The compounds of formulae I, Ia, and Ib contain at least one chiral center, and as such exist as two individual isomers or as a mixture of both. This invention relates to the levorotatory isomer (ℓ), as well as mixtures containing both the d and the ℓ isomers. An additional chiral center is present when A is $R_1S(O)_n$ and n is 1 and this invention relates to both of the possible isomers at that center. Additional chiral centers may be present in the group B and this invention relates to all possible stereoisomers in the group B.

For the purposes of this invention, the ℓ-isomer of compounds of formulae I, Ia, and Ib is intended to mean compounds of the configuration depicted:

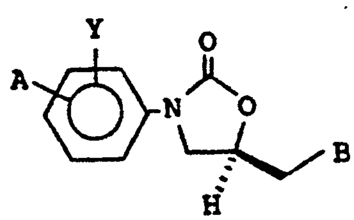

## Synthesis

Compounds of Formula (I) can be prepared as follows:

Scheme 1:

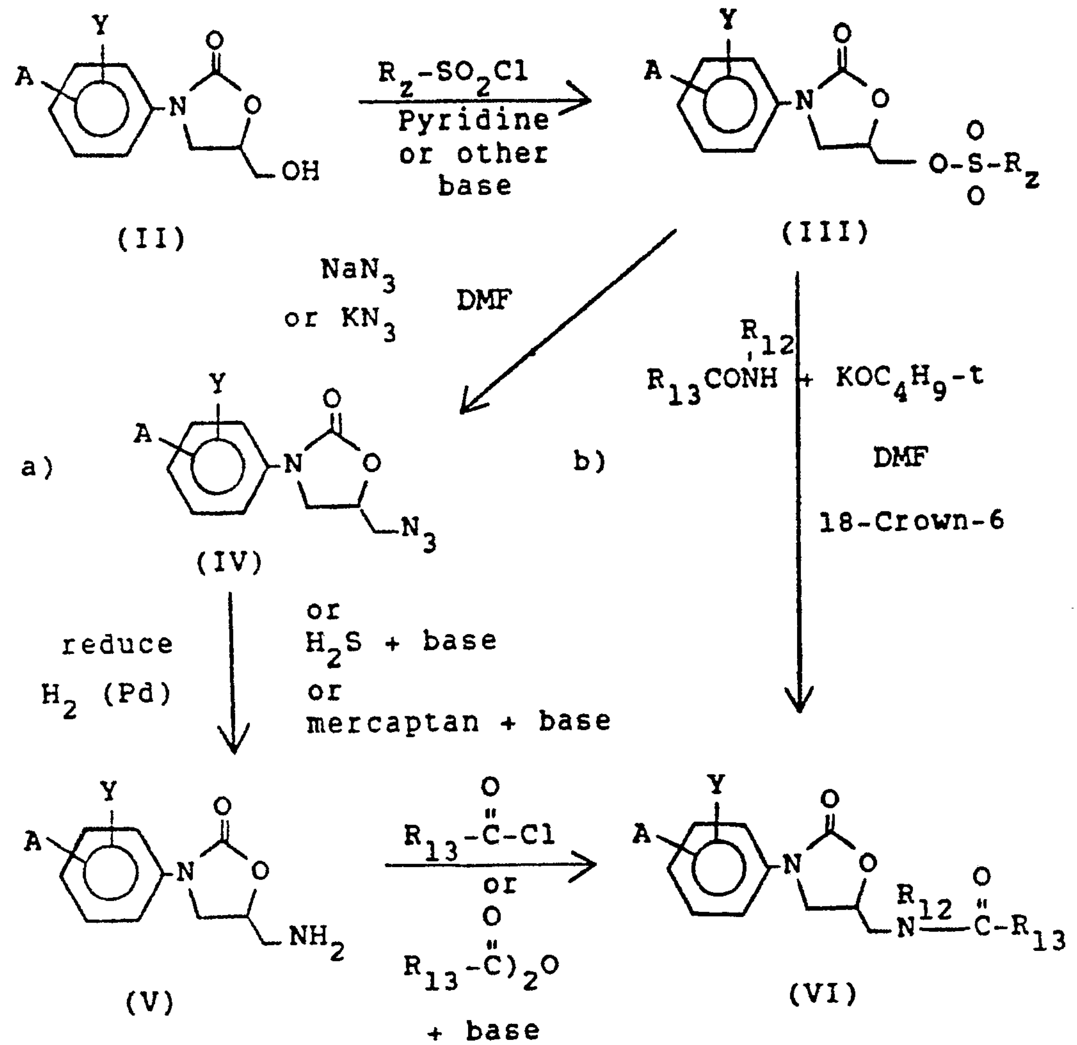


Where $R_2$ may be 4-tolyl, phenyl, 4-chlorophenyl, $C_1$-$C_4$ alkyl or haloalkyl, such as trifluoromethyl.

When the synthetic path a) is used, the group A may be -H or any of the groups previously shown except where $R_1$ is $-N_3$, $-NX_2$, $-NR_9X$, $-^{-}NXZ^{+}$. When the synthetic path b) is used the group A may be -H or any of the groups previously shown except when A is $R_1S(O)_n$ and $R_1$ is $NR_9R_{10}$, $R_9$, $R_{10}$, $R_{11}$, and $R_{11a}$cannot be H.

Compounds of Formula (II) may be converted to sulfonate esters (III) by reaction with the appropriate sulfonyl halide or sulfonic anhydride in a solvent plus a base or in a basic organic solvent such as pyridine. It is desirable when the A group has a sulfonamide hydrogen to use pyridine or other mildly basic solvents such as the picolines or collidines. As solvents, 1,2-dimethoxyethane, dioxane, bis-(2-methoxyethyl)ether, N,N-dimethylformamide (DMF), N,N-dimethylacetamide (DMAc), acetonitrile, or tetramethylenesulfone may be used. As a base, triethylamine, N-methylmorpholine, tributylamine or one of the heterocyclic bases can be used.

Compounds (III) may be reacted with sodium, potassium, lithium, cesium or rubidium azides in a dipolar aprotic solvent such as DMF, N-methylpyrrolidone, DMAc, sulfolane, dimethylsulfoxide, tetramethylurea, hexamethylphosphoramide (HMPA), etc. along with the appropriate catalyst such as 18-crown-6 for sodium and potassium azide and 12-crown-4 for lithium azide. This reaction is carried out from about 60° to 125° C, with the preferred temperatures being 70° to 90° C. The products are azides of structure (IV).

The azides (IV) may be reduced by any of several methods, including hydrogenation over palladium-on-charcoal. It is also possible to reduce the azides by treating with 1,3-propanedithiol and a base such as triethylamine. Azides may also be reduced to amines by hydrogen sulfide and by trivalent phosphorous compounds such as trimethylphosphine and trimethylphosphite, and by mercaptans such as mercaptoacetic acid. Reduction with hydrogen can best be used where A is hydrogen, but it will work where A is a

hexavalent sulfur containing group. The reduction is carried out using a solvent such as ethanol, methanol, 1,2-dimethoxyethane, acetic acid, trifluoroacetic acid, or isopropanol. A solution may be stirred at ambient temperature with palladium-on-charcoal catalyst present and the hydrogen introduced at atmospheric pressure through a glass frit. In some instances the reduction is exothermic.

The reduction using 1,3-propanedithiol is carried out in methanol or other alcohol solvents containing an equivalent of triethylamine, by warming until $N_2$ evolution occurs. At ambient temperatures, slow reduction occurs. Temperatures of 20° to 100° C may be used; temperatures of 40° to 60° C are preferred. Warming an azide (IV) with trimethylphosphite causes a rapid evolution of $N_2$. The reaction may be carried out in 1,2-dimethoxyethane or bis-(2-methoxyethyl)ether and the crude intermediate, when hydrolyzed with water or acid, gives the desired amine (V).

The aminomethyl compounds (V) are acylated by reaction of the amine with an acid chloride or anhydride in a basic solvent such as pyridine or by reaction in a water miscible solvent such as THF or 1,2-dimethoxyethane in the presence of an aqueous base such as sodium hydroxide or potassium hydroxide, sodium bicarbonate or sodium carbonate. When pyridine is used as solvent for the reaction, the acid chloride or anhydride is added to the mixture at 0° to 10° C. The reaction may be carried out between -30° and 50° C. With very reactive acid chlorides or anhydrides such as trifluoromethanesulfonyl chloride or anhydride the reaction is preferably carried out at -60° to -40° C. The acylations using aqueous bases are done by stirring the amine (V) in a water miscible solvent such as tetrahydrofuran (THF), 1,2-dimethoxyethane, or dioxane and adding 1-5 N NaOH to keep the mixture basic as the acid chloride or anhydride is added, while keeping the temperature between -5° and 20° C. The compounds (V) can also be acylated by any of the standard peptide synthesis methods where the free acid is reacted with the amine using N,N-dicyclohexylcarbodiimide, or where a mixed anhydride is first formed from the acid using a chloroformate ester and a tertiary base such as triethylamine, followed by reaction with the amine. In the mixed anhydride procedure, the acid to be used is allowed to react with a chloroformate such as ethyl chloroformate or isobutyl chloroformate in a solvent such as THF, DMF or 1,2-dimethoxyethane, in the presence of a tertiary base such as triethylamine or N-methylmorpholine at -30° to 10° C. To this mixture the amine (V) is added and the mixture stirred at -10° C for 1-5 hours. When N,N-dicyclohexylcarbodiimide is used as the condensing agent, the conditions and solvents may be the same but it is often advantageous to add N-hydroxyphthalimide or N-hydroxysuccinimide.

Further, these amines may be acylated by reaction with esters such as methyl dichloroacetate, ethyl trifluoroacetate or n-butyl formate. In this method, the amine (V) is combined with the ester and a solvent such as 1,2-dimethoxyethane, bis-(2-methoxyethyl)ether, or toluene (in some cases the ester may be used as the solvent) and the mixture is heated at reflux until the reaction is shown to be complete by an assay such as thin-layer chromatography. More reactive esters such as p-nitrophenyl esters, pentafluorophenyl esters, thio esters, enol esters, N-hydroxyphthalimide esters, N-hydroxysuccinimide esters, 1-hydroxybenzotriazole esters, 2,4,5-trichlorophenyl esters, and pentachlorophenyl esters, may be used. Further, other acylating agents such as acyl azides, acyl imidazoles and acyl phosphates, may be used.

When synthetic path b) is used, the sulfonate ester (III) is allowed to react with an amide in the form of its sodium or potassium salt, generated using NaH, KH or $KOC_4H_9$-t in a dipolar aprotic solvent such as DMF, DMAc, HMPA, N-methylpyrrolidinone, or tetramethylenesulfone. To the salt preparation is added the sulfonate ester (III) and the mixture is heated to 30° to 150° C. A catalyst such as 18-crown-6 may be used. Heating is continued for 3-50 hours.

In Scheme 1, the starting compound (II) may be dℓ- (the racemate) or the ℓ-isomer. The ℓ-isomer is a precursor for the preferred ℓ-amides (VI).

When the acylating group is derived from an α-amino acid and $R_{13}$ contains an amino function it is necessary to protect that amino function with one of the commonly used protective groups such as benzyloxycarbonyl, t-butyloxycarbonyl, 9-fluorenylmethyloxycarbonyl, or phthaloyl. Following the acylation, the protective group is removed by one of the standard methods to which the oxazolidinone ring is inert. The benzyloxycarbonyl group may be removed by hydrogenation in a solvent such as methanol, DMF, acetic acid, or mixtures of these solvents, using a catalyst such as 10% palladium-on-carbon or palladium black (100 to 500 mg of catalyst per mmole of compound). Alternatively the benzyloxycarbonyl group may be removed by dissolving the compound in acetic acid, adding an equal volume of 4 N HBr in acetic acid, and keeping the solution at room temperature for 1 to 5 hours. The $N^{\alpha}$-t-butyloxycarbonyl groups are removed by hydrolysis with trifluoroacetic acid at room temperature.

Scheme 2:

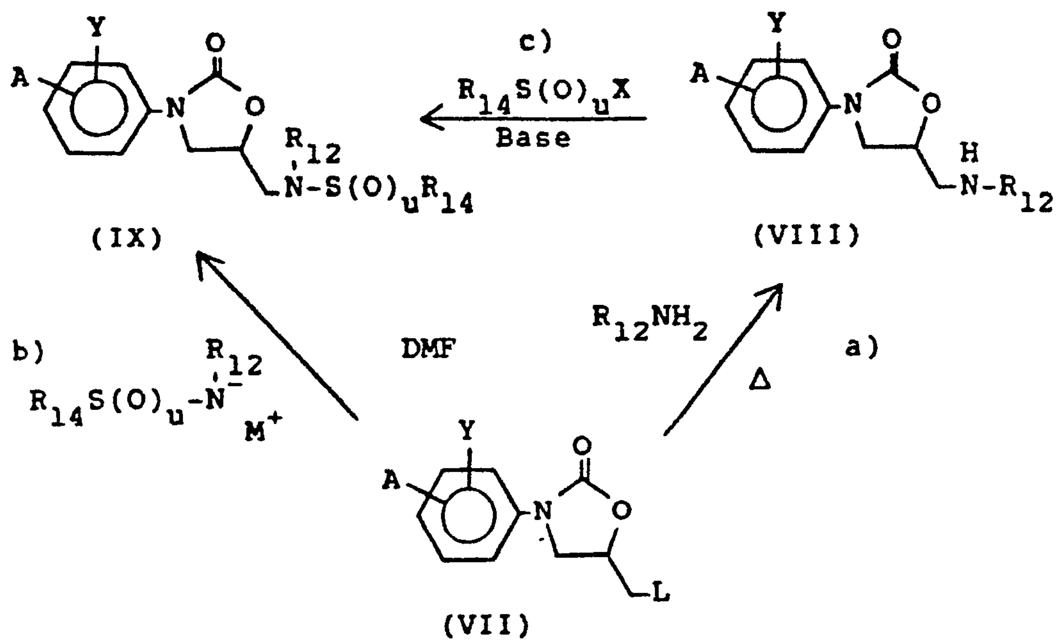


Compounds of formula (I) which may be made using the procedures of Scheme 2 are those where A is H or any of the groups previously shown except that when A is $R_1S(O)_n$ and $R_1$ is $NR_9R_{10}$, $R_9$, $R_{10}$, $R_{11}$ and $R_{11a}$ cannot be H. L may be any suitable leaving group such as I, Br, Cl, benzenesulfonyloxy, 4-toluenesulfonyloxy, methanesulfonyloxy or trifluoromethanesulfonyloxy. In route a) the compound (VII) is allowed to react with ammonia or an amine in a solvent such as ethanol at temperatures of 50° to 150° C. Where the amine or solvent is low-boiling, the reaction is carried out in a sealed vessel to allow the desired temperature to be reached. The solvent may be ethanol, DMF, DMAc, N-methylpyrrolidinone, tetramethylenesulfone, or HMPA. The reaction time may be 1 to 24 hours. Where (VII) is optically active (i.e., the ℓ-isomer) the product is optically active. The acylation of product VIII is carried out as described for Scheme 1, Path a).

The reaction of (VII) with the anion of a sulfonamide shown in Scheme 2. Path b) is carried out in a polar solvent such as DMF, DMAc, N-methylpyrrolidinone, tetramethylenesulfone, or HMPA. In some cases the use of a catalyst such as 18-crown-6 may improve the reaction. Temperatures of 50° to 150° C are employed; the time for the reaction can vary between 2 to 48 hours.

Alternatively, the sulfonamides (IX) can be prepared by reaction of the amine (VIII) with a sulfonyl halide in the presence of a base such as triethylamine or a basic solvent such as pyridine [Path c)].

Scheme 3:

a) 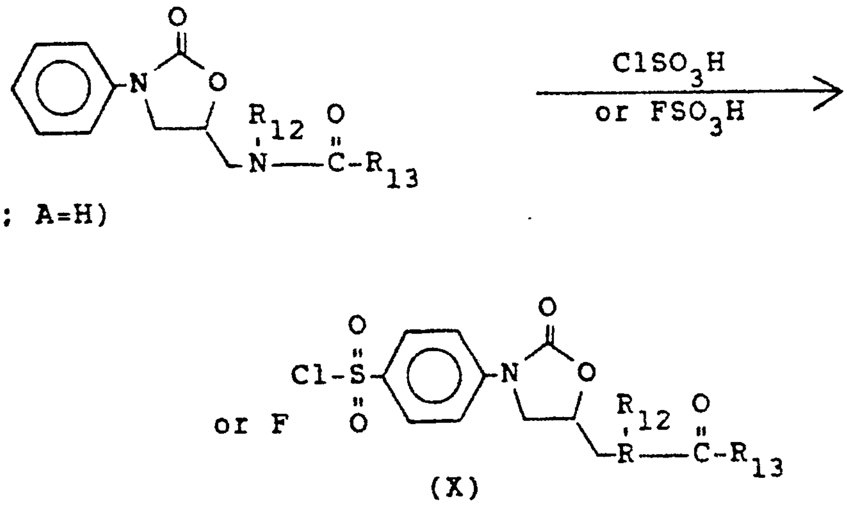


(VI; A=H)

(X)

b) (X) 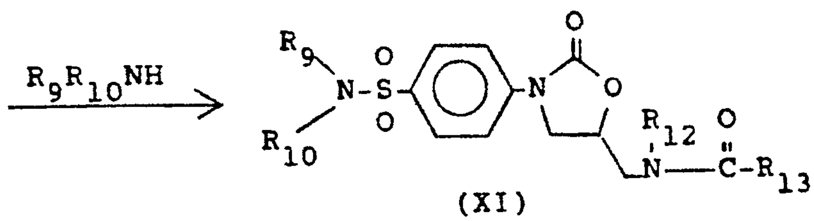


(XI)

c) (X) 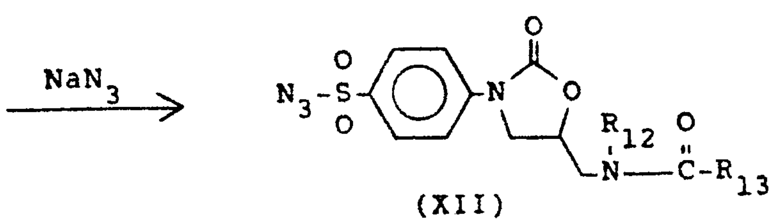


(XII)

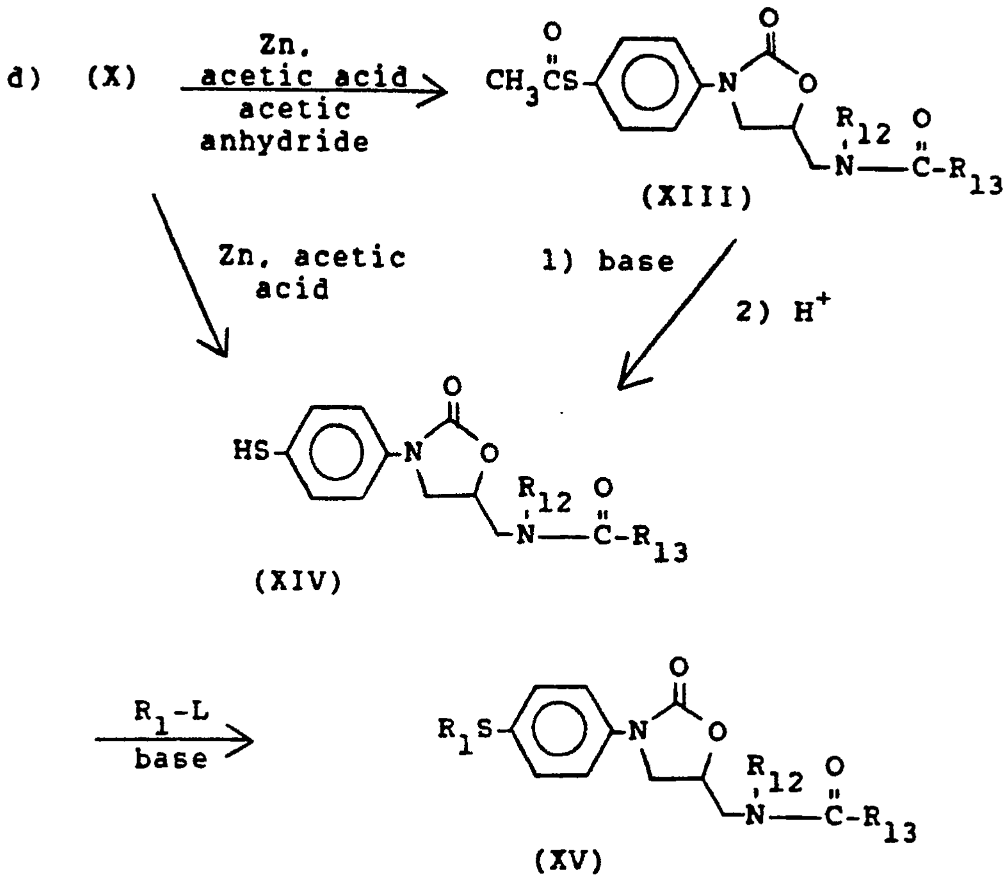


(XIII)

(XIV)

(XV)

Compounds of Formula I, where B is

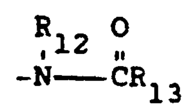

wherein $R_{13}$ is not $CH(OR_{16})OR_{17}$ or $CH_2N_3$ can be prepared as shown in Scheme 3. The halosulfonation (particularly, chlorosulfonation) shown in Scheme 3. Path a), can be carried out by adding the compound of formula VI where A is H to chlorosulfonic acid or fluorosulfonic acid at room temperature in the absence of solvent. The temperature may be 10° to 100° C; preferred temperatures are 15° to 35° C. A solvent inert to chlorosulfonic acid or fluorosulfonic acid may be employed (examples include carbon tetrachloride, nitrobenzene, or a fluorocarbon) but using neat chlorosulfonic acid or fluorosulfonic acid is preferred.

The sulfonyl chloride or fluoride (X) may then be reacted by the procedure of Scheme 3, Path b), with ammonia, a mono- or disubstituted amine, a hydroxylamine or a hydrazine in a solvent such as THF, 1,2-dimethoxyethane, dioxane, bis-(2-methoxyethyl)ether or DMF. The reaction may be run at temperatures of -20° to 40° C; temperatures of -10° to 10° C are preferred.

The sulfonyl chloride or fluoride (X), may be reacted with sodium azide or potassium aside in a mixture of acetone and water to give the sulfonyl azide (XII) as shown in Scheme 3, Path c). Other water-miscible solvents such as acetonitrile, DMF, 1,2-dimethoxyethane, THF, or dimethylsulfoxide may be used in place of acetone. An aqueous solution of sodium azide is added to acetone, the mixture is cooled in an ice-bath, the sulfonyl halide (X) is added, and the mixture is allowed to come to room temperature. The reaction may be carried out at -10° to 20° C. Preferred temperatures are -5° to 10° C.

The sulfonyl chlorides (X) may be reduced by several methods, as shown in Scheme 3, path d). The use of zinc metal added to a hot mixture of acetic acid, acetic anhydride and sodium acetate gives the S-acetates (XIII) in good yield. This is carried out at reflux temperature of the mixture, but may be carried out between 50° C to 120° C. Alternatively, the sulfonyl halides may be reduced by using zinc in acetic acid to give the mercaptans (XIV). The reduction may also be carried out using an iodide such as trimethylsilyl iodide or mixtures of trimethylsilyl chloride and sodium iodide in an inert solvent such as dichloromethane,

benzene or toluene; stirring in the temperature range of 0° C to 50° C with the preferred temperature 20-30° C. This reduction gives the disulfide which is then reduced by sodium borohydride in an alcohol solvent such as methanol. The disulfide may also be reduced by dithiothreitol or by zinc and acid. The product is the mercaptans (XIV). If desired the mercaptans may be alkylated with the halides $R_1$-L to give the sulfides (XV). This reaction may be carried out using base such as potassium carbonate, sodium methoxide, sodium ethoxide or potassium t-butoxide. The alkylation can be done using sodium hydroxide in dimethylsulfoxide.

The reactions of Scheme 3 may be carried out starting with the ℓ-isomer of (VI) where A = H to give products of the preferred ℓ-form (the preferred configuration shown above).

Scheme 4:

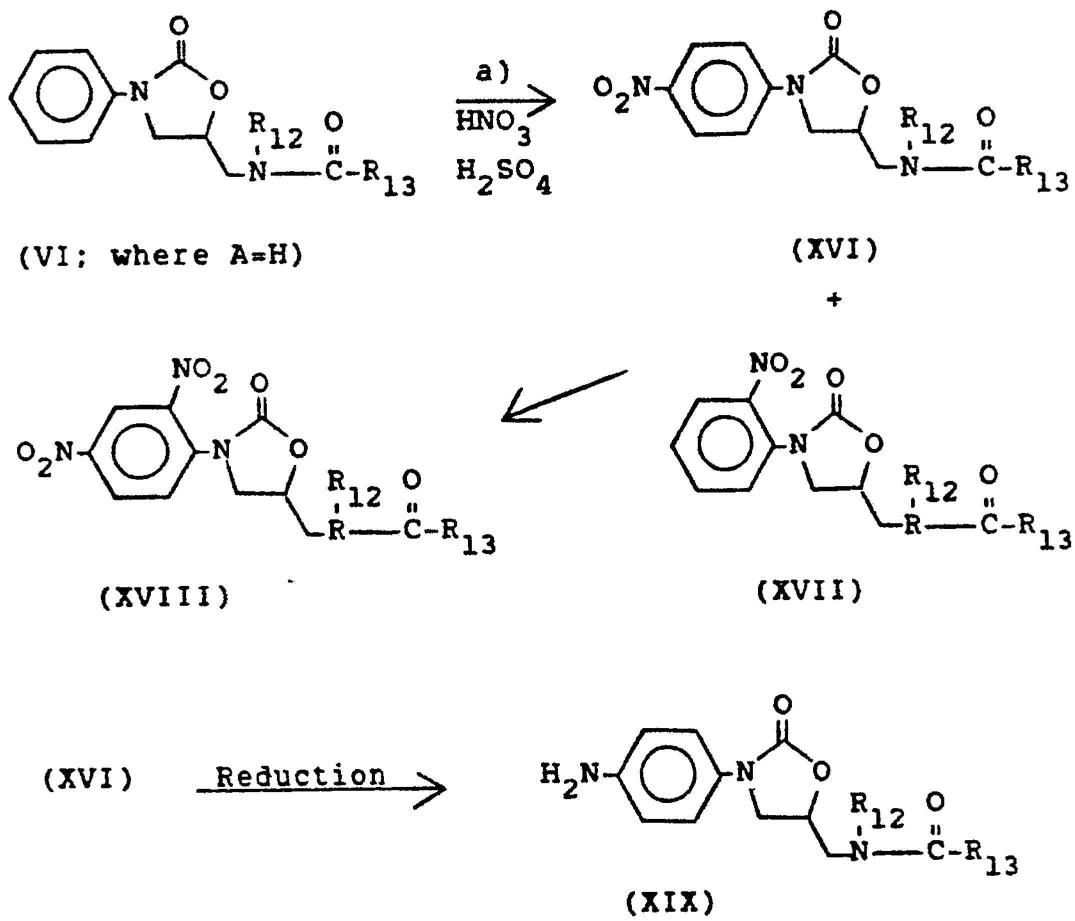


(VI; where A=H)

(XVI)

+

(XVIII)

(XVII)

(XVI)

(XIX)

(XIX)

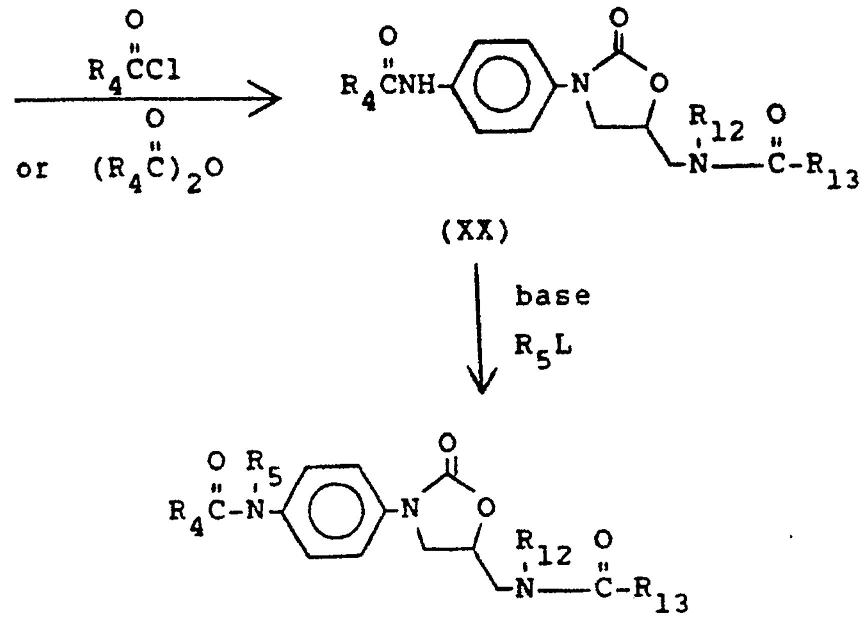


(XX)

(XXI)

(XIX)

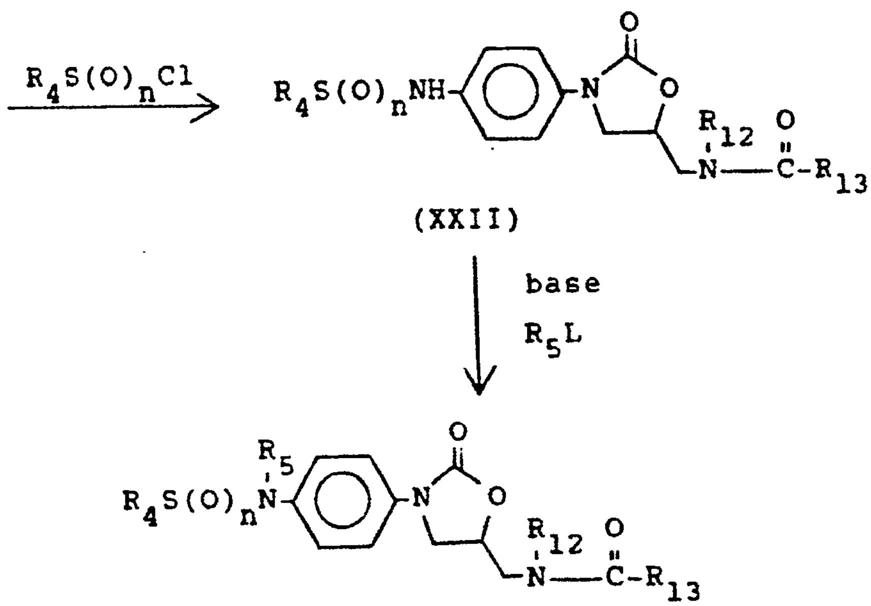


(XXII)

(XXIII)

The nitration of Scheme 4, Path a) is carried out by adding the compound of formula (VI) (A=H) to concentrated sulfuric acid containing one equivalent of nitric acid. Nitrate may be added in the form of a salt such as potassium nitrate. The nitration mixture is cooled to about -5°C, kept below 0°C during the addition, and then allowed to warm to room temperature. The nitration may be carried out at temperatures of -20° to 15°C, over time periods of 30 to 180 minutes.

In the nitration shown in Scheme 4 it has been found that some ortho nitration occurs as well as the formation of 2,4-dinitro-compound. These products may be isolated by use of preparative chromography, and/or crystallization. The ortho nitro compound may be made in higher amounts by nitration in acetic acid by generating acetyl nitrate. The dinitro-compound can easily be made by using a higher molar ratio of nitrating agent.

The nitro-compounds (XVI, XVII, XVIII) can be reduced by using Raney nickel catalyst and hydrazine or by catalytic hydrogenation in a Parr shaker under 10-50 lbs. of hydrogen using palladium-on-charcoal as the

catalyst. The products are the anilines (XIX). The anilines (XIX) may be acylated using an acyl halide or an acyl anhydride in the presence of an organic base such as pyridine or triethylamine or N-methylmorpholine; or using aqueous sodium hydroxide in an organic solvent such as tetrahydrofuran, 1,2-dimethoxyethane or DMF. A catalyst such as 4-dimethylaminopyridine may be used. In a similar way the anilines may be reacted with a sulfonyl halide to give the sulfonamides. In turn, the amides (XX) and sulfonamides (XXII) may be alkylated using base and the appropriate alkyl halide, alkyl sulfonate or sulfate ester.

Compounds where $R_1$ is $-NX_2$, $-NR_4X$, $-NXZ$ or $-N=S(O)_pR_2R_3$ may be made as shown in Scheme 5.

Scheme 5:

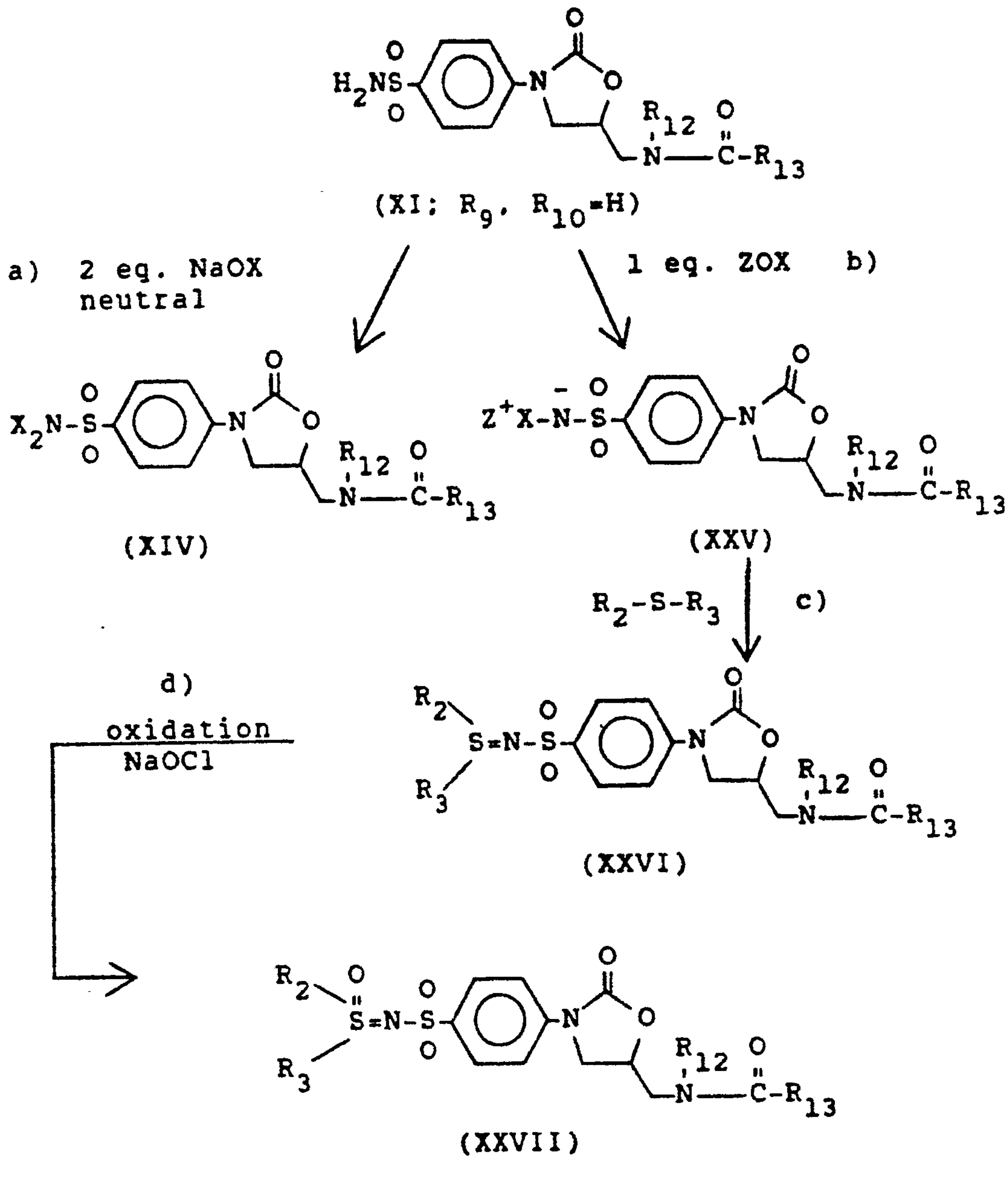


(XI; $R_9$, $R_{10}$=H)

(XIV)

(XXV)

(XXVI)

(XXVII)

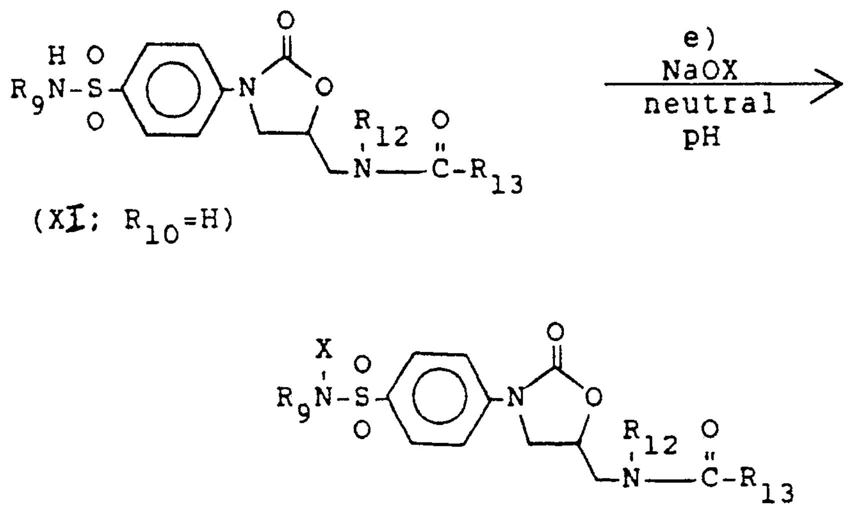


(XI; $R_{10}$=H)

(XXVIII)

Part a) of Scheme 5 is carried out by adding the sulfonamide (XI; $R_9$, $R_{10}$ = H) to 1,3-2 N sodium or other hypohalite (2 equivalents) while keeping the pH between 6 and 7 by adding a dilute acid solution or acetic acid. This reaction may be carried out at -20° to 50° C; it goes well at room temperatures of 20° to 30° C. The reaction is complete in 30 minutes to 2 hours. To make the metal salts of the haloamide (XXV), Scheme 5, Path b), one keeps the solution basic and uses approximately an equivalent amount of the hypohalite.

The sulfilimines (XXVI) are made by reacting the haloamide (XXV) with the appropriate sulfide in an alcohol-water mixture at 50° to 70° C. These products may be converted to the sulfoximines by oxidation using an oxidant such as hypochlorite anion in a phase transfer catalyzed system. This oxidation is carried out by stirring (XXVI) in a mixed solvent (ethyl acetate and dichloromethane) with tetra-n-butylammonium bromide while a two-fold excess of aqueous NaOCl are added at room temperature.

The preparation of N-alkyl haloamides (XXVIII) (Scheme 5, step e)) is carried out using the procedure of Scheme 5, Path a), except employing one equivalent of hypohalite.

An alternative synthesis of the glycinamides of Formula I where B is

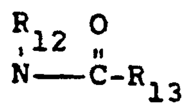

wherein $R_{13}$ is $CH_2NH_2$ as well as compounds where $R_{13}$ is $CH_2N_3$ is shown in Scheme 6.

Scheme 6:

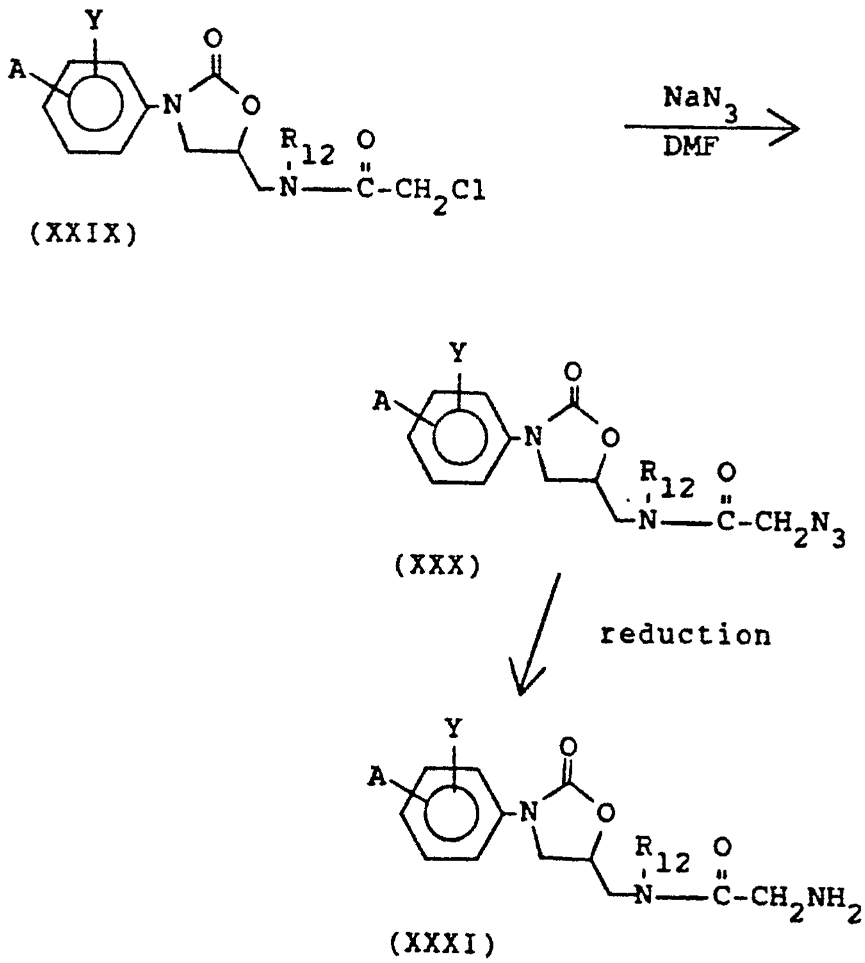


(XXIX)

(XXX)

(XXXI)

Glycine amides (XXXI) may be prepared by making the chloroacetyl or bromoacetyl or iodoacetyl compounds (XXIX) followed by reacting these with sodium azide in dimethylsulfoxide or other dipolar aprotic solvents to give the azidoacetyl compounds (XXX). The azidoacetyl compounds then may be reduced by hydrogen using a palladium catalyst or by any of the other reduction methods such as 1,3-propanedithiol and triethylamine, thioglycolic acid or hydrogen sulfide. The products are the glycine amides (XXXI).

The compounds of Formula I where A is

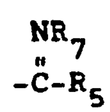

or

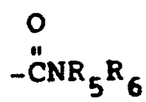

are obtained as shown in Scheme 7.

Scheme 7:

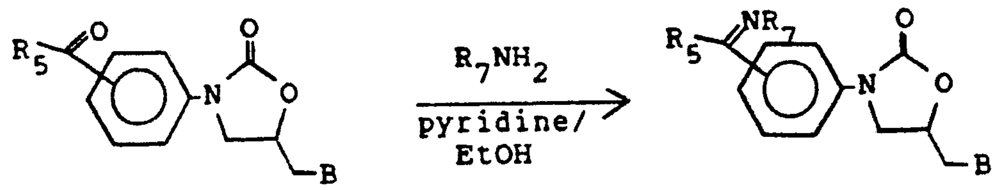


(XXXII) (XXXIII)

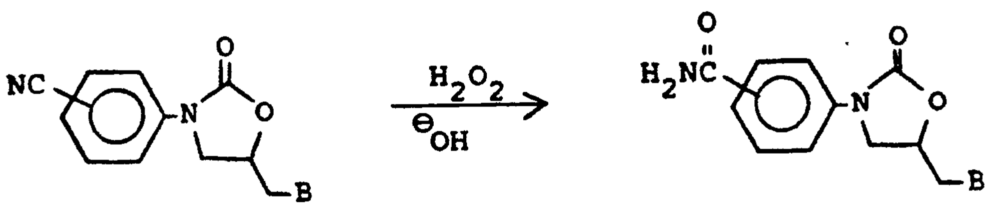


(XXXIV) (XXXV)

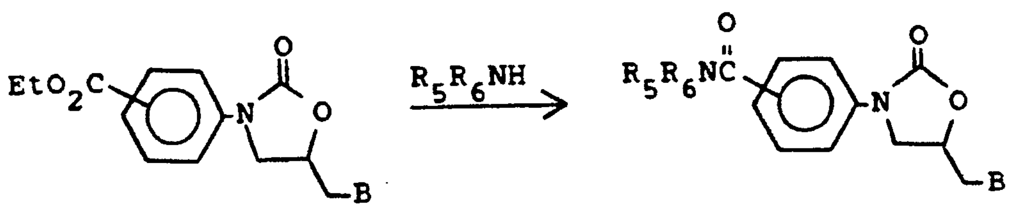


(XXXVI) (XXXVII)

Reaction of the ketones (XXXII) with a hydroxylamine or hydrazine gives the corresponding oxime or hydrazone derivative (XXXIII). The reaction is carried out in a solvent mixture of pyridine in ethanol at a temperature of 50° C to the reflux temperature of the solvent mixture.

The amides (XXXV) can be prepared by hydrolysis of the nitriles (XXXIV) with basic hydrogen peroxide. The reaction is conducted in aqueous alcoholic solvent at a temperature between 0 and 60° C. The substituted amides (XXXVII) can be prepared by aminolysis of the esters (XXXVI). For low boiling amines, the reaction can be oarried out under pressure. For higher boiling amines, a mixture of the amine and (XXXVI) is stirred optionally in an alcoholic or polar aprotic solvent at a temperature of 50 to 150° C.

An alternate synthesis of compounds of structure (V) is carried out as shown in Scheme 8.

Scheme 8:

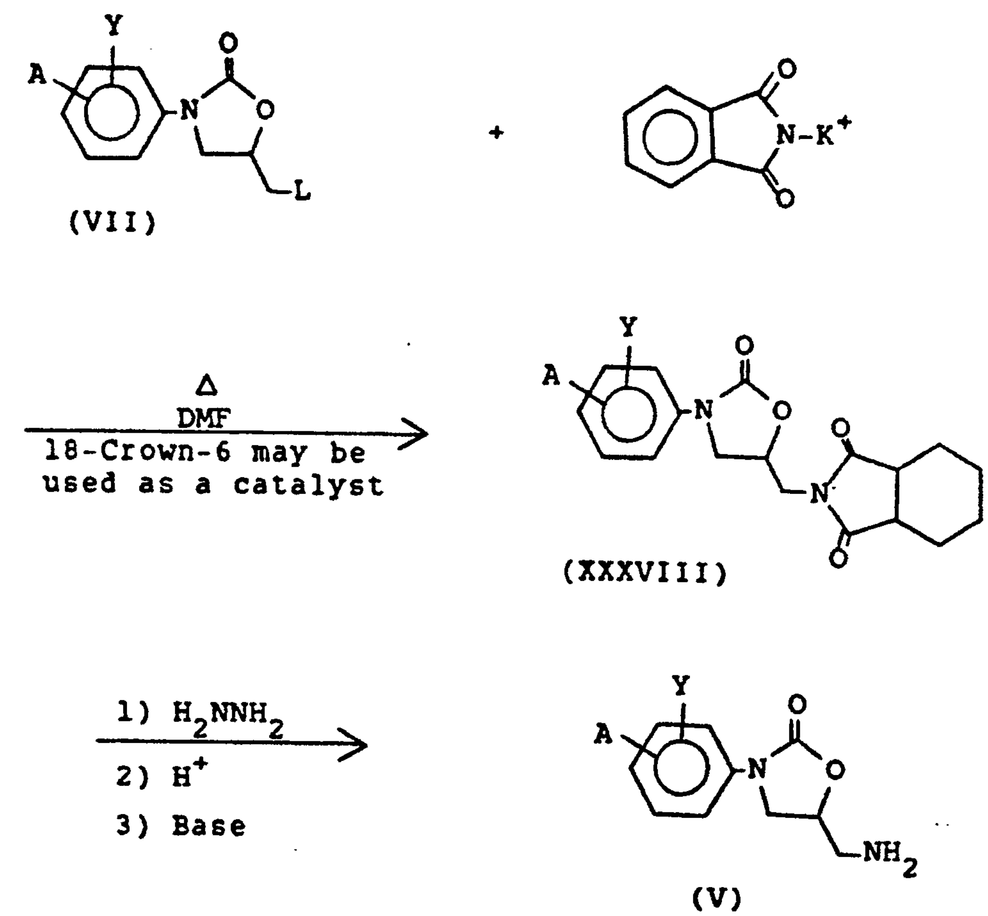


(VII)

(XXXVIII)

(V)

In Scheme 8, A may be H, or any of the groups previously shown except that when A is $R_1S(O)_n$, $R_1$ cannot be $N_3$, and when $R_1$ is $NR_9R_{10}$, $R_9$, $R_{10}$, $R_{11}$ and $R_{11a}$ cannot be H. L may be any suitable leaving group such as I, Br, Cl, benzenesulfonyloxy, 4-toluenesulfonyloxy, methanesulfonyloxy, or trifluoromethanesulfonyloxy. The reaction is carried out by heating at temperatures of 25° to 150°C in a dipolar aprotic solvent such as DMF, DMAc, N-methylpyrrolidinone, tetramethylenesulfone or HMPA. The phthalimide group is then removed by treatment with hydrazine in alcohol at 20°C to 50°C for 5-30 hours followed by adjusting to neutral pH with acid. An alternate method is first to react (XXXVIII) with sodium sulfide, then to dehydrate with N,N-dicyclohexylcarbodiimide, followed by reaction with hydrazine and then treatment with dilute acid. This last method is very mild.

Compounds where A is $-S(O)R_1$ or $-S(O)_2R_1$ may be made as shown in Scheme 9.

Scheme 9

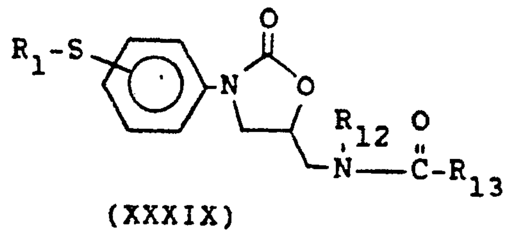

(XXXIX)

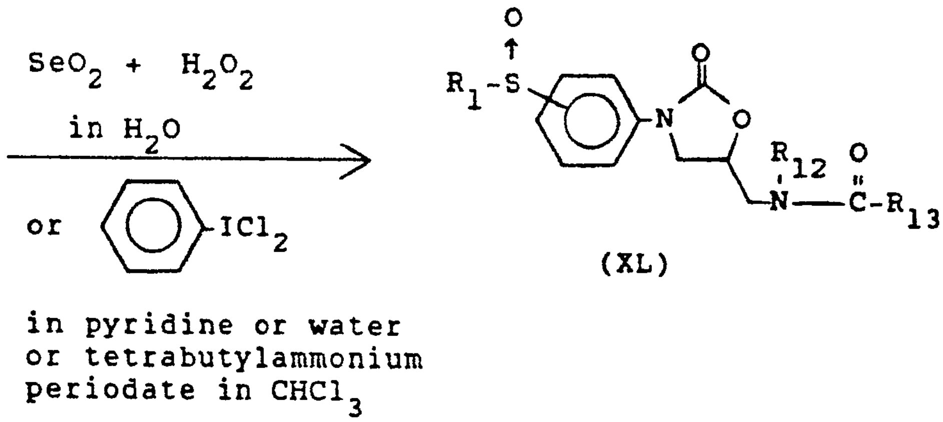


(XL)

(XXXIX)

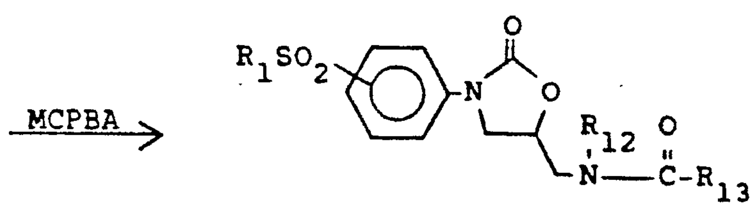


(XLI)

Sulfides of structure (XXXIX) where $R_{12}$ and $R_{13}$ are as defined above may be oxidized to sulfoxides having the structure (XL) by using one equivalent of an oxidant. The preferred oxidant is a water-solution of selenium dioxide containing hydrogen peroxide. Other oxidants which may be used include iodobenzene dichloride in a pyridine-water mixture, or tetrabutylammonium periodate in refluxing chloroform. Strong oxidants such as m-chloroperoxybenzoic acid or peracetic acid may be used; the mixtures containing varying amounts of sulfide, sulfoxide and sulfone thus obtained may be separated by conventional techniques such as chromatography.

Use of two equivalents of a strong oxidizing agent such as m-chloroperoxybenzoic acid results in the sulfone (XLI).

The alcohols (II) and halides (VII) required as starting materials are readily available by any of a number of standard methods for the preparation of oxazolidones. [M. E. Dyen and D. Swern, Chem. Rev., 67, 197-246 (1967)].

Of these methods, the two which are noteworthy for the variety of compounds prepared are outlined in Scheme 10.

Scheme 10:

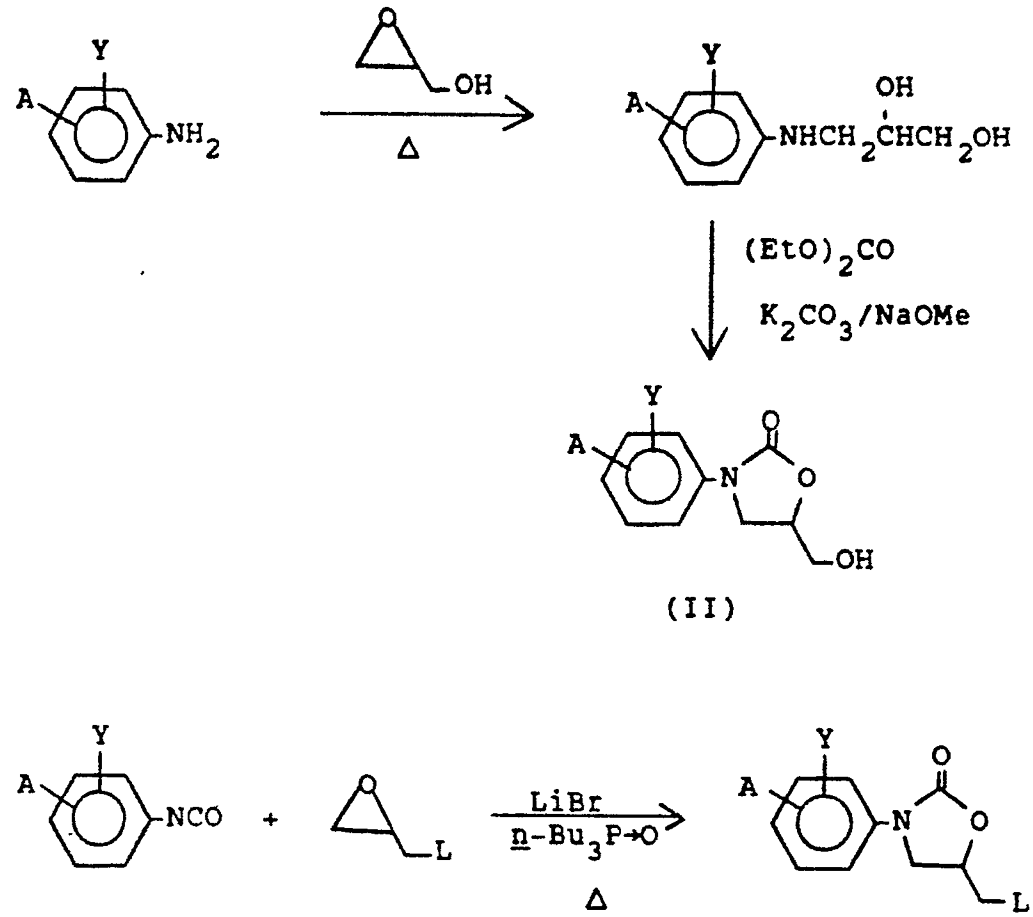


(II)

Pharmaceutically suitable salts of compounds of formula I can be prepared in a number of ways known in the art. In the definition of $R_1$, cations indicated by Z include alkali and alkaline earth metal ions such as $K^+$, $Mg^{++}$, $Ca^{++}$, $Li^+$, $Na^+$ and tetraalkylammonium. Where B is $-NH_2$ or where $R_{10}$ contains an amino group and A is not $S(O)_nNXZ$, pharmaceutically suitable salts include those resulting from treatment with acetic, hydrochloric, sulfuric, phosphoric, succinic, fumaric, ascorbic, and glutaric acid.

Example 1

Preparation of (dℓ)-5-Azidomethyl-3-[4(methylsulfonyl)phenyl]-2-oxazolidinone (I; A = 4-$CH_3SO_2$, B = $N_3$)

Part A

Preparation of (dℓ)-5-Iodomethyl-3-[4(methylsulfonyl)phenyl]-2-oxazolidinone

A mixture of 50 g (345 mmole) of (dℓ)-5-chloromethyl-3-[4-(methylsulfonyl)phenyl]-2-oxazolidinone and 100 g of sodium iodide in 300 ml of 2-butanone was refluxed overnight. This was cooled and poured into 1 liter of ice and water; sodium sulfite was added until all the yellow iodine color was gone; the mixture was filtered and washed with water to provide 61.7 g of iodomethyl compound, m.p. 175.5-177° C. This material was recrystallized from 370 ml of acetonitrile to give 44.8 g, m.p. 177.5-179° C.

Part B

A mixture of 7.6 g (20 mmole) of (dℓ)-5-iodomethyl-3-[4-(methylsulfonyl)phenyl]-2-oxazolidinone and 4 g of sodium azide in 150 ml of (dry) DMAC was heated at 125° C for three hours. It was then poured into ice and water. The product was extracted with chloroform three times and the extracts dried over sodium

sulfate and concentrated to a semi-solid paste. The product was stirred with ether, filtered and dried; yield 4.7 g. This was recrystallized from 14 ml of acetonitrile to give 2.2 g of azidomethyl compound, m.p. 152.5-153.5° C.

Example 2

Preparation of (ℓ)-5-Azidomethyl-3-[4-(methylsulfonyl)phenyl]-2-oxazolidinone (I; A = 4-$MeSO_2$, B = $N_3$)

Part A

Preparation of (ℓ)-5-Hydroxymethyl-3-[4-(methylsulfonyl)phenyl]-2-oxazolidinone, 4-methylbenzenesulfonate (I; A = 4-$MeSO_2$, B = $OSO_2C_6H_4Me$)

A solution of 5.00 g of (ℓ)-5-hydroxymethyl-3-[4-(methylsulfonyl)phenyl]-2-oxazolidinone in 30 ml of pyridine (dry) was stirred at 0-5° C and a solution of 3.7 g of p-toluenesulfonyl chloride in 10 ml of pyridine was added slowly. At the end of the addition the mixture was stirred one hour; the mixture crystallized to a semi-solid mass. A few drops of water were added with evolution of heat. The mixture was poured onto a water-ice mixture, filtered, and washed with water. The product yield was 4.02 g, m.p. 187.1-188.6° C.

Part B

A mixture of 3.5 g of (ℓ)-5-hydroxymethyl-3-[4-(methylsulfonyl)phenyl]-2-oxazolidinone, 4-methylbenzenesulfonate and 2 g of sodium azide in 20 ml of DMF was heated to 90-100° C. At the end of one hour, the mixture was cooled and diluted with ice-water, the product crystallized and was filtered and washed well with water; yield 1.25 g; m.p. 146.5-148.5° C. This product may be crystallized from methanol to give a product melting at 148.9-149.4° C.

Example 3

Preparation of (ℓ)-4-[5-(Azidomethyl)-2-oxooxazolidin-3-yl]benzenesulfonamide (I; A = 4-$H_2NSO_2$, B = $N_3$)

Part A

Preparation of (ℓ)-4-[5-(Hydroxymethyl)-2-oxooxazolidin-3-yl]benzenesulfonamide, 4-methylbenzenesulfonate (I; A = 4-$H_2NSO_2$, B = $OSO_2C_6H_4Me$)

A mixture of 13.61 g (50 mmole) of (ℓ)-4-[5-hydroxymethyl)-2-oxooxazolidin-3-yl]benzenesulfonamide in 50 ml of dry pyridine was stirred at -5 to 0° C as solution of 9.53 g of 4-methylbenzenesulfonyl chloride in 25 ml of pyridine was added dropwise. The reaction was allowed to warm to room temperature and stirred three hours. It was then poured into ice-water, the crystalline product filtered and washed well with water and dried. The yield of product was 19.0 g, m.p. 213.5-217.5° C.

Part B

A mixture of 18.759 (44 mmole) of (ℓ)-4-[5-(hydroxymethyl)-2-oxooxazolidin-3-yl]benzenesulfonamide, 4-methylbenzenesulfonate and 3 g of sodium azide in 75 ml of DMF was heated at 50° C for three hours. The reaction at this stage was only about one-half done, so further sodium azide (2 g) was added and the reaction heated at 50° C for 6 hours and then at 60° C for one hour. It was poured into ice and water, filtered, washed well with water and dried; yield 11.24 g, m.p. 139.1-140.1° C. This was recrystallized from 50 ml of acetonitrile to give 6.1 g of product, m.p. 139.5-140.1° C.

Using the procedures described in Examples 1-3, the following azides could be prepared.

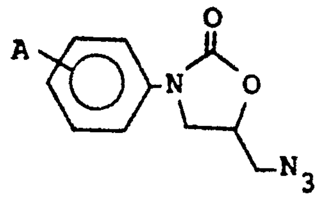

Table 1

| Ex. | A | m.p. (°C) | isomer |
|---|---|---|---|
| 4 | 4-$CH_3S$ | 97.4-98.2° | ℓ |
| 5 | 4-$CH_3CO$ | 101-102° | dℓ |
| 6 | 4-$CF_3$ | | dℓ |
| 7 | 4-$(CH_3)_2CH$ | 63-64° | dℓ |
| 8 | 3-$CH_3CO$ | | dℓ |
| 9 | 4-$CH_3O$ | | dℓ |

Example 10

Preparation of (dℓ)-5-Aminomethyl-3-[4-(methylsulfonyl)phenyl]-2-oxazolidinone trifluoroacetic Acid Salt (A = 4-$CH_3SO_2$, B = $NH_2 \cdot CF_3CO_2H$)

A solution of 1.1 g of (dℓ)-5-azidomethyl-3-[4-(methylsulfonyl)phenyl]-2-oxazolidinone in 75 ml of trifluoroacetic acid and 0.5 g of 10% palladium-on-charcoal was shaken under hydrogen pressure (approximately 3447.4 hPa(50 psig)) for one hour. The mixture was filtered and concentrated to give 0.8 g of product, m.p. 150-170° C (dec.).

Example 11

Preparation of intermediate compound(ℓ)-5-Aminomethyl-3-[4-(methylsulfonyl)-phenyl]-2-oxazolidinone (I; A = 4-$MeSO_2$, B = $NH_2$)

A mixture of 3.48 g (0.0117 mole) of (ℓ)-5-azidomethyl-3-[4-(methylsulfonyl)phenyl]-2-oxazolidinone, 11 ml of 1,3-propanedithiol and 15 ml of triethylamine in 30 ml methanol was warmed to 40-50° C as nitrogen evolution occurred at an appreciable rate. After nitrogen evolution ceased, the solution was concentrated under reduced pressure, the residue stirred with ether, and the solid filtered and dried; yield 3.09 g, m.p. 137-142° C. This was dissolved in about 200 ml of absolute alcohol at reflux (some brown solid did not dissolve) and filtered hot. The product crystallized to yield 2.46 g of product, m.p. 146.6-147.1° C.

Example 12

Preparation of intermediate compound (ℓ-)4-[5-(Aminomethyl)-2-oxooxazolidin-3-yl]benzenesulfonamide (I; A = 4-$H_2NSO_2$, B = $NH_2$)

A suspension of 4.5 g (15.1 mmole) of (ℓ)-4-(5-(azidomethyl)-2-oxooxazolidin-3-yl]benzenesulfonamide in 30 ml of methanol and 3 ml of triethylamine was stirred and 3 ml of 1,3-propanedithiol added. Evolution of nitrogen started and the mixture was warmed to reflux. In 15 minutes, all of the solid had dissolved, and heating was continued thirty minutes longer. The methanol was evaporated in a nitrogen stream and ether was added to the residue and a solid crystallized. The filtered solid was dried; yield 5.01 g m.p. 148-150° C.

This was dissolved in 30 ml water by adding acid, filtered and made strongly basic with concentrated ammonium hydroxide and filtered to give 1.32 g of product, m.p. 151.7-152.4° C.

Anal. Calcd. for $C_{10}H_{13}N_3O_4S$: C, 44.27; H, 4.83; N, 15.49. Found: C, 44.00, 44.13; H, 5.06, 4.85; N, 15.21, 15.21.

Example 13

Preparation of intermediate compound (ℓ)-5-Aminomethyl-3-[4-(methylsulfonyl)-phenyl]-2-oxazolidinone (I; A = 4-MeSO₂, B = NH₂)

A 2.00 g (6.75 mmole) portion of (ℓ)-5-azidomethyl-3-[4-(methylsulfonyl)phenyl]-2-oxazolidinone in 25 ml of 1,2-dimethoxyethane was stirred under nitrogen as 3.2 ml of trimethylphosphite in 5 ml of 1,2-dimethoxyethane was added. The mixture became warm and a rapid evolution of nitrogen occurred. The mixture was concentrated to leave a brown gum. The gum was stirred with water and solid crystallized. This was dissolved in water by adding dilute acetic acid to pH = 4, filtered and the water made basic with concentrated ammonium hydroxide. The yield of product was 0.94 g, m.p. 129-132.8° C.

Example 14

Preparation of (ℓ)-5-Aminomethyl-3-[4-(methylthio)-phenyl]-2-oxazolidinone (I; A = 4-MeS, B = $NH_2$)

A mixture of 30.3 g (115 mmole) of (ℓ)-5-azidomethyl-3-[4-(methylthio)phenyl]-2-oxazolidinone, 13.1 ml of 1,3-propanedithiol and 18.2 ml of triethylamine in 150 ml of methanol was stirred at 50° C for eight hours. It was then concentrated. The residue was stirred with aqueous citric acid, filtered, and the filtrate made basic with concentrated ammonium hydroxide. The product was filtered; yield 16.5 g, m.p. 160-162° C.

Using the procedures of Examples 10-14, the following amines could be prepared.

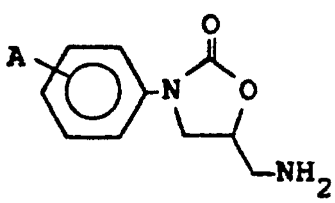

Table 2

| Ex. | A | m.p. (°C) | isomer |
|---|---|---|---|
| 15 | 4-$CH_3CO$ | 115-116° | dℓ |
| 16 | 3-$CH_3CO$ | | dℓ |
| 17 | 4-$(CH_3)_2CH$ | 104.1-105.1 | dℓ acetate salt |
| 18 | 4-$CF_3$ | | dℓ |
| 19 | 4-$CH_3O$ | | dℓ |
| 20 | 4-NC | | dℓ |

Example 21

Preparation of (ℓ)-N-[3-[4-(Methylsulfonyl)phenyl]-2-oxooxazolidin-5-ylmethyl]formamide (I; A = 4-$MeSO_2$, B = NHCHO)

A solution of 1.00 g (3.70 mmole) of (ℓ)-5-aminomethyl-3-[4-(methylsulfonyl)phenyl]-2-oxazolidinone, in 10 ml of 2-propanol containing 2.5 ml of ethyl formate was heated at reflux for twenty-four hours. The mixture was cooled and diluted with ether to give 0.96 g of material which was recrystallized from 9.5 ml of acetonitrile to give 0.65 g of product, m.p. 190-191.6° C.

Example 22

Preparation of (ℓ)-2,2-Dichloro-N-[3-[4-(methylsulfonyl)phenyl]-2-oxooxazolidin-5-ylmethyl]acetamide(I; A = 4-$MeSO_2$, B = $NHCOCHCl_2$)

A mixture of 2.00 g (7.4 mmole) of (ℓ)-5-aminomethyl-3-[4-(methylsulfonyl)phenyl]-2-oxazolidinone, 2 ml methyl dichloroacetate and 10 ml of ethanol was refluxed under nitrogen for five hours. The mixture was concentrated under reduced pressure then stirred with ether and filtered; yield 2.72 g, m.p. 174.0-181.9°. This was stirred with water made acid with acetic acid, filtered and washed with water; yield 2.60 g. m.p. 194.5-196.1° C. This was dissolved in boiling 70% ethanol water made acid with acetic acid, cooled and filtered; yield of product 1.65 g, m.p. 203.3-204.3° C.

Anal. Calcd. for $C_{13}H_{14}Cl_2N_2O_5S$: C, 40.95; H, 3.70; N, 7.35. Found: C, 40.82; H, 3.70; N, 7.10, 7.15.

Example 23

Preparation of (ℓ)-N-[3-[4-(Methylsulfonyl)phenyl]-2-oxooxazolidin-5-ylmethyl]acetamide (I; A = 4-$MeSO_2$, B = $NHCOCH_3$)

A 2.00 g (7.4 mmole) portion of (ℓ)-5-aminomethyl-3-[4-(methylsulfonyl)phenyl]-2-oxazolidinone in 10 ml of pyridine was cooled in a ice-bath as 0.72 ml of acetic anhydride was added. The mixture was stirred for 10 to 20 minutes then diluted with ice-water. The product was filtered and washed with water; m.p. 191.9-192.9° C. After recrystallization from acetonitrile, there was obtained 1.01 g of product, m.p. 192.7-193.2° C.

Anal. Calcd. for $C_{13}H_{16}N_2O_5S$: C, 49.99; H, 5.16; N, 8.97. Found: C, 49.48; H, 5.17; N, 8.93, 8.88.

Example 24

Preparation of (ℓ)-N-[3-[4-(Aminosulfonyl)phenyl]-2-oxooxazolidin-5-ylmethyl]formamide (I; A = 4-$H_2NSO_2$, B = NHCHO)

A mixture of 2.00 g (7.37 mmole) of (ℓ)-[5-(aminomethyl)-2-oxooxazolidin-3-yl]benzenesulfonamide, 2 ml of n-butyl formate and 0.5 g of 1,4-diazobicyclo-[2.2.2]octane (DABCO) in 30 ml of DMF was heated at 90-100° C for about 24 hours. It was concentrated under reduced pressure and the residue stirred with 10 ml of water. The product crystallized, 2.60 g, m.p. 184.5-186.5° C. This was recrystallized from 70% ethanol in water followed by recrystallization from acetonitrile. The product melted at 191-192° C (dec.).

Example 25

Preparation of (ℓ)-N-[3-[4-(Methylsulfonyl)phenyl]-2-oxooxazolidin-5-ylmethyl]methanesulfonamide (I; A = 4-$MeSO_2$, B = $NHSO_2Me$)

A solution of 1.00 g (3.70 mmole) of (ℓ)-5-aminomethyl-3-[4-(methylsulfonyl)phenyl]-2-oxazolidinone in 50 ml of dry pyridine was stirred in an ice-bath as methanesulfonyl chloride (2.3 ml) was slowly added. After the addition was complete, 3 drops of water were added and the mixture concentrated. The residue was stirred with water and a few drops of concentrated HCl added until the solution was acid. The precipitate was filtered, washed with water and dried. The yield was 0.77 g. m.p. 216.7-220.7° C. This was recrystallized from acetonitrile, water (4:1) to give 0.51 g of product. m.p. 219.7-220.7° C.

Example 26

Preparation of (ℓ)-N-[3-[4-(Methylsulfonyl)phenyl]-2-oxooxazolidin-5-ylmethyl]carbamic acid, methyl ester (I; A = 4-$MeSO_2$, B = $NHCO_2Me$)

A mixture of 5.41 g (0.02 mole) of (ℓ)-5-aminomethyl-3-[4-(methylsulfonyl)phenyl]-2-oxazolidinone in 50 ml of tetrahydrofuran was stirred in an ice-bath as a solution of 2 ml of methyl chloroformate in 10 ml of tetrahydrofuran was added along with 2 N NaOH to keep the pH between 10-11. The mixture was stirred 45 minutes after all of the methyl chloroformate had been added. The organic solvents were removed under reduced pressure and the residue diluted with water and the pH brought to 7, the solid filtered and washed with water; yield 6.5 g, m.p. 210-211° C. This was recrystallized from acetonitrile to give 3.5 g of product, m.p. 214-215° C.

A further recrystillized simple melted at 216.9-217.6° C.

Anal. Calcd. for $C_{13}H_{16}O_6N_2S$: C, 47.55; H, 4.91; N, 8.53. Found: C, 47.55, 47.46; H, 4.88, 4.81; N, 8.73, 8.62.

$[\alpha]_D^{25}$ = -47.7 ± 0.4° (c = 1 in acetonitrile)

In the same manner, by reacting the appropriate acyl halide, isocyanate, chloroformate ester, or ester with an amine of the structure:

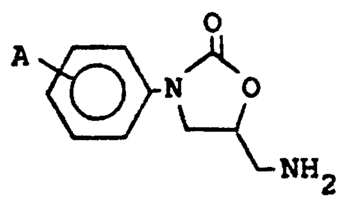

the following compounds could be prepared:

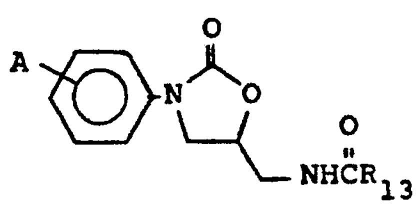

**Table 3**

| Ex. | A, Y | $R_{13}$ | m.p.°C | Isomer |
|---|---|---|---|---|
| 27 | 4-$CH_3SO_2$, H | -$CH_2CH_3$ | 195.8-197.1 | ℓ |
| 28 | 4-$CH_3SO_2$, H | -$CF_3$ | 239.6-240.3 | ℓ |
| 29 | 4-$CH_3SO_2$, H | $CH_2CH_2CH_3$ | 208.1-208.7 | ℓ |
| 30 | 4-$CH_3SO_2$, H | $C(CH_3)_3$ | 172.3-172.9 | ℓ |
| 31 | 4-$CH_3S$, H | $CH_3$ | 166.7-167.1 | ℓ |
| 32 | 4-$CH_3S$, H | $OCH_3$ | 140.5-141.5 | ℓ |
| 33 | 4-$CH_3S$, H | $OCH_2CH_3$ | 140-142 | ℓ |
| 34 | 4-$CH_3SO_2$, H | $C_6H_5$ | 221.6-221.9 | ℓ |
| 35 | 4-$CH_3SO_2$, H | $NHCH_3$ | 197.8-198.7 | ℓ |
| 36 | 4-$CH_3CO$, H | $CH_3$ | 205-207 | dℓ |
| 37 | 3-$CH_3CO$, H | $CH_3$ | 145-146 | dℓ |
| 38 | 4-$(CH_3)_2CH$, H | $CH_3$ | 142.7-143.3 | dℓ |
| 39 | 4-$(CH_3)_2CH$, H | $OCH_3$ | 107.8-108.3 | dℓ |
| 40 | 4-$CH_3S$, H | $CH{=}CH_2$ | 172-174 | dℓ |
| 41 | 4-$CF_3$, H | $CH_3$ | 179.0-179.8 | dℓ |
| 42 | 4-$CF_3$, H | $OCH_3$ | 153.3-153.6 | dℓ |
| 43 | 4-$CH_3O$, H | $OCH_3$ | | |
| 44 | 4-$CH_3O$, H | $CH_3$ | 149.0-149.6 | dℓ |
| 45 | 4-$H_2NSO_2$, H | $OCH_3$ | 229.9-230.5 | ℓ |
| 46 | 4-$CH_3NHSO_2$, H | $CH_3$ | 181.5-182 | ℓ |
| 47 | 4-$(CH_3)SO_2$, H | $CHCl_2$ | | |
| 48 | 4-$CH_2CH$-$CH_2NHSO_2$, H | $CH_2OCH_3$ | | |
| 49 | 4-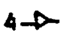$NHSO_2$, H | $CHBr_2$ | | |
| 50 | 4-$CH_3ON(CH_3)SO_2$, H | $OC_2H_5$ | | |
| 51 | 4-$(CH_3)_2CH_3$, H | $CH_3$ | 118.9-119.4 | ℓ |
| 52 | 4-$(CH_3)_2CH$, H | $OCH_3$ | 129.0-129.3 | ℓ |
| 53 | 4-$CH_3NHN(CH_3)SO_2$, H | $CHCl_2$ | | |
| 54 | 4-*n*-$C_4H_9NHSO_2$, H | $CH{=}CH_2$ | | |
| 55 | 4-cyclooctyl $NHSO_2$,H | $CH_2Br$ | | |
| 56 | 4-$H_2NNHSO_2$, H | $CH(OCH_3)_2$ | | |
| 57 | 4-$CH_3SO_2$, H | $CH_2OCH_3$ | 164.6-165.6 | ℓ |
| 58 | 4-$CF_3S$, H | O-$C_4H_9$-*t* | | |

Table 3 (continued)

| Ex. | A, Y | $R_{13}$ | m.p.°C | Isomer |
|---|---|---|---|---|
| 59 | 4-NC, H | $CH_3$ | 153-154 | dℓ |
| 60 | $4-CF_2HSO$, H | $CH=CH_2$ | | |
| 61 | $4-CH_2=CH-CH_2S$, H | $CH_3$ | | |
| 62 | $3,4-OCH_2O-$ | $CH_3$ | 156-157 | dℓ |
| 63 | $4-Cl_2CHSO$, H | $CH(OCH_3)_2$ | | |
| 64 | $4-CH_2FS$, H | $SCH_3$ | | |
| 65 | $4-CCl_3SO$, H | $CH_2-S(O)_2CH_3$ | | |
| 66 | $4-CH_2BrSO_2$, H | $S-C_4H_9$-*n* | | |
| 67 | $4-CH_3SO_2$, H | $CH_2Cl$ | 195.1-195.9 | ℓ |
| 68 | $4-(CH_3)S$, H | $NHCOCOCH_3$ | 142.9-143.5 | ℓ |
| 69 | $4-CH_3SO_2$, H | $CH=CH_2$ | 180-183 | dℓ |
| 70 | $4-CH_3SO_2$, H | $OCH_2CH_2CH_3$ | 170-173 | dℓ |
| 71 | $4-CH_3S$, H | ◁ | 197-199 | dℓ |
| 72 | $4-CH_3SO_2$, H | ◁ | 210-211 | dℓ |
| 73 | $4-CH_3S$, H | $CH(OCH_3)_2$ | 89-90 | dℓ |
| 74 | $4-CH_3SO_2$, H | $CH(OCH_3)_2$ | 175-178 | dℓ |
| 75 | $4-CH_3S$, H | $CH(OC_2H_5)_2$ | 68-69 | dℓ |
| 76 | $4-CH_3SO_2$, H | $NH_2$ | 146-149 | dℓ |
| 77 | $4-CH_3SO_2$, H | $CH(NH_2)C_6H_5$ HCl | 250 | dℓ |

Example 81

Preparation of (ℓ)-2,2-Dichloro-N-[3-[4-(aminosulfonyl)phenyl]-2-oxooxazolidin-5-ylmethyl]acetamide (I; $A = 4-H_2NSO_2$, $B = NHCOCHCl_2$)

Part A

Preparation of (ℓ)-5-hydroxymethyl-3-phenyl-2-oxazolidinone, 4-methylbenzenesulfonate, (I; A = H, $B = OSO_2C_6H_4Me$)

A mixture of 51.5 g of (ℓ)-5-hydroxymethyl-3-phenyl-2-oxazolidinone in 250 ml of dry pyridine was stirred under $N_2$ in an ice-bath as a solution of 53 g of *p*-toluenesulfonyl chloride in 50 ml of pyridine was added. After the addition, cooling was ceased, the mixture allowed to stand for one hour, and then a few drops of water were added (the temperature rose to 39°C as the water reacted with the excess *p*-toluenesulfonyl chloride). The reaction mixture was poured into ice water; the white solid was filtered, washed well with water, and dried. The yield of product was 70.0 g, m.p. 146.3-147.8°C. This product was used without further purification.

Part B

Preparation of (ℓ)-5-Azidomethyl-3-phenyl-2-oxazolidinone (I; A = H, B = $N_3$)

A mixture of 5.00 g (14.4 mmole) of (ℓ)-5-hydroxymethyl-3-phenyl-2-oxazolidinone, 4-methylbenzenesulfonate, 2.1 g sodium azide and 1 g 18-crown-6 in 35 ml of DMF was heated at 100° C for three hours. The mixture was poured into ice-water and filtered. The dried yield was 2.47 g, m.p. 71.5-72.5° C. This was recrystallized from diethyl ether to give 1.44 g of product, m.p. 72.5-73° C.

Part C

Preparation of (ℓ-)5-Aminomethyl-3-phenyl-2-oxazolidinone (I; A = H, B = $NH_2$)

A mixture of 37.0 (170 mmole) of (ℓ)-5-azidomethyl-3-phenyl-2-oxazolidinone, 26 ml of triethylamine, 19.5 ml of 1,3-propanedithiol in 150 ml of methanol was warmed to 50° C. Nitrogen was evolved (at the end of 2 hours, 3.9 liters had been measured). The solvent was removed and the residue crystallized on stirring with ether (crude yield, 20.3 g). This material was used without further purification.

Part D

Preparation of (ℓ)-2,2-Dichloro-N-(3-phenyl-2-oxazolidin-5-ylmethyl)acetamide (I; A = H, B = $NHCOCHCl_2$)

A solution of 12.5 g (64.5 mmole) of (ℓ)-5-aminomethyl-3-phenyl-2-oxazolidinone in 45 ml of methyl dichloroacetate and 45 ml of 1,2-dimethoxyethane containing 1 g of 4-dimethylaminopyridine was refluxed four hours. It was concentrated, the residue stirred with ethyl acetate and the product crystallized and was filtered and dried. The yield was 9.18 g, m.p. 142.3-144.8° C. This was recrystallized from ethanol, filtered hot, and cooled to give 7.46 g, m.p. 150.3-151.3° C.

Part E

A 15 ml portion of chlorosulfonic acid was cooled and stirred under nitrogen as 8.77 g (28.9 mmole) of (ℓ)-2,2-dichloro-N-(3-phenyl-2-oxooxazolidin-5-ylmethyl)acetamide was added. Hydrogen chloride bubbled from the acid and the solid dissolved. After one hour the acid solution was poured into ice with good stirring, filtered and dried on the filter under nitrogen for one hour. This solid was added to a mixture of 25 ml of concentrated ammonium hydroxide in 50 ml of tetrahydrofuran. After stirring for four minutes, the resulting mixture was concentrated under reduced pressure; water was added and the product filtered, washed with water, and dried; yield 9.13 g, m.p. 208-209° C. This was recrystallized from 70% ethanol water to give 6.65 g, m.p. 214.8-215.4° C. It was then recrystallized from acetonitrile to yield 6.54 g, m.p. 216.5-217.5° C.

Example 82

Preparation of (ℓ)-N-[3-[4-(aminosulfonyl)phenyl]-2-oxooxazolidin-5-ylmethyl]acetamide (I; A = 4-$H_2NSO_2$, B = $NHCOCH_3$)

Part A

Preparation of (ℓ)-N-(3-Phenyl-2-oxazolidin-5-ylmethyl)acetamide (I; A = H, B = $NHCOCH_3$)

A solution of 12.5 g (65.0 mmole) of (ℓ)-5-aminomethyl-3-phenyl-2-oxazolidinone in 50 ml of dry pyridine was stirred as 7 ml of acetic anhydride was added. The mixture was allowed to stand overnight, then concentrated. The residue was stirred with water and the solid filtered and dried; yield 10.2 g, m.p. 122.4-124.5° C. This was recrystallised from ethanol to give 5.02 g, m.p. 126.8-127.3° C. A second crop was obtained and recrystallized from ethanol to give 3.08 g, m.p. 127.3-127.8° C.

Part B

The chlorosulfonation and amidation procedures of Example 81E were used, starting with 7.91 g (33.8 mmoles) of (ℓ)-N-(3-phenyl-2-oxooxazolidin-5-ylmethyl)acetamide. The yield of product was 6.85 g, m.p. 236.4-236.6° C.

Example 83

Preparation of (ℓ)-N-[3-(4-Azidosulfonylphenyl)-2-oxooxazolidin-5-ylmethyl]acetamide (I; A = 4-$N_3SO_2$-, B = NH-$COCH_3$)

A 5.0 g (21.3 mmole) portion of (ℓ)-N-(3-phenyl-2-oxooxazolidin-5-ylmethyl)acetamide was added to 25 ml of chlorosulfonic acid, stirred for 2 hours, poured onto ice, filtered, and washed well. After the product was sucked dry on a filter, it was added to a solution made by dissolving 2.0 g sodium azide in 5 ml of water and diluting this with 50 ml of acetone. The mixture was stirred for 2 hours; the acetone was evaporated under reduced pressure. The residue was diluted with water and filtered to provide 5.81 g of product, m.p. 102-104° C (dec.). This was recrystallized from ethanol to give 5.0 g of material, m.p. 122.5-123.4° C (dec.).

Using the chlorosulfonation described in Examples 81 through 83, the following compounds could be prepared.

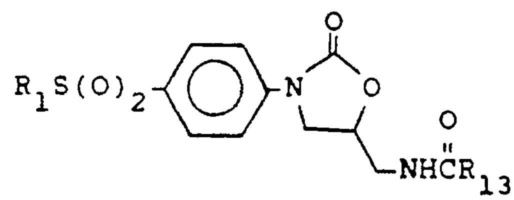

Table 5

| Ex. | $R_1$ | $R_{13}$ | m.p. | isomer |
|---|---|---|---|---|
| 84 | $H_2N$ | $OCH_3$ | 229.9-230.5 | ℓ |
| 85 | $CH_3ON(CH_3)$ | $OCH_3$ | 128.1-129.1 | ℓ |
| 86 | $N_3$ | $OCH_3$ | 107.0-107.5 | ℓ |
| 87 | $CH_3ONH$ | $CH_2CH_3$ | | |
| 88 | $H_2NNH$ | $OCH_2CH_3$ | | |

Example 89

Preparation of (ℓ)-N-[3-[4-(Methylsulfinyl)phenyl)-2-oxooxazolidin-5-ylmethyl]acetamide (I; A = 4-MeSO, B = $NHCOCH_3$)

A 5.61 g (20 mmole) portion of (ℓ)-N-(3-[4-(methylthio)phenyl]-2-oxooxazolidin-5-ylmethyl]acetamide in 200 ml of methanol was stirred at 0° C as a solution of 12.3 g of Oxone® ($2KHSO_5 \cdot KHSO_4 \cdot K_2SO_4$) in 50 ml of water was added slowly. At the end of the addition the sulfide had all been consumed as determined by thin layer chromatography, and the product was a mixture of sulfoxide and sulfone. The solution was heated with 12 ml of methyl sulfide to reduce the excess Oxone®, concentrated under reduced pressure to give 2.0 g of product, m.p. 188.6-189.9° C. This was recrystallized from 70% ethanol-water to give 1.5 g of the sulfoxide, m.p. 193.7-197° C.

Example 90

Preparation of (ℓ)-N-[3-[4-(Methylsulfinyl)phenyl]-2-oxooxazolidin-5-ylmethyl]carbamic acid, methyl ester (I; A = 4-$CH_3SO$, B = $NHCO_2CH_3$)

Using the procedure of Example 89, the title compound could be prepared starting from the compound of Example 32, m.p. 150.5-159.5 °C.

Example 91

Preparation of (dℓ)-N-hexyl-N-[3-[4-(methylsulfonyl)phenyl]-2-oxooxazolidin-5-ylmethyl]acetamide (I; A = 4-$MeSO_2$, B = $N(C_6H_{13})COCH_3$)

Part A

Preparation of (dℓ)-5-(Hexylaminomethyl)-3-[4-(methylsulfonyl)phenyl]-2-oxazolidinone (I; A = $MeSO_2$, B = $NHC_6H_{13}$)

(dℓ)-5-Bromomethyl-3-[4-(methylsulfonyl)phenyl]-2-oxazolidinone (21.92 g) was added to a mixture of 50 ml hexylamine and 25 ml N,N-dimethylformamide. This mixture was heated to 80 °C under nitrogen with vigorous stirring overnight, and allowed to cool to room temperature. The mixture was poured into water with vigorous stirring and the product was collected and washed with ethanol and diethyl ether. The dried weight of crude product was 6.25 g which was recrystallized from acetonitrile to give 4.7 g of (dℓ)-5-(hexylaminomethyl)-3-[4-(methylsulfonyl)phenyl]-2-oxazolidinone, m.p. 132-133 °C.

Part B

To a solution of 3.4 g of (dℓ)-5-(hexylaminomethyl)-3-[4-(methylsulfonyl)phenyl]-2-oxazolidinone in 30 ml of pyridine was added 1.8 ml of acetic anhydride. The mixture was stirred at room temperature overnight. The mixture was evaporated and the residue was triturated with dilute aqueous HCl. The product was collected and washed thoroughly with water to give, after drying, 3.4 g of crude product. This was recrystallized from aqueous ethanol to give 2.6 g of (dℓ)-N-hexyl-N-[3-[4-(methylsulfonyl)phenyl]-2-oxooxazolidin-5-ylmethyl]acetamide, m.p. 123-124 °C.

Example 92

Preparation of (dℓ)-N-hexyl-N-[3-[4-(methylsulfonyl)phenyl]-2-oxooxazolidin-5-ylmethyl]carbamic acid, methyl ester (I; A = 4-$MeSo_2$, B = $N(C_6H_{13})CO_2CH_3$)

In the same manner as in Example 91, Part B, the product of Example 91, Part A is reacted with methyl chloroformate to provide (dℓ)-N-hexyl-N-[3-[4-(methylsulfonyl)phenyl]-2-oxooxazolidin-5-ylmethyl]carbamic acid, methyl ester, m.p. 126-127 °C.

Example 93

(dℓ)-N-Cyclohexyl-N-[[3-[4-(methylsulfonyl)phenyl-2-oxooxazolidin-5-yl]methylacetamide (I; A = 4-$MeSO_2$, B = $N(C_6H_{11})COCH_3$)

Part A

(dℓ)-5-(Cyclohexylaminomethyl)-3-[4-(methylsulfonyl)phenyl]-2-oxazolidinone (I; A = 4-$MeSO_2$, B = $NHC_6H_{11}$)

(dℓ)-5-Hydroxymethyl-3-[4-(methylsulfonyl)phenyl]-2-oxazolidinone, 4-methylbenzenesulfonate (15 g) was added to a mixture of 60 ml cyclohexylamine and 30 ml N,N-dimethylformamide and heated gently to 70 °C under nitrogen with vigorous stirring overnight. The mixture was allowed to cool to room temperature and was then poured onto water. The product precipitated and was collected and dried; yield 7.48 g.

A portion of the solid obtained above (3.75 g) was purified by dissolving in dilute aqueous HCl, washing with ethyl acetate, and precipitating by addition of concentrated ammonium hydroxide. The pure product was washed with water and dried to give 1.1 g of (dℓ)-5-(cyclohexylaminomethyl)-3-[4-(methylsulfonyl)-phenyl]-2-oxazolidinone, m.p. 154-155 °C.

Part B

To a solution of 2.56 g of (dℓ)-5-(cyclohexylaminomethyl)-3-[4-(methylsulfonyl)phenyl]-2-oxazolidinone in 25 ml pyridine was added 2 ml acetic anhydride and the mixture was stirred at room temperature under nitrogen overnight. The mixture was evaporated and the residue was triturated with dilute aqueous HCl. The gummy residue was dissolved in ethyl acetate and washed with saturated aqueous $NaHCO_3$ and brine, and dried over sodium sulfate. Evaporation gave a solid which was triturated with ethyl acetatediethyl ether and collected to give 2.28 g of (dℓ)-N-cyclohexyl-N-[3-[4-(methylsulfonyl)phenyl]-2-oxooxazolidin-5-ylmethyl]-acetamide, m.p. 149-151 ° C.

Using the procedures described above, the following compounds could be prepared.

**Table 6**

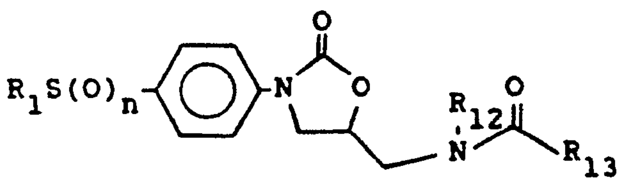

| Ex. | n | $R_1$ | $R_{12}$ | $R_{13}$ | m.p.(°C) | Isomer |
|---|---|---|---|---|---|---|
| 94 | 1 | $-CF_3$ | n-$C_9H_{19}$- | H | | (ℓ)- |
| 95 | 2 | n-$C_4H_9$ | $-CH_3$ | H | | (ℓ)- |
| 96 | 1 | $-C_2H_5$ | $-CH_3$ | $-OCH_3$ | | (ℓ)- |
| 97 | 2 | $-CH_3$ | $-CH_3$ | $-OCH_3$ | 152-155° | (dℓ)- |

## Example 98

Preparation of (ℓ)-N-[3-(4-Nitrophenyl)-2-oxooxazolidin-5-ylmethyl]acetamide (I; A = 4-$NO_2$, B = $NHCOCH_3$)

A 30 ml portion of concentrated sulfuric acid was stirred under dry nitrogen and cooled to -10 ° C; 5 g (21.3 mmole) of (ℓ)-N-(3-phenyl-2-oxooxazolidin-5-ylmethyl)acetamide was added. When all of the solid dissolved, 2.2 g of potassium nitrate was added at -10 ° to 0 ° C. The mixture was then allowed to warm to room temperature over a 2 hour period. The mixture was poured onto ice; the product was filtered, washed well with water, and dried. The yield was 3.47 g. A thin layer chromatogram on silica gel plate eluted with chloroform-methanol (9:1) showed a spot $R_f$ = 0.37 for the p-nitro- and a spot Rf = 0.28 for the o-nitro-compound. The product was recrystallized from acetonitrile to give 2.15 g, m.p. 194.5-195.0 ° C which showed one spot in the thin layer chromatogram, indicating it to be the para-nitro product.

## Example 99

Preparation of (ℓ)-N-[3-(2,4-Dinitrophenyl)-2-oxooxazolidin-5-ylmethyl]acetamide (I; A = 4-$NO_2$, Y = 2-$NO_2$, B = $NHCOCH_3$).

The nitration shown in Example 98 was repeated starting with 15 g of (ℓ)-N-(3-phenyl-2-oxooxazolidin-5-ylmethyl)acetamide. The mother liquor from the crystallization of the crude product (9.82 g) was concentrated and purified by preparative chromatography using the Waters "Prep 500®" and silica gel columns, eluting with 9:1 chloroform-methanol. A fast moving component was the pure p-isomer. The slow moving product 1.02 g, m.p. 142.2-142.6 ° C was the 2,4-dinitro compound.

## Example 100

Preparation of (ℓ)-N-[3-(2-Nitrophenyl)-2-oxooxazolidin-5-ylmethyl]acetamide (I; A = 2-$NO_2$ B = $NHCOCH_3$)

A 90 ml portion of concentrated sulfuric was stirred under dry nitrogen as 11 g of potassium nitrate was added. The mixture became warm and it was cooled in an ice bath to 0-10° C as 23.4 g (0.10 mole) of (ℓ)-N-(3-phenyl-2-oxooxazolidin-5-ylmethyl)acetamide was added slowly. After stirring one hour a thin layer chromatogram showed that there was starting compound left. A further 3 g of potassium nitrate was added and stirring continued two hours. The reaction was poured into ice-water and the product extracted with chloroform. The extract was concentrated and the residue (20 g) was fractionated by preparative chromatography using the Waters Prep 500®. The first fraction amounted to 2.8 g, m.p. 130-136° C.

Example 101

Preparation of (ℓ)-N-[3-(4-Aminophenyl)-2-oxooxazolidin-5-ylmethyl]acetamide (I; A = 4-$H_2N$, B = $NHCOCH_3$)

A mixture of 5.00 g (17.9 mmole) of (ℓ)-N-[3-(4-nitrophenyl)-2-oxooxazolidin-5-ylmethyl]acetamide, 50 ml absolute ethanol and 3 g of Raney nickel catalyst was stirred and heated to 50° C as a solution of 5 ml of 95% hydrazine diluted with 20 ml of absolute ethanol was added slowly. The temperature rose to reflux and gas was evolved. After refluxing thirty minutes, the solution was filtered and concentrated to a glass which crystallized. This was stirred with acetonitrile and filtered; yield 3.42 g, m.p. 147.5-148.3° C.

Example 102

Preparation of (ℓ)-N-[3-[4-(Acetylamino)phenyl]-2-oxooxazolidin-5-ylmethyl]acetamide (I; A = 4-$CH_3CONH$, B = $NHCOCH_3$)

A 0.95 g portion of the above aniline (Example 101) in 5 ml of tetrahydrofuran and 5 ml of triethylamine, 2 ml of acetic anhydride, 0.01 g 4-dimethylaminopyridine (DMAP) and 10 ml of dimethylacetamide was warmed, then concentrated under reduced pressure, water added and the white solid filtered and washed with water to yield 0.56 g, m.p. 224.1-224.9° C (dec.). This was recrystallized from 50 ml of acetonitrile to yield 0.44 g, m.p. 225.5-225.8° C (dec).

Example 103

Preparation of (ℓ)-N-[3-[4-(Methylsulfonylamino)phenyl]-2-oxooxazolidin-5-ylmethyl]acetamide (I; A = $CH_3$-$SO_2$-NH-, B = -NH-$COCH_3$)

A solution of 1.24 g (5 mmole) of the above aniline (Example 101) in 5 ml of pyridine was stirred in an ice-acetone bath under nitrogen as 0.4 ml of methane- sulfonyl chloride was added. An intense red color developed and solid separated. The mixture was stirred one hour, diluted with water and made acidic with hydrochloric acid. This was concentrated under reduced pressure and the residue was stirred with acetonitrile and filtered; yield 0.50 g, m.p. 223.5-224.4° C. This solid is quite water soluble.

Example 104

Preparation of (ℓ)-N-[3-[4-(Acetylthio)phenyl]-2-oxooxazolidin-5-ylmethyl)acetamide

$$(I;\ A=4\text{-}CH_3\overset{O}{\overset{\|}{C}}S,\ B=NHCOCH_3).$$

A 10.0 g (0.0427 mole) portion of (ℓ)-N-(3-phenyl-2-oxooxazolidin-5-ylmethyl)acetamide was chlorosulfonated by adding it to 40 ml of chlorosulfonic acid cooled to 0° C under nitrogen. The mixture was stirred for 1.5 hours, poured on ice and the white solid filtered and washed well with water and dried. The yield was 13 g, m.p. 134.9-135.9° C.

The sulfonyl chloride was added to a mixture of 180 ml of acetic acid, 60 ml of acetic anhydride and 30 g of anhydrous sodium acetate, the mixture heated to 75° C, and zinc dust added slowly. The temperature rose to reflux and the zinc was added until it was no longer consumed (16 g). Reflux was then continued for one and one half hours. The cooled mixture was filtered and concentrated. The residue was stirred with

tetrahydrofuran, filtered and concentrated, diluted with ether to give 10.1 g, m.p. 130-180° C. This was dissolved in hot acetonitrile and filtered, concentrated and cooled to yield 5.57 g, m.p. 138.5-139.1° C.

Example 105

Preparation of (ℓ)-N-[3-(4-Mercaptophenyl)-2-oxooxazolidin-5-ylmethyl]acetamide (I, A=4-HS, B=$NHCOCH_3$).

A 4.1 g of (ℓ)-N-[3-[4-(acetylthio)phenyl]-2-oxooxazolidin-5-ylmethyl]acetamide in 20 ml of absolute ethanol was stirred at 25° C as 5 ml of pyrrolidine was added. The temperature rose to 40° C, and all of the solid dissolved. Stirring was continued for one hour, the mixture concentrated, diluted with water and filtered to give 3.32 g, m.p. 205-209° C (dec.).

Example 106

Preparation of (ℓ)-N-[3-[4-(Cyanomethylthio)phenyl]-2-oxooxazolidin-5-ylmethyl]acetamide (I; A=4-$N{\equiv}CCH_2S$, B=$NHCOCH_3$).

A suspension of 1.5 g of powdered potassium carbonate in dimethylformamide was stirred under dry nitrogen as 2.5 g (9.4 mmole) of (ℓ)-N-[3-[4-mercaptophenyl)-2-oxooxazolidin-5-ylmethyl]acetamide was added. To this was added 0.65 ml of chloroacetonitrile. After stirring for an hour, the mixture was concentrated. The residue was dissolved in dichloromethane and chromatographed on a 10 inch column of silica gel. The fast moving spot (eluted with 90% dichloromethane, 10% methanol) yield 0.070 g, was recrystallized from ethyl acetate to yield 60 mg. m.p. 90.4° C using a Metler Melting Point apparatus.

Example 107

Preparation of (ℓ)-N-[3-[4-(Acetylthio)phenyl]-2-oxooxazolidin-5-ylmethyl]carbamic acid (I; A=4-$CH_3CO$-S-, B=$NHCOOCH_3$).

A 12.0 g (48 mmole) of (ℓ)-(3-phenyl-2-oxooxazolidin-5-ylmethyl)carbamic acid methyl ester was added to 60 ml of chlorosulfonic acid cooled to -10° C under nitrogen. The solid slowly dissolved. The addition required thirty minutes. The mixture was allowed to warm and at 10° C a very rapid evolution of hydrogen chloride occurred, and all solid dissolved. The stirring was continued two hours at 20-25° C and then the reaction was quenched on ice, the solid was filtered and washed well with water and dried in a nitrogen stream. The yield was 14.6 g, m.p. 155.4° C (Metler apparatus).

The sulfonyl chloride (9 g; 33.7 mmole) was added to a mixture of 145 ml acetic acid, 50 ml acetic anhydride, and 14 g anhydrous sodium acetate and stirred well as 12 g of zinc dust was added. The mixture was refluxed for one hour, cooled, filtered and concentrated. The residue was stirred with water and filtered to give 4.42 g. This was recrystallized from acetonitrile to give 3.22 g, m.p. 156.4-156.8° C.

Example 108

Preparation of (ℓ)-[3-(4-Mercaptophenyl)-2-oxooxazolidin-5-ylmethyl]carbamic acid, methyl ester (I; A=4-HS, B=$NHCOOCH_3$).

A mixture of 2.00 g (6.17 mmole) of (ℓ)-[3-[4-(acetylthio)phenyl]-2-oxooxazolidin-5-ylmethyl]carbamic acid, methyl ester in 10 ml of absolute ethanol was stirred under nitrogen as 2 ml of pyrrolidine was added and then refluxed for thirty minutes, concentrated under reduced pressure, diluted with water and made acid with acetic acid. The white solid was filtered, washed with water and dried; yield 1.7 g, m.p. 131.7-132.6° C.

Example 109

Preparation of (dℓ)-2-Amino-N-[3-[4-(1-methylethyl)phenyl]-2-oxooxazolidin-5-ylmethyl]acetamide (I; A=4-$(CH_3)_2CH$, B=$NHCOCH_2NH_2$)

Part A

A solution of 5 g (16.1 mmole) of (dℓ)-2-chloro-N-[3-[4-(1-methylethyl)phenyl]-2-oxooxazolidin-5-ylmethyl]acetamide in 50 ml of dry dimethylsulfoxide and 1.5 g sodium azide was stirred and heated to 90 °C under dry nitrogen for five hours. The mixture was concentrated at reduced pressure and the residue stirred with water. A partially crystalline solid separated and solidified on standing, yield 5.8 g. This was recrystallized from ethyl acetate to give 3.4 g, m.p. 122.4-123.4 (dec.). A thin layer chromatogram on silica using 9:1 $CHCl_3$-methanol indicated that this was a mixture of the starting compound and the desired product. This was used in the next step without further purification.

Part B

A suspension of 3.4 g (dℓ)-2-Azido-N-[3-[4-(1-methylethyl)phenyl]-2-oxooxazolidin-5-ylmethyl]acetamide in 50 ml of ethanol, 5 ml of water and 5 ml of acetic acid containing 0.5 g 10% palladium-on-charcoal was stirred as hydrogen was passed into the solution through a dispersion tube. The reaction was continued three hours, the solution was filtered and concentrated, the residue stirred with water and made basic with concentrated ammonium hydroxide to give a gummy solid. This was extracted with ethyl acetate, dried over sodium sulfate and concentrated. The residue was stirred with ether and filtered; yield 1.4 g, m.p. 82-92 °C. This was recrystallized from 10 ml of ethyl acetate and a few drops of triethylamine to give 0.84 g, m.p. 105-107 °C.

Example 110

Preparation of ℓ-2-Azido-N-[3-(4-Methylsulfonyl)phenyl]-2-oxooxazolidin-5-ylmethyl]acetamide, (I; A = 4-$CH_3SO_2$, B = $NHCOCH_2N_3$).

Substituting ℓ-2-chloro-N-[3-[4-(methylsulfonyl)phenyl]-2-oxooxazolidin-5-ylmethyl]acetamide in the azide displacement of Example 109, Part A gives the title compound, m.p. 188.8-189.8 °C.

Example 111

N-[3-(4-Acetylphenyl)-2-oxooxazolidin-5-ylmethyl]-acetamide Oxime

$$(I:(A=4-CH_3\overset{\overset{NOH}{\|}}{C},\quad B=NHCOCH_3)$$

N-[3-(4-Acetylphenyl)-2-oxooxazolidin-5-ylmethyl]acetamide (3.16 g) was dissolved in a mixture of 20 ml pyridine and 20 ml ethanol and 5 g hydroxylamine hydrochloride was added. The mixture was heated to reflux under nitrogen for 2 hours. After allowing to cool to room temperature, the solvents were evaporated and the residue was triturated with dilute aqueous hydrochloric acid. The solid was collected and washed with water. Recrystallization from aqueous ethanol gave 1.6 g pure N-[3-(4-acetylphenyl)-2-oxooxazolidin-5-ylmethyl]acetamide oxime, m.p. 213-215 °C.

Example 112

N-[3-(4-Acetylphenyl)-2-oxooxazolidin-5-ylmethyl]acetamide Oxime, methyl ether

$$(I:\ A=CH_3\overset{\overset{NOCH_3}{\|}}{C}\ ,\quad B=NHCOCH_3)$$

Substitution of methoxylamine hydrochloride for the hydroxylamine hydrochloride in the procedure of Example 111 gave 1.8 g N-(3-(4-acetylphenyl)-2-oxooxazolidin-5-ylmethyl]acetamide oxime methyl ether, m.p. 208-211 °C.

## Dosage Forms

The antibacterial agents of this invention can be administered by any means that produces contact of the active agent with the agent's site of action in the body of a mammal. They can be administered by any conventional means available for use in conjunction with pharmaceuticals, either as individual therapeutic agents or in a combination of therapeutic agents. They can be administered alone, but are generally administered with a pharmaceutical carrier selected on the basis of the chosen route of administration and standard pharmaceutical practice.

The dosage administered will, of course, vary depending upon known factors such as the pharmacodynamic characteristics of the particular agent, and its mode and route of administration; age, health, and weight of the recipient; nature and extent of symptoms, kind of concurrent treatment, frequency of treatment, and the effect desired. Usually a daily dosage of active ingredient can be about 5 to 20 milligrams per kilogram of body weight. Ordinarily, when the more potent compounds of this invention are used, 5 to 15, and preferably 5 to 7.5 milligrams per kilogram per day, given in divided doses 2 to 4 times a day or in sustained release form, is effective to obtain desired results. These drugs may also be administered parenterally.

Dosage forms (compositions) suitable for internal administration contain from about 1.0 milligram to about 500 milligrams of active ingredient per unit. In these pharmaceutical compositions the active ingredient will ordinarily be present in an amount of about 0.5 - 95% by weight based on the total weight of the composition.

The active ingredient can be administered orally in solid dosage forms, such as capsules, tablets, and powders, or in liquid dosage forms, such as elixirs, syrups, and suspensions, it can also be administered parenterally, in sterile liquid dosage forms.

Gelatin capsules contain the active ingredient and powdered carriers, such as lactose, sucrose, mannitol, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration contain preferably a water soluble salt of the active ingredient, suitable stabilizing agents, and if necessary, buffer substances. Antioxidants such as sodium bisulfate, sodium sulfite, or ascorbic acid either alone or combined are suitable stabilizing agents. Also used are citric acid and its salts and sodium EDTA. In addition parenteral solutions can contain preservatives, such as benzalkonium chloride, methyl-or propyl-paraben, and chlorobutanol.

Suitable pharmaceutical carriers are described in Remington's Pharmaceutical Sciences, A. Osol, a standard reference text in this field.

Useful pharmaceutical dosage forms for administration of the compounds of this invention can be illustrated as follows:

### Capsules

A large number of unit capsules are prepared by filling standard two-piece hard gelatin capsules each with 75 milligrams of powdered active ingredient, 150 milligrams of lactose, 24 milligrams of talc and 6 milligrams of magnesium stearate.

### Soft Gelatin Capsules

A mixture of active ingredient in soybean oil is prepared and injectd by means of a positive displacement pump into gelatin to form soft gelatin capsules containing 75 milligrams of the active ingredient. The capsules are washed and dried.

### Tablets

A large number of tablets are prepared by conventional procedures so that the dosage unit is 75

milligrams of active ingredient, 0.2 milligrams of colloidal silicon dioxide, 5 milligrams of magnesium stearate, 250 milligrams of microcrystalline cellulose, 11 milligrams of cornstarch and 98.8 milligrams of lactose. Appropriate coatings may be applied to increase palatability or delay absorption.

Injectable

A parenteral composition suitable for administration by injection is prepared by stirring 1.5% by weight of active ingredient in 10% by volume propylene glycol and water. The solution is made isotonic with sodium chloride and sterilized.

Suspension

An aqueous suspension is prepared for oral administration so that each 5 milliliters contain 75 milligrams of finely divided active ingredient, 200 milligrams of sodium carboxymethyl cellulose, 5 milligrams of sodium benzoate, 1.0 grams of sorbitol solution, U.S.P., and 0.025 milliliters of vanillin.

Utility

Test results indicate that the novel compounds of this invention are biologically active against gram negative and gram positive bacteria including betalactamase producing Staphylococcus aureus isolates. These agents are potentially useful for the treatment of both human and animal bacterial infections including diseases of the respiratory, gastrointestinal, genito-urinary and central nervous systems; blood; interstitial fluids, soft tissue; and bone.

As shown in Table 7, compounds of formula I exert an in vitro antibacterial effect. A standard microdilution method (Conrath, Theodore B., 1972 Handbook of Microtiter Procedures, Dynatech Corporation, Cambridge, Massachusetts) with Mueller-Hinton broth is used to determine the 24-hour minimal inhibitory concentrations (MIC's) for test strains of Staphylococcus epidermidis and Escherichia coli.

In vitro tests conducted with the compound of Example 92 using the same procedures as described above, resulted in no control of Staphylococcus aureus or Escherichia coli. It is believed that the compound of Example 92 would provide control at higher concentrations or under different conditions. It was found to exhibit an antibacterial effect in vivo (see Tables 8 and 9).

The in vivo potency of these compounds is exemplified by the data summarized in Tables 8 and 9. Determinations of in vivo efficacy ore performed by inoculating mice intraperitoneally with cultures of the infecting organism diluted to produce 90-100% mortality in control animals within seven days. The diluents used were trypticase soy broth for E. coli and 5% aqueous hog gastric mucin for Staphylococcus aureus infections. The compounds are dissolved or suspended in 0.25% aqueous Methocel® (Methocel®: Hydroxypropyl Methylcellulose E15 Premium, Dow Chemical Company) for oral administration or sterile distilled water containing 5% dimethylsulfoxide (Fisher Scientific Company, Fairlawn, N.J.) for subcutaneous administration. The mice are dosed at the time of infection and again at four hours post-infection. Mortality is recorded daily until test termination and the 50 percent effective dose. $ED_{50}$, is calculated by the Reed-Muench method (Reed, L. G. and Muench, H., "A simple method of estimating fifty percent end points," American Journal of Hygiene. 27. 493-497 (1938).

Projected therapeutic levels in humans should be attained from the oral administration of 5-20 mg/kg of body weight given in divided doses two to four times daily. The dosages may be increased in severe or life-threatening infections.

## Table 7

### *IN VITRO* BROTH DILUTION MINIMAL INHIBITORY CONCENTRATIONS

Microdilution Broth MIC in μg/ml

| Ex. No. | Staphylococcus epidermidis | Escherichia coli |
|---|---|---|
| 2 | 6.3 | >100.0 |
| 3 | 25.0 | >100.0 |
| 4 | >200.0 | >200.0 |
| 5 | 200.0 | >200.0 |
| 7 | 100.0 | >200.0 |
| 10 | 50.0 | >100.0 |
| 11 | >100.0 | >100.0 |
| 12 | >100.0 | >100.0 |
| 15 | >200.0 | >200.0 |
| 17 | >200.0 | >200.0 |
| 21 | 6.3 | 100.0 |
| 22 | 2.4 | 9.4 |
| 23 | 3.2 | 25.0 |
| 24 | >100.0 | >100.0 |
| 25 | 100.0 | >100.0 |
| 26 | 6.3 | 100.0 |
| 27 | 6.3 | 50.0 |
| 28 | 12.5 | 50.0 |
| 29 | 12.5 | 100.0 |
| 30 | 200.0 | >200.0 |
| 31 | 3.9 | >200.0 |
| 32 | 12.5 | >200.0 |
| 33 | 50.0 | >200.0 |
| 34 | 25.0 | >200.0 |
| 35 | 25.0 | 200.0 |
| 36 | 25.0 | >200.0 |
| 37 | 200.0 | >200.0 |

Table 7 (continued)

IN VITRO BROTH DILUTION
MINIMAL INHIBITORY CONCENTRATIONS

| Ex. No. | Microdilution Broth MIC in μg/ml: Staphylococcus epidermidis | Escherichia coli |
|---|---|---|
| 38 | 9.4 | >200.0 |
| 39 | 12.5 | >200.0 |
| 40 | 12.5 | >200.0 |
| 41 | 12.5 | >200.0 |
| 42 | 100.0 | >200.0 |
| 44 | 100.0 | >200.0 |
| 45 | 37.5 | >200.0 |
| 46 | 12.5 | >200.0 |
| 51 | 3.1 | >200.0 |
| 52 | 6.3 | >200.0 |
| 57 | 12.5 | 100.0 |
| 59 | 100.0 | >200.0 |
| 67 | 3.2 | 2.5 |
| 68 | 100.0 | >200.0 |
| 69 | 9.4 | 150.0 |
| 70 | 50.0 | >200.0 |
| 71 | 50.0 | >200.0 |
| 72 | 25.0 | 200.0 |
| 73 | >200.0 | >200.0 |
| 74 | 100.0 | >200.0 |
| 75 | >200.0 | >200.0 |
| 76 | 200.0 | >200.0 |
| 77 | >200.0 | >200.0 |
| 81 | 12.5 | 50.0 |
| 82 | 25.0 | 100.0 |
| 83 | 200.0 | 200.0 |
| 84 | 37.5 | >200.0 |

Table 7 (continued)

IN VITRO BROTH DILUTION
MINIMAL INHIBITORY CONCENTRATIONS

Microdilution Broth MIC in μg/ml

| Ex. No. | Staphylococcus epidermidis | Escherichia coli |
|---|---|---|
| 85 | 12.5 | >200.0 |
| 86 | 200.0 | >200.0 |
| 87 | 9.4 | 166.7 |
| 90 | 18.8 | >200.0 |
| 91 | >200.0 | >200.0 |
| 92 | >200.0 | >200.0 |
| 93 | >200.0 | >200.0 |
| 97 | >200.0 | >200.0 |
| 98 | 2.4 | 200.0 |
| 99 | 200.0 | >200.0 |
| 100 | 200.0 | >200.0 |
| 101 | 100.0 | >200.0 |
| 102 | 200.0 | >200.0 |
| 103 | 200.0 | >200.0 |
| 104 | >200.0 | >200.0 |
| 105 | 32.0 | >32.0 |
| 106 | 3.2 | >200.0 |
| 107 | >200.0 | >200.0 |
| 108 | >200.0 | >200.0 |
| 109 | 50.0 | >200.0 |
| 110 | 6.3 | 50.0 |
| 111 | 50.0 | >200.0 |
| 112 | 50.0 | >200.0 |

Table 8

IN VIVO EFFICACY OF ORALLY ADMINISTERED COMPOUNDS IN MOUSE INTRAPERITONEAL INFECTIONS

| Ex. No. | Infecting Bacterial Organism<br>Staphylococcus aureus<br>$ED_{50}$ | <br>Escherichia coli<br>$ED_{50}$ |
|---|---|---|
| 2 | 7.3 | N.T. |
| 3 | 29.3 | >120.0 |
| 4 | 43.3 | N.T. |
| 5 | 172.0 | N.T. |
| 7 | 24.2 | N.T. |
| 11 | 29.9 | 47.4 |
| 12 | 179.0 | N.T. |
| 15 | 40.0 | N.T. |
| 17 | 44.4 | N.T. |
| 21 | 7.3 | 30.3 |
| 22 | 14.2 | 71.1 |
| 23 | 3.3 | 14.0 |
| 24 | 74.3 | N.T. |
| 25 | >360.0 | N.T. |
| 26 | 1.7 | 56.2 |
| 27 | 8.0 | 37.0 |
| 28 | 71.3 | N.T. |
| 29 | 88.7 | N.T. |
| 30 | >120.0 | N.T. |
| 31 | 3.5 | 19.6 |
| 32 | 3.5 | 70.9 |
| 33 | 12.2 | >120.0 |
| 34 | >120 | N.T. |
| 35 | 35.8 | N.T. |
| 36 | 4.7 | 47.2 |
| 37 | 62.9 | N.T. |
| 38 | 9.1 | >120.0 |

Table 8 (continued)

IN VIVO EFFICACY OF ORALLY ADMINISTERED COMPOUNDS IN MOUSE INTRAPERITONEAL INFECTIONS

| | Infecting Bacterial Organism | |
|---|---|---|
| Ex. No. | Staphylococcus aureus $ED_{50}$ | Escherichia coli $ED_{50}$ |
| 39 | 6.1 | >120.0 |
| 40 | 53.1 | N.T. |
| 41 | 5.3 | >120.0 |
| 42 | 45.5 | N.T. |
| 44 | 30.3 | N.T. |
| 45 | >120.0 | N.T. |
| 46 | 15.8 | 62.5 |
| 51 | 6.4 | 62.9 |
| 52 | 4.9 | >120.0 |
| 57 | 10.8 | 39.0 |
| 59 | 4.3 | N.T. |
| 62 | 18.1 | N.T. |
| 67 | 42.5 | >120.0 |
| 68 | 48.0 | N.T. |
| 69 | 12.0 | 85.0 |
| 70 | 51.7 | N.T. |
| 71 | >120.0 | N.T. |
| 72 | >120.0 | N.T. |
| 73 | 59.5 | N.T. |
| 74 | 96.6 | N.T. |
| 75 | 130.9 | N.T. |
| 81 | >360.0 | >360.0 |
| 82 | 17.2 | 29.7 |
| 83 | 15.3 | 10.5 |
| 84 | >120.0 | N.T. |
| 85 | 25.9 | N.T. |
| 86 | 16.1 | >120.0 |

Table 8 (continued)

IN VIVO EFFICACY OF ORALLY ADMINISTERED COMPOUNDS IN MOUSE INTRAPERITONEAL INFECTIONS

Infecting Bacterial Organism

| Ex. No. | Staphylococcus aureus $ED_{50}$ | Escherichia coli $ED_{50}$ |
|---|---|---|
| 89 | 3.3 | 11.1 |
| 90 | 2.5 | 55.9 |
| 91 | 31.3 | N.T. |
| 92 | 27.6 | N.T. |
| 93 | 48.4 | >120.0 |
| 97 | 62.0 | N.T. |
| 98 | 2.0 | 29.8 |
| 99 | 38.4 | N.T. |
| 100 | 21.0 | >120.0 |
| 101 | 20.2 | >120.0 |
| 102 | 56.9 | N.T. |
| 103 | 62.9 | N.T. |
| 104 | 4.4 | 24.8 |
| 105 | 5.7 | 17.0 |
| 107 | 3.0 | 82.2 |
| 108 | 4.5 | >120.0 |
| 109 | 58.9 | N.T. |
| 110 | 11.4 | 56.5 |
| 111 | 6.5 | 71.5 |
| 112 | 5.1 | 105.3 |

[1] $ED_{50}$ = 50 percent effective dose in mg/kg

[2] N.T. = Not tested.

Table 9

IN VIVO EFFICACY OF COMPOUNDS ADMINISTERED SUBCUTANEOUSLY IN MOUSE INTRAPERITONEAL INFECTIONS

| Ex. No. | Infecting Bacterial Organism: Staphylococcus aureus $ED_{50}$ | Escherichia coli $ED_{50}$ |
|---|---|---|
| 5 | 41.2 | N.T. |
| 7 | 33.7 | N.T. |
| 11 | 16.4 | N.T. |
| 12 | 89.8 | N.T. |
| 15 | 24.9 | N.T. |
| 17 | 24.9 | N.T. |
| 22 | N.T. | 11.8 |
| 25 | 83.6 | >100.0 |
| 26 | N.T. | 40.7 |
| 30 | 57.4 | >120.0 |
| 31 | >4.4 | N.T. |
| 32 | >4.4 | N.T. |
| 33 | 8.6 | N.T. |
| 34 | 49.6 | N.T. |
| 36 | 7.4 | >120.0 |
| 38 | 4.8 | 60.4 |
| 39 | 5.5 | >120.0 |
| 41 | 6.1 | N.T. |
| 42 | 20.9 | N.T. |
| 45 | 9.6 | N.T. |
| 46 | >13.0 | 91.0 |
| 57 | N.T. | 12.9. |
| 67 | 18.6 | 99.0 |
| 71 | 69.3 | N.T. |

Table 9 (continued)

IN VIVO EFFICACY OF COMPOUNDS ADMINISTERED SUBCUTANEOUSLY IN MOUSE INTRAPERITONEAL INFECTIONS

| | Infecting Bacterial Organism | |
|---|---|---|
| Ex. No. | Staphylococcus aureus $ED_{50}$ | Escherichia coli $ED_{50}$ |
| 72 | 15.2 | N.T. |
| 76 | 70.9 | N.T. |
| 77 | 67.1 | N.T. |
| 81 | 14.4 | 62.7 |
| 82 | 9.6 | 11.7 |
| 83 | N.T. | 12.5 |
| 84 | 9.6 | N.T. |
| 85 | 14.9 | N.T. |
| 86 | 7.2 | >120.0 |
| 89 | >4.4 | N.T. |
| 91 | 29.3 | N.T. |
| 92 | 46.6 | N.T. |
| 93 | 16.3 | >120.0 |
| 97 | 33.6 | N.T. |
| 98 | >13.0 | 40.0 |
| 100 | 21.5 | N.T. |
| 101 | 10.3 | N.T. |
| 103 | 9.7 | N.T. |
| 104 | >2.5 | N.T. |
| 105 | >13.0 | 57.2 |
| 107 | > 4.4 | N.T. |
| 108 | > 4.4 | N.T. |
| 109 | 19.6 | N.T. |
| 110 | >13.0 | 25.0 |

[1] $ED_{50}$ = 50 percent effective dose in mg/kg
[2] N.T. = Not tested.

**Claims**

**Claims for the following Contracting States: BE, CH, DE, FR, GB, IT, LI, LU, NL, SE**

**1.** A compound of the formula

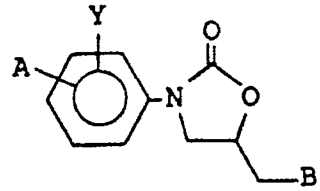

(I)

wherein, for the ℓ, and mixtures of the d and ℓ stereoisomers of the compound,

A is $-NO_2$, $-S(O)_nR_1$, -SH,

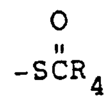

$-COR_5$,

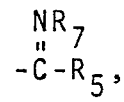

-CN, $-OR_5$, $-NR_5R_6$,

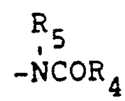 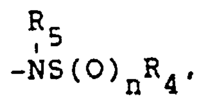

alkyl of 1 to 5 carbons, optionally substituted with one or more halogen atoms or alkenyl of 2-5 carbons;

$R_1$ is $C_1$-$C_4$ alkyl, optionally substituted with one or more halogen atoms, CN; $-NR_9R_{10}$; $-N_3$;

$R_4$ is alkyl of 1-4 carbons,

$R_5$ and $R_6$ are independently H, alkyl of 1-4 carbons

$R_7$ is $-OR_5$;

$R_8$ is H or alkyl of 1-4 carbons

$R_9$ is H, $C_1$-$C_4$ alkyl or $c_3$-$C_8$ cycloalkyl;

$R_{10}$ is H, $-OR_8$ or $-NR_{11}R_{11a}$;

$R_{11}$ and $R_{11a}$ are independently H or $C_1$-$C_4$ alkyl;

Y is H or $NO_2$, or A and Y taken together can be $-O(CH_2)_tO-$;

n is 0, 1 or 2;

t is 1, 2 or 3;

B is,

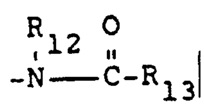

or $N_3$;

$R_{12}$ is H, $C_1$-$C_{10}$ alkyl or $C_3$-$C_8$ cycloalkyl;

$R_{13}$ is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $CH_2S(O)_vR_{14}$; $-OR_{18}$; $-SR_{14}$; $-CH_2N_3$; $-NR_{10}R_{20}$; or $C(NH_2)R_{21}R_{22}$;

$R_{14}$ is $C_1$-$C_4$ alkyl, optionally substituted with one or more halogen atoms;

| | |
|---|---|
| $R_{15}$ | is $C_1$-$C_4$ alkyl; |
| $R_{16}$ and $R_{17}$ | are independently $C_1$-$C_4$ alkyl; |
| $R_{18}$ | is $C_1$-$C_4$ alkyl |
| $R_{19}$ and $R_{20}$ | are independently H or $C_1$-$C_4$ alkyl; |
| $R_{21}$ and $R_{22}$ | are independently H, phenyl: |
| u | is 1 or 2; |
| v | is 0, 1 or 2; |

or a pharmaceutically suitable salt thereof; provided that:

1) when A is $CH_3S$-, then B is not

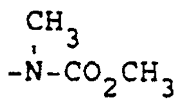

;

2) when A is $CH_3SO_2$-, then B is not

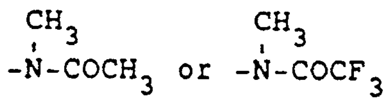

;

3) when A is $H_2NSO_2$- and B is

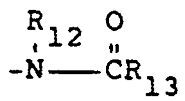

,

then $R_{12}$ is H;

4) when A is -CN, B is not $-N_3$;

5) when A is $(CH_3)_2CH$, B is not $NHCOCH_2Cl$;

**2.** A compound of Claim 1 wherein, for the ℓ, and mixtures of the d and ℓ stereoisomers of the compound,

| | |
|---|---|
| Y | is H; |
| A, | substituted in the para position is $-NO_2$, $-S(O)_nR_1$; |
| $R_1$ | is $C_1$-$C_4$ alkyl optionally substituted with one or more halogen atoms, $-NR_9R_{10}$, $-N_3$; |
| $R_9$ | is H, $C_1$-$C_4$ alkyl or $C_3$-$C_8$ cycloalkyl; |
| $R_{10}$ | is H, $-OR_8$ or $-NR_{11}R_{11a}$; |
| $R_8$ | is H or $C_1$-$C_4$ alkyl; |
| $R_{11}$ and $R_{11a}$ | are independently H or $C_1$-$C_4$ alkyl; |
| n | is 0, 1 or 2; |
| B | is 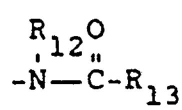 or $N_3$; |
| $R_{12}$ | is H, $C_1$-$C_{10}$ alkyl or $C_3$-$C_8$ cycloalkyl; |
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$: $-SR_{14}$; or $-NR_{19}R_{20}$; |
| $R_{14}$ | is $C_1$-$C_4$ alkyl, optionally substituted with one or more halogen atoms; |
| $R_{15}$ | is $C_1$-$C_4$ alkyl; |

| | |
|---|---|
| $R_{16}$ and $R_{17}$ | are independently $C_1$-$C_4$ alkyl; |
| $R_{18}$ | is $C_1$-$C_4$ alkyl; |
| $R_{19}$ | is H or $C_1$-$C_4$ alkyl; |
| $R_{20}$ | is H or $C_1$-$C_4$ alkyl; |
| u | is 1 or 2; |
| v | is 0, 1 or 2; |

or a pharmaceutically suitable salt thereof; provided that:

1) when A is $CH_3S$-, then B is not

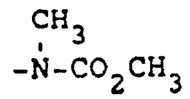;

2) when A is $CH_3SO_2$-, then B is not

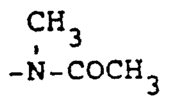 or 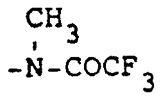;

3) when A is $H_2NSO_2$- and B is

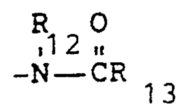

then $R_{12}$ is H.

**3.** A compound of Claim 1 wherein

| | |
|---|---|
| Y | is H; |
| A, | substituted in the para position, is $-S(O)_nR_1$, $NO_2$, |

$NO_2$, 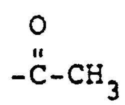,

or $-CH(CH_3)_2$;

| | |
|---|---|
| $R_1$ | is $C_1$-$C_2$ alkyl optionally substituted with one or more halogen atoms or-$NR_9R_{10}$; |
| n | is 0, 1 or 2 when $R_1$ is alkyl or substituted alkyl. |

**4.** A compound of Claim 1 wherein

B is

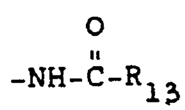;

| | |
|---|---|
| $R_{13}$ | is H, $CH_3$, $OR_{18}$, $CHCl_2$, $CH_2Cl$ or $CH_2OR_{15}$; |
| $R_{15}$ | is $C_1$-$C_4$ alkyl; and |
| $R_{18}$ | is $C_1$-$C_4$ alkyl. |

**5.** A compound of Claim 1 with the stereo-chemical configuration

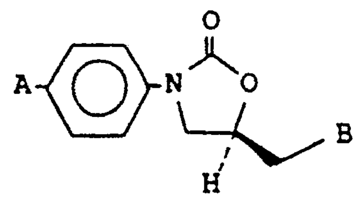

wherein

A is $-S(O)CH_3$, $-S-CH_3$, $-S(O)_2CH_3$, $SO_2NH_2$, $-COCH_3$ or $-CH(CH_3)_2$.

**6.** A compound of Claim 1 with the stereo-chemical formula

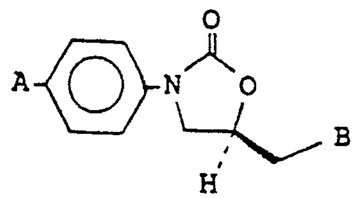

wherein

B is

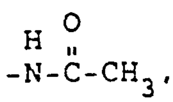, 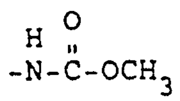 or 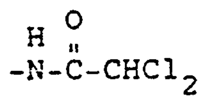.

or

**7.** A compound of Claim 5 wherein

B is

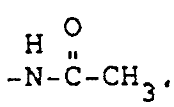, 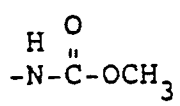 or 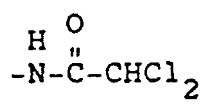.

**8.** A compound of Claim 1 selected from (ℓ)-N-[3-[4-(methylsulfonyl)phenyl]-2-oxooxazolidin-5-ylmethyl)-carbamic acid, methyl ester,

(ℓ)-N-[3-[4-(methylthio)phenyl]-2-oxooxazolidin-5-ylmethyl]carbamic acid, methyl ester,

(ℓ)-N-[3-[4-(methylsulfonyl)phenyl]-2-oxooxazolidin-5-ylmethyl]formamide,

(ℓ)-N-[3-[4-(methylsulfonyl)phenyl]-2-oxooxazolidin-5-ylmethyl]acetamide,

(ℓ)-N-[3-[4-(methylthio)phenyl]-2-oxooxazolidin-5-ylmethyl]acetamide,

(ℓ)-N-[3-[4-(aminosulfonyl)phenyl]-2-oxooxazolidin-5-ylmethyl]acetamide,

(ℓ)-N-3-[4-(methylsulfinyl)phenyl]-2-oxooxazolidin-5-ylmethyl]acetamide,

(ℓ)-2,2-dichloro-N-[3-[4-(methylsulfonyl)phenyl]-2-oxooxazolidin-5-ylmethyl]acetamide,

(ℓ)-N-[3-(4-isopropylphenyl)-2-oxooxazolidin-5-ylmethyl]acetamide, and

(ℓ)-N-[3-(4-acetylphenyl)-2-oxooxazolidin-5-ylmethyl]-acetamide.

**9.** A compound having the formula:

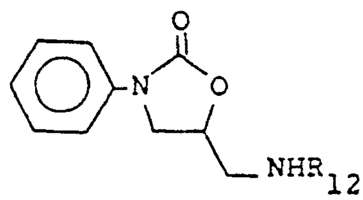

(Ia)

wherein, for the ℓ, and mixtures of the d and ℓ stereoisomers of the compound,
$R_{12}$ is H, $C_1$-$C_{10}$ alkyl or $C_3$-$C_8$ cycloalkyl.

**10.** A compound of the formula

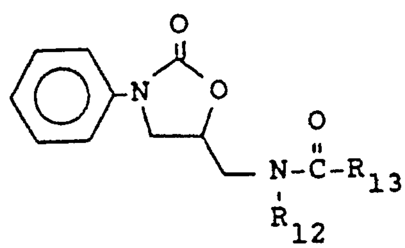

(Ib)

wherein, for the ℓ, and mixtures of the d and ℓ stereoisomers of the compound,

| | |
|---|---|
| $R_{12}$ | is H, $C_1$-$C_{10}$ alkyl or $C_3$-$C_8$ cycloalkyl; |
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$; $-SR_{14}$; $-NR_{19}R_{20}$; or $C(NH_2)R_{21}R_{22}$; |
| $R_{14}$ | is $C_1$-$C_4$ alkyl, optionally substituted with one or more halogen atoms; |
| $R_{15}$ | is $C_1$-$C_4$ alkyl; |
| $R_{16}$ and $R_{17}$ | are independently $C_1$-$C_4$ alkyl; |
| $R_{18}$ | is $C_1$-$C_4$ alkyl; |
| $R_{19}$ and $R_{20}$ | are independently H or $C_1$-$C_4$ alkyl; |
| $R_{21}$ and $R_{22}$ | are independently H, phenyl; |
| v | is 0, 1 or 2. |

**11.** A pharmaceutical composition comprising a suitable pharmaceutical carrier and an antibacterially effective amount of at least one compound of claims 1 to 8.

**12.** A process for preparing compounds of Claim 1 comprising
(a) contacting a compound of the formula

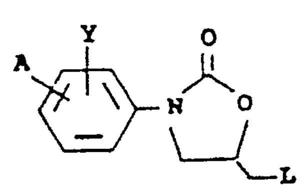

where A and Y are as defined in original Claim 1, and L is any suitable leaving group with azide ion to prepare a compound of the formula

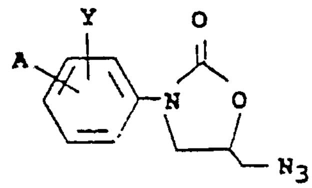 ;

(b) optionally reducing the product of Step (a) to prepare a compound of the formula

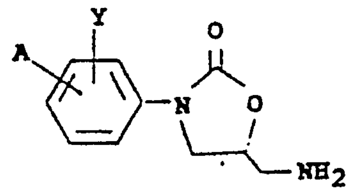 ;

(c) optionally reacting the amine obtained in Step (b) with an acid chloride, anhydride or sulfonyl chloride or with a carboxylic acid in the presence of an appropriate coupling reagent to prepare a compound of the formula

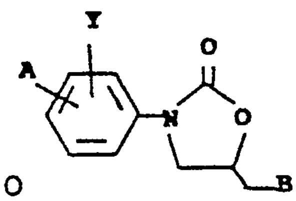

where B is

$$NH\overset{O}{\overset{\|}{C}}R_{13}$$

or $NHS(O)_uR_{14}$;

(d) when A is $SR_1$, optionally oxidizing the product of Step (c) to prepare a compound of the formula

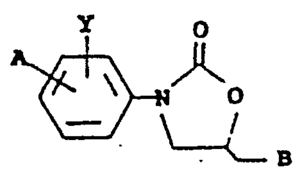

where A is $R_1S(O)_n$ and n is 1 or 2;

(e) when A is H, optionally reacting the product of Step (c) with chlorosulfonic acid followed by an amine, hydrazine, hydroxylamine or azide ion to prepare a compound of the formula

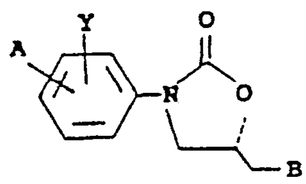

where A is $R_1SO_2$ wherein $R_1$ is $R_{10}R_9N$ or $N_3$.

**13.** A process for preparing compounds of Claim 1 comprising
(a) contacting a compound of the formula

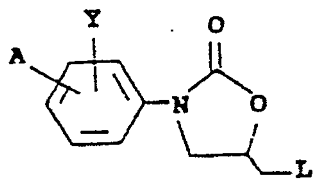

where A and Y are as defined in original Claim 1; and L is any suitable leaving group with a primary amine or ammonia to prepare a compound of the formula

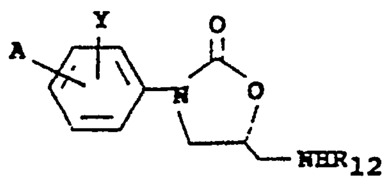 ;

(b) optionally treating the amine obtained in Step (a) according to Steps (c), (d) or (e) above.

**Claims for the following Contracting state: AT**

**1.** A process for preparing a compound of the formula

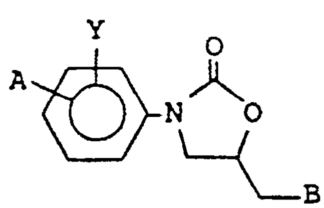

(I)

wherein, for the ℓ, and mixtures of the d and ℓ stereoisomers of the compound,

A is $-NO_2$, $-S(O)_nR_1$, -SH,

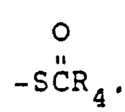

$-COR_5$,

$$-\overset{NR_7}{\overset{\|}{C}}-R_5,$$

$-CN$, $-OR_5$, $-NR_5R_6$,

$$-\overset{R_5}{\overset{|}{N}}COR_4,\quad -\overset{R_5}{\overset{|}{N}}S(O)_nR_4,$$

| | |
|---|---|
| | alkyl of 1 to 5 carbons, optionally substituted with one or more halogen atoms or alkenyl of 2-5 carbons; |
| $R_1$ | is $C_1$-$C_4$ alkyl, optionally substituted with one or more halogen atoms, CN; $-NR_9R_{10}$; $-N_3$; |
| $R_4$ | is alkyl of 1-4 carbons, |
| $R_5$ and $R_6$ | are independently H, alkyl of 1-4 carbons |
| $R_7$ | is $-OR_5$; |
| $R_8$ | is H or alkyl of 1-4 carbons; |
| $R_9$ | is H, $C_1$-$C_4$ alkyl or $C_3$-$C_8$ cycloalkyl; |
| $R_{10}$ | is H, $-OR_8$ or $-NR_{11}R_{11a}$; |
| $R_{11}$ and $R_{11a}$ | are independently H or $C_1$-$C_4$ alkyl; |
| Y | is H or $NO_2$, or A and Y taken together can be $-O(CH_2)_tO-$; |
| n | is 0, 1 or 2; |
| t | is 1, 2 or 3; |
| B | is |

$$-\overset{R_{12}}{\overset{|}{N}}\!-\!\!-\overset{O}{\overset{\|}{C}}-R_{13}$$

| | |
|---|---|
| | or $N_3$; |
| $R_{12}$ | is H, $C_1$-$C_{10}$ alkyl or $C_3$-$C_8$ cycloalkyl; |
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$; $-SR_{14}$; $-CH_2N_3$; $-NR_{19}R_{20}$; or $C(NH_2)R_{21}R_{22}$; |
| $R_{14}$ | is $C_1$-$C_4$ alkyl, optionally substituted with one or more halogen atoms; |
| $R_{15}$ | is $C_1$-$C_4$ alkyl; |
| $R_{16}$ and $R_{17}$ | are independently $C_1$-$C_4$ alkyl; |
| $R_{18}$ | is $C_1$-$C_4$ alkyl |
| $R_{19}$ and $R_{20}$ | are independently H or $C_1$-$C_4$ alkyl; |
| $R_{21}$ and $R_{22}$ | are independently H, phenyl; |
| u | is 1 or 2; |
| v | is 0, 1 or 2; |

or a pharmaceutically suitable salt thereof; provided that:

1) when A is $CH_3S-$, then B is not

$$-\overset{CH_3}{\overset{|}{N}}-CO_2CH_3;$$

2) when A is $CH_3SO_2-$, then B is not

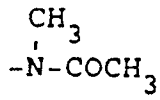 or 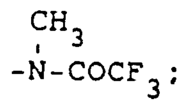

3) when A is $H_2NSO_2$- and B is then $R_{12}$ is H;

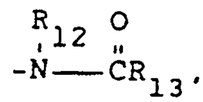

4) when A is -CN, B is not $-N_3$;

5) when A is $(CH_3)_2CH$, B is not $NHCOCH_2Cl$;

which comprises

(a) contacting a compound of the formula

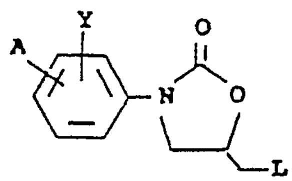

where A and Y are as defined in original Claim 1, and L is any suitable leaving group with azide ion to prepare a compound of the formula

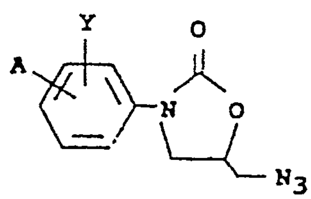 ;

(b) optionally reducing the product of Step (a) to prepare a compound of the formula

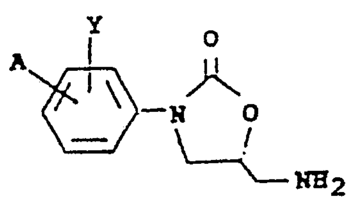 ;

(c) optionally reacting the amine obtained in Step (b) with an acid chloride, anhydride or sulfonyl chloride or with a carboxylic acid in the presence of an appropriate coupling reagent to prepare a compound of the formula

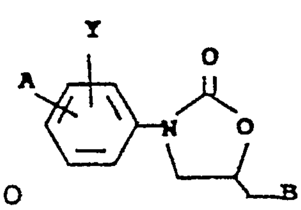

O

where B is

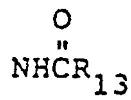

or $NHS(0)_uR_{14}$;

(d) when A is $SR_1$, optionally oxidizing the product of Step (c) to prepare a compound of the formula

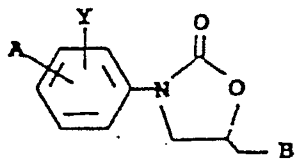

where A is $R_1S(O)_n$ and n is 1 or 2;

(e) when A is H, optionally reacting the product of Step (c) with chlorosulfonic acid followed by an amine, hydrazine, hydroxylamine or azide ion to prepare a compound of the formula

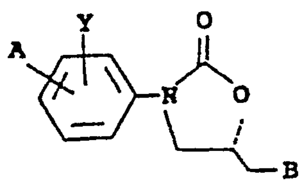

where A is $R_1SO_2$ wherein $R_1$ is $R_{10}R_9N$ or $N_3$.

2. A process of Claim 1 wherein, for the ℓ, and mixtures of the d and ℓ stereoisomers of the compound,

| | |
|---|---|
| Y | is H; |
| A, | substituted in the para position is $-NO_2$, $-S(O)_nR_1$; |
| $R_1$ | is $C_1$-$C_4$ alkyl optionally substituted with one or more halogen atoms, $-NR_9R_{10}$, $-N_3$; |
| $R_9$ | is H, $C_1$-$C_4$ alkyl or $C_3$-$C_8$ cycloalkyl; |
| $R_{10}$ | is H, $-OR_8$ or $-NR_{11}R_{11a}$; |
| $R_8$ | is H or $C_1$-$C_4$ alkyl; |
| $R_{11}$ and $R_{11a}$ | are independently H or $C_1$-$C_4$ alkyl; |
| n | is 0, 1 or 2; |
| B | is 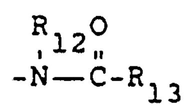 or $N_3$; |
| $R_{12}$ | is H, $C_1$-$C_{10}$ alkyl or $C_3$-$C_8$ cycloalkyl; |
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$; $-SR_{14}$; or $-NR_{19}R_{20}$; |
| $R_{14}$ | is $C_1$-$C_4$ alkyl, optionally substituted with one or more halogen atoms; |
| $R_{15}$ | is $C_1$-$C_4$ alkyl; |
| $R_{16}$ | and $R_{17}$ are independently $C_1$-$C_4$ alkyl; |
| $R_{18}$ | is $C_1$-$C_4$ alkyl; |
| $R_{19}$ | is H or $C_1$-$C_4$ alkyl; |
| $R_{20}$ | is H or $C_1$-$C_4$ alkyl; |
| u | is 1 or 2; |

v is 0, 1 or 2;

or a pharmaceutically suitable salt thereof; provided that:

1) when A is $CH_3S$-, then B is not

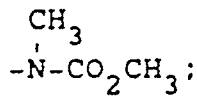

2) when A is $CH_3SO_2$-, then B is not

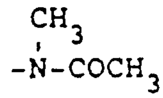

or

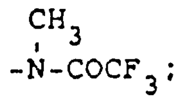

3) when A is $H_2NSO_2$- and B is

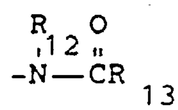

then $R_{12}$ is H.

**3.** A process of Claim 1 wherein

Y is H;

A, substituted in the para position, is

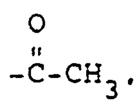

$R_1$ is $C_1$-$C_2$ alkyl optionally substituted with one or more halogen atoms or $-NR_9R_{10}$;

n is 0, 1 or 2 when $R_1$ is alkyl or substituted alkyl;

**4.** A process of Claim 1 wherein

B is

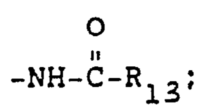

$R_{13}$ is H, $CH_3$, $OR_{18}$, $CHCl_2$, $CH_2Cl$ or $CH_2OR_{15}$;

$R_{15}$ is $C_1$-$C_4$ alkyl; and

$R_{18}$ is $C_1$-$C_4$ alkyl.

**5.** A process of Claim 1 for preparing a compound with the stereochemical configuration

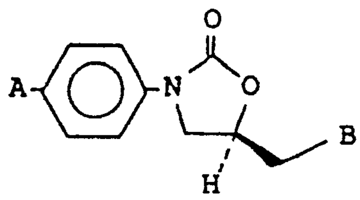

wherein

A is $-S(O)CH_3$, $-S-CH_3$, $-S(O)_2CH_3$, $SO_2NH_2$, $-COCH_3$ or $-CH(CH_3)_2$.

6. A process of Claim 1 for preparing a compound with the stereochemical formula

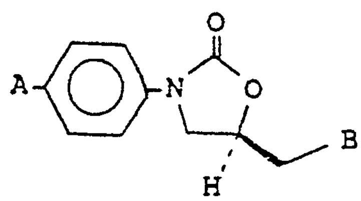

wherein

B is

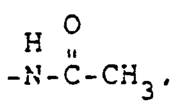, 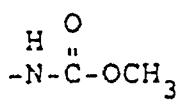 or 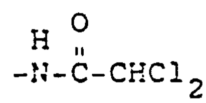.

7. A ccmpound of Claim 5 wherein

B is

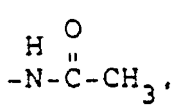, 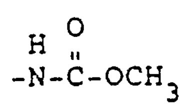 or 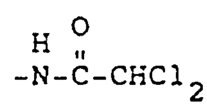.

8. A process of Claim 1 wherein the compounds are selected from (ℓ)-N-[3-[4-(methylsulfonyl)phenyl]-2-oxooxazolidin-5-ylmethyl]-carbamic acid, methyl ester,

(ℓ)-N-[3-[4-(methylthio)phenyl]-2-oxooxazolidin-5-ylmethyl]carbamic acid, methyl ester,

(ℓ)-N-[3-[4-(methylsulfonyl)phenyl]-2-oxooxazolidin-5-ylmethyl]-formamide,

(ℓ)-N-[3-[4-(methylsulfonyl)phenyl]-2-oxooxazolidin-5-ylmethyl]acetamide,

(ℓ)-N-[3-[4-(methylthio)phenyl]-2-oxooxazolidin-5-ylmethyl]acetamide,

(ℓ)-N-[3-[4-(aminosulfonyl)phenyl]-2-oxooxazolidin-S-ylmethyl]acetamide,

(ℓ)-N-[3-[4-(methylsulfinyl)phenyl]-2-oxooxazolidin-5-ylmethyl]acetamide,

(ℓ)-2,2-dichloro-N-[3-[4-(methylsulfonyl)phenyl]-2-oxooxazolidin-5-ylmethyl]acetamide,

(ℓ)-N-[3-(4-isopropylphenyl)-2-oxooxazolidin-5-ylmethyl)acetamide, and

(ℓ)-N-[3-(4-acetylphenyl)-2-oxooxazolidin-5-ylmethyl]acetamide.

9. A process for preparing a compound having the formula:

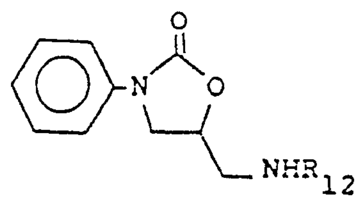

wherein, for the ℓ, and mixtures of the d and ℓ stereoisomers of the compound,

$R_{12}$ is H, $C_1$-$C_{10}$ alkyl or $C_3$-$C_8$ cycloalkyl,

comprising contacting a compound of the formula

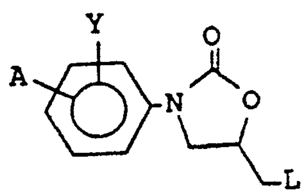

wherein A and Y are H and L is any suitable leaving group with a primary amine or ammonia to prepare a compound of the formula

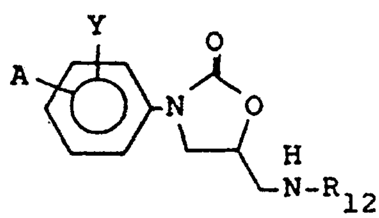

wherein A and Y are H.

10. A process for preparing a compound of the formula

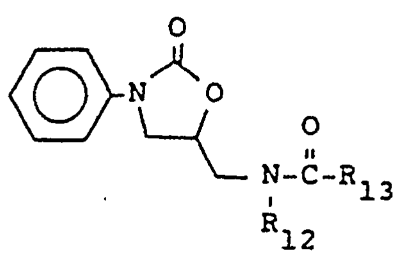

(Ib)

wherein, for the ℓ, and mixtures of the d and ℓ stereoisomers of the compound,

| | |
|---|---|
| $R_{12}$ | is H, $C_1$-$C_{10}$ alkyl or $C_3$-$C_8$ cycloalkyl; |
| $R_{13}$ | is H; $C_1$-$C_4$ alkyl optionally substituted with one or more halogen atoms; $C_2$-$C_4$ alkenyl; $C_3$-$C_4$ cycloalkyl; phenyl; $-CH_2OR_{15}$; $-CH(OR_{16})OR_{17}$; $-CH_2S(O)_vR_{14}$; $-OR_{18}$; $-SR_{14}$; $-NR_{19}R_{20}$; or $C(NH_2)R_{21}R_{22}$; |
| $R_{14}$ | is $C_1$-$C_4$ alkyl, optionally substituted with one or more halogen atoms; |
| $R_{15}$ | is $C_1$-$C_4$ alkyl; |

| | |
|---|---|
| $R_{16}$ and $R_{17}$ | are independently $C_1$-$C_4$ alkyl; |
| $R_{18}$ | is $C_1$-$C_4$ alkyl; |
| $R_{19}$ and $R_{20}$ | are independently H or $C_1$-$C_4$ alkyl; |
| $R_{21}$ and $R_{22}$ | are independently H, phenyl; |
| v | is 0, 1 or 2, |

comprising
contacting a compound of the formula

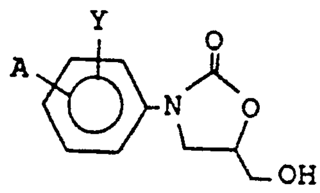

with the appropriate sulfonyl halide or sulfonic anhydride to prepare a compound

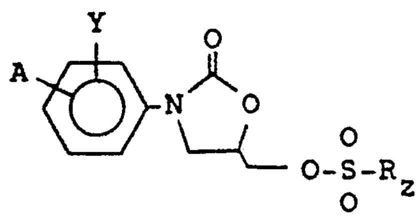

and reacting this compound with the appropriate amide and a catalyst, whereby A and Y in both formulae are H.

**11.** A process for preparing compounds of Claim 1 comprising
(a) contacting a compound of the formula

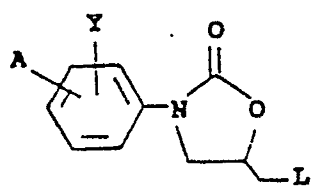

where A and Y are as defined in original Claim 1; and L is any suitable leaving group with a primary amine or ammonia to prepare a compound of the formula

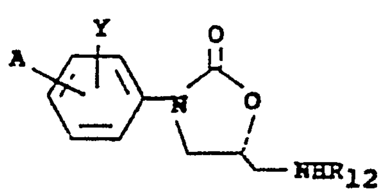

;

(b) optionally treating the amine obtained in Step (a) according to Steps (c), (d) or (e) above.

**12.** A pharmaceutical composition comprising a suitable pharmaceutical carrier and an antibacterially effective amount of at least one compound of claims 1 to 8.

**Revendications**

**Revendications pour les Etats contractants suivants: BE, CH, DE, FR, GB, IT, LT, LU, NL, SE**

**1.** Un dérivé répondant à la formule :

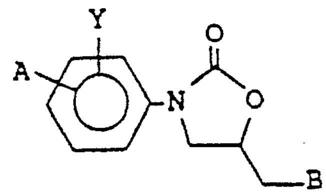

dans laquelle, pour les stéréoisomères ℓ et les mélanges des stéréoisomères d et ℓ du dérivé,

A représente $-NO_2$, $-S(O)_nR_1$, -SH

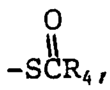, $-COR_5$, 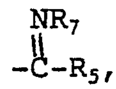,

-CN, $-OR_5$ $-NR_5R_6$,

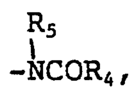, 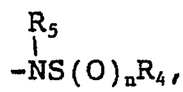,

un radical alkyl renfermant de 1 à 5 atomes de carbone, éventuellement substitué par un ou plusieurs atomes d'halogène ou un radical alkényle renfermant de 2 à 5 atomes de carbone ;

$R_1$ représente un radical alkyl en $C_1$ à $C_4$, éventuellement substitué par un ou plusieurs atomes d'halogène, CN, $-NR_9R_{10}$ ; $-N_3$ ;

$R_4$ représente un radical alkyl renfermant de 1 à 4 atomes de carbone ;

$R_5$ et $R_6$ représentent indépendamment un atome de H, un radical alkyl renfermant de 1 à 4 atomes de carbone ;

$R_7$ représente $-OR_5$ ;

$R_8$ représente un atome de H ou un radical alkyl renfermant de 1 à 4 atomes de carbone ;

$R_9$ représente un atome de H, un radical alkyl en $C_1$ à $C_4$ ou cycloalkyl en $C_3$ à $C_8$ ;

$R_{10}$ représente un atome de H, $-OR_8$ ou $-NR_{11}R_{11a}$,

$R_{11}$ et $R_{11a}$ représentent indépendamment un atome de H ou un radical alkyl en $C_1$ à $C_4$ ;

Y représente H ou $NO_2$, ou bien A et Y pris ensemble peuvent représenter $-O(CH_2)_tO-$ ;

n est égal à 0, 1 ou 2 ;

t est égal à 1, 2 ou 3 ;

B représente

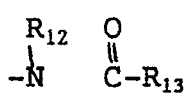

ou $N_3$ ;

$R_{12}$ représente H, un radical alkyl en $C_1$ à $C_{10}$ ou cycloalkyl en $C_3$ à $C_8$ ;

$R_{13}$ représente H, un radical alkyl en $C_1$ à $C_4$ éventuellement substitué par un ou plusieurs atomes d,halogène ; un groupe alkényl en $C_2$ à $C_4$ ; un groupe cycloalkyl en $C_3$ à $C_4$ ; phényl ; $-CH_2OR_{15}$ ; $-CH(OR_{16})OR_{17}$ ; $CH_2s(O)_vR_{14}$ ; $-OR_{18}$ ; $-SR_{14}$ ; $-CH_2N_3$ ; $-NR_{19}R_{20}$ ; ou $C(NH_2)R_{21}R_{22}$ ;

$R_{14}$ représente un radical alkyl en $C_1$ à $C_4$, éventuellement substitué par un ou plusieurs atomes d'halogène ;

$R_{15}$ représente un radical alkyle en $C_1$ à $C_4$ ;

$R_{16}$ et $R_{17}$ représentent indépendamment un radical alkyl en $C_1$ à $C_4$ ;

$R_{18}$ représente un radical alkyle en $C_1$ à $C_4$ ;

$R_{19}$ et $R_{20}$ représentent indépendamment H ou un radical alkyl en $C_1$ à $C_4$ ;

$R_{21}$ et $R_{22}$ représentent indépendamment un atome de H ou un radical phényl ;
u est égal à 1 ou 2 ;
v est égal à 0, 1 ou 2 ;
ou un de leurs sels pharmaceutiquement convenable pourvu que :

1) lorsque A représente $CH_3S$-, alors B ne représente pas

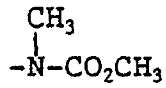 ;

2) lorsque A représente $CH_3SO_2$-, alors B ne représente pas

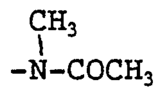 ou 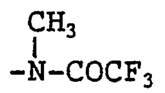 ;

3) quand A est $H_2NSO_2$- et B est

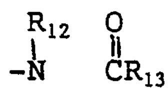,

alors $R_{12}$ représente H ;

4) lorsque A représente -CN, B ne représente pas $-N_3$ ;

5) lorsque A représente$(CH_3)_2CH$, B ne représente pas $NHCOCH_2Cl$.

**2.** Un dérivé selon la revendication 1, caractérisé en ce que pour les stéréoisomères ℓ et les mélanges stéréoisomères d et ℓ du dérivé,

Y représente H ;
A, substitué en position para, représente $-NO_2$, $-S(O)_nR_1$ ;
$R_1$ représente un radical alkyl en $C_1$ à $C_4$ éventuellement substitué par un ou plusieurs atomes d'halogène ou $-NR_9R_{10}$, $-N_3$ ;
$R_9$ représente H, un radical alkyle en $C_1$ à $C_4$ ou cycloalkyl en $C_3$ à $C_8$ ;
$R_{10}$ représente H, $-OR_8$, ou $-NR_{11}R_{11a}$ ;
$R_8$ représente H ou un radical alkyle en $C_1$ à $C_4$ ;
$R_{11}$ et $R_{11a}$ représentent indépendamment H, ou un radical alkyl en $C_1$ à $C_4$ ;
n est égal à 0, 1 ou 2 ;
$R_{12}O$
B représente -N C-$R_{13}$ ; ou $N_3$ ;
$R_{12}$ représente H, un radical alkyl en $C_1$ à $C_{10}$ ou cycloalkyl en $C_1$ à $C_8$ ;
$R_{13}$ représente H, un radical alkyl en $C_1$ à $C_4$ éventuellement substitué par un ou plusieurs atomes d'halogène ; un radical alcényl en $C_2$ à $C_4$, cycloalkyl en $C_3$ à $C_4$, phényl, $CH_2OR_{15}$ ; $-CH(OR_{16})OR_{17}$ ; $-CH_2S(O)_vR_{14}$ ; $-OR_{18}$ ; $-SR_{14}$ ; ou $-NR_{19}R_{20}$ ;
$R_{14}$ représente un radical alkyl en $C_1$ à $C_4$ ; et éventuellement substitué par un ou plusieurs atomes d'halogène ;
$R_{15}$ représente un radical alkyl en $C_1$ à $C_4$ ; et
$R_{16}$ et $R_{17}$ représentent indépendamment un radical alkyl en $C_1$ à $C_4$ ;
$R_{18}$ représente un radical alkyl en $C_1$ à $C_4$.
$R_{19}$ représente H ou un radical alkyl en $C_1$ à $C_4$ ;
$R_{20}$ représente H ou un radical alkyl en $C_1$ à $C_4$ ;
u est 1 ou 2 ;
v est 0, 1 ou 2 ;
ou un de leurs sels pharmaceutiquement acceptable pourvu que :

1) lorsque A représente $CH_3S$-, alors B ne représente pas

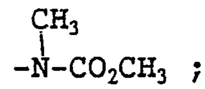

2) lorsque A représente $CH_3SO_2$, alors B ne représente pas

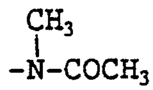 ou 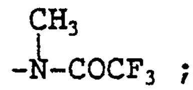

3) quand A est $H_2NSO_2$- et B représente

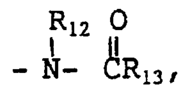

alors $R_{12}$ représente H ;

**3.** Un dérivé selon la revendication 1, caractérisé en ce que :
Y représente H ;
A, substitué en position para, représente -$S(O)_nR_1$, $NO_2$,

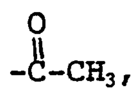

ou -$CH(CH_3)_2$ ;
$R_1$ représente un radical alkyl en $C_1$ à $C_2$ éventuellement substitué par un ou plusieurs atomes d'halogène ou -$NR_9R_{10}$ ;
n est égal à 0, 1 ou 2 lorsque $R_1$ est un radical alkyl ou alkyl substitué.

**4.** Un dérivé selon la revendication 1, caractérisé en ce que : B représente

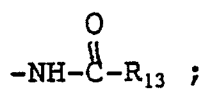

$R_{13}$ représente H, $CH_3$, $OR_{18}$, $CHCl_2$, $CH_2Cl$ ou $CH_2OR_{15}$ ;
$R_{15}$ représente un radical alkyl en $C_1$ à $C_4$ ; et
$R_{18}$ représente un radical alkyl en $C_1$ à $C_4$.

**5.** Un dérivé selon la revendication 1, présentant la configuration stéréochimique :

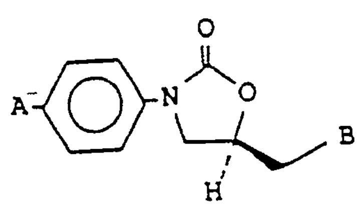

dans laquelle
A représente $S(O)CH_3$, -$S$-$CH_3$, $S(O)_2CH_3$, $SO_2NH_2$, -$COCH_3$ ou -$CH(CH_3)_2$.

**6.** Un dérivé selon la revendication 1, présentant la formule stéréochimique :

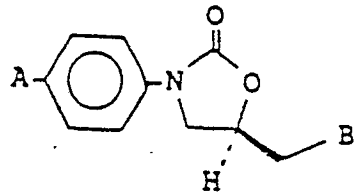

dans laquelle B représente

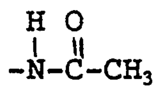 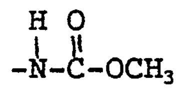 ou 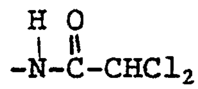

7. Un dérivé selon la revendication 5, caractérisé en ce que B représente

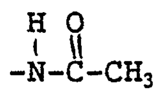 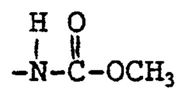 ou 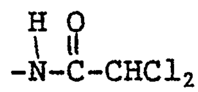

8. Un dérivé selon la revendication 1, choisi parmi :

- ester méthylique d'acide (ℓ)-N-[3[4-(méthylsulfonyl)phényl]-2-oxooxazolidin-5-ylméthyl]-carbamique ;
- ester méthylique d'acide (ℓ)-N-[3-[4-(méthylthio)phényl]-2-oxooxazolidin-5-ylméthyl]carbamique ;
- (ℓ)-N-[3-[4-(méthylsulfonyl)phényl]2-oxooxazolidin-5-ylméthyl]formamide ;
- (ℓ )N-[3[4-(méthylsulfonyl)phényl]-2-oxooxazolidin-5-ylméthyl]acétamide ;
- (ℓ)-N-[3-[4-(méthylthio)phényl]-2-oxooxazolidin-5-ylméthyl]acétamide ;
- (ℓ)-N-[3-[4-(aminosulfonyl)phényl]-2-oxooxazolidin-5-ylméthyl]acétamide ;
- (ℓ)-N-[3-[4-(méthylsulfinyl)phényl]-2-oxooxazolidin-5-ylméthyl]acétamide ;
- (ℓ)-2,2-dichloro-N-[3-[4-(méthylsulfonyl)phényl]-2-oxooxazolidin-5-ylméthyl]acétamide ;
- (ℓ)-N-[3-(4-(isopropylphényl)-2-oxooxazolidin-5-ylméthyl]acétamide ; et
- (ℓ)-N-[3-(4-(acétylphényl)-2-oxooxazolidin-5-ylméthyl]acétamide ;

9. Un dérivé répondant à la formule :

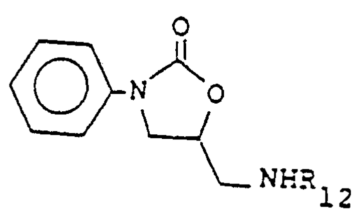

dans laquelle, pour le stéréoisomère ℓ et les mélanges stéréoisomères d et ℓ du dérivé,
$R_{12}$ représente H, un radical alkyl en $C_1$ à $C_{10}$ ou cycloalkyl en $C_3$ à $C_8$.

10. Dérivé répondant à la formule :

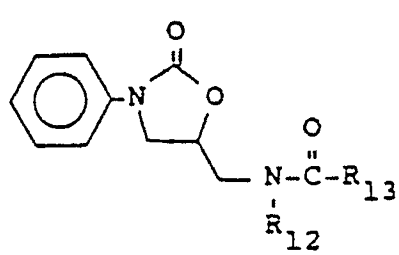

dans laquelle, pour le stéréoisomère ℓ et les mélanges du stéréoisomère d et ℓ du dérivé,

$R_{12}$ représente un atome d'H, un radical alkyl en $C_1$ à $C_{10}$ ou cycloalkyl en $C_3$ à $C_8$ ;

$R_{13}$ représente un atome d'H, un radical alkyl en $C_1$ à $C_4$ éventuellement substitué par un ou plusieurs atomes d'halogène ; alkényl en $C_2$ à $C_4$ ; cycloalkyl en $C_3$ à $C_4$ ; phényl ; $-CH_2OR_{15}$ ; $-CH(OR_{16})OR_{17}$ ; $CH_2S(O)_vR_{14}$ ; $-OR_{18}$ ; $-SR_{14}$ ; $-NR_{19}R_{20}$ ; ou $C(NH_2)R_{21}R_{22}$ :

$R_{14}$ représente un radical alkyl en $C_1$ à $C_4$, éventuellement substitué par un ou plusieurs atomes d'halogène ;

$R_{15}$ représente un radical alkyle en $C_{1\ \text{à}\ c}4$ ;

$R_{16}$ et $R_{17}$ représentent indépendamment un radical alkyl en $C_1$ à $C_4$ ;

$R_{18}$ représente un radical alkyle en $C_1$ à $C_4$ ;

$R_{19}$ et $R_{20}$ représentent indépendamment un atome d'H ou un radical alkyl en $C_1$ à $C_4$ ;

$R_{21}$ et $R_{22}$ représentent indépendamment un atome de H, le radical phényl ; et

v est égal à 0, 1 ou 2.

**11.** Une composition pharmaceutique comprenant un support pharmaceutique approprié et une quantité antibactérienne efficace d'au moins un dérivé selon les revendications 1 à 8.

**12.** Procédé de préparation de dérivés selon la revendication 1, comprenant les étapes suivantes :

(a) mise en contact d'un dérivé de formule :

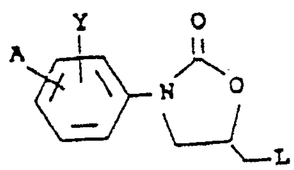

dans laquelle A et Y sont tels que définis dans la revendication 1 originale et L représente n'importe quel groupe partant convenable avec l'ion azoture pour préparer un dérivé répondant à la formule :

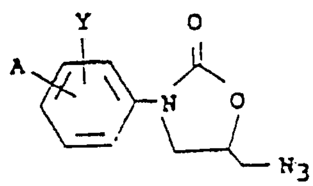

(b) réduction éventuelle du produit d'étape (a) pour préparer un dérivé répondant à la formule :

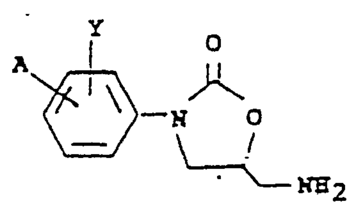

(c) éventuellement mise en réaction de l'amine obtenue dans l'étape (b) avec un chlorure d'acide, un anhydride d'acide ou du chlorure de sulfonyle ou avec un acide carboxylique en présence d'un réactif de couplage approprié pour préparer un dérivé répondant à la formule

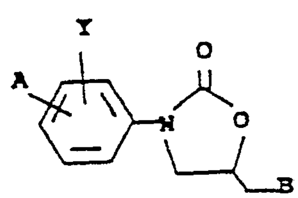

dans laquelle B est égal à

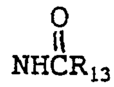

ou $NHS(O)_uR_{14}$ ;
(d) lorsque A représente $SR_1$, on oxyde éventuellement le produit d'étape (c) pour préparer un dérivé répondant à la formule

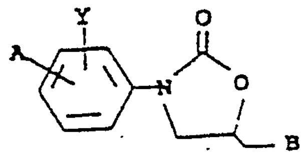

dans laquelle A représente $R_1S(O)_n$ et n est égal à 1 ou 2 ;
(e) lorsque A représente un atome d'H, on fait éventuellement réagir le produit d'étape (c) avec de l'acide chlorosulfonique et ensuite avec une amine hydrazine, hydroxylamine ou un ion azoture pour préparer un dérivé de formule

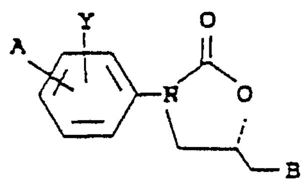

dans laquelle A représente $R_1SO_2$, dans lequel $R_1$ représente $R_{10}R_9$ N ou $N_3$.

**13.** Un procédé de préparation du dérivé selon la revendication 1, comprenant les étapes suivantes :
(a) mise en contact d'un dérivé de formule :

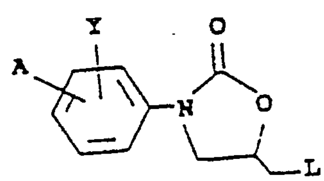

dans laquelle A et Y sont tels que définis dans la revendication 1 originale et L représente un groupe partant approprié quelconque avec une amine primaire ou de l'ammoniac pour préparer un dérivé de formule :

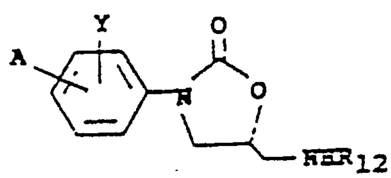

(b) éventuellement, traitement de l'amine obtenue à l'étape (a) selon les étapes (c), (d) ou (e) ci-dessus.

**Revendications pour l'Etat contractant suiviant: AT**

1. Un procédé de préparation d'un dérivé de formule :

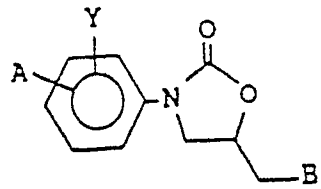

dans laquelle, pour les stéréoisomères ℓ et les mélanges des stéréoisomères d et ℓ du dérivé,
A représente $-NO_2$, $-S(O)_nR_1$, -SH

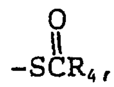

$-COR_5$,

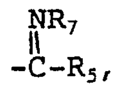

-CN, $-OR_5$ $-NR_5R_6$,

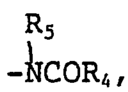

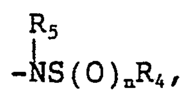

un radical alkyl renfermant de 1 à 5 atomes de carbone, éventuellement substitué par un ou plusieurs atomes d'halogène ou un radical alkényle renfermant de 2 à 5 atomes de carbone ;
$R_1$ représente un radical alkyl en $C_1$ à $C_4$, éventuellement substitué par un ou plusieurs atomes d'halogène, CN, $-NR_9R_{10}$ ; $-N_3$ ;
$R_4$ représente un radical alkyl renfermant de 1 à 4 atomes de carbone ;
$R_5$ et $R_6$ représentent indépendamment un atome de H, un radical alkyl renfermant de 1 à 4 atomes de carbone ;
$R_7$ représente $-OR_5$ ;
$R_8$ représente un atome de H ou un radical alkyl renfermant de 1 à 4 atomes de carbone ;
$R_9$ représente un atome de H, un radical alkyl en $C_1$ à $C_4$ ou cycloalkyl en $C_3$ à $C_8$ ;
$R_{10}$ représente un atome de H, $-OR_8$ ou $-NR_{11}R_{11a}$,
$R_{11}$ et $R_{11a}$ représentent indépendamment un atome de H ou un radical alkyl en $C_1$ à $C_4$ ;
Y représente H ou $NO_2$, ou bien A et Y pris ensemble peuvent représenter $-O(CH_2)_tO-$ ;
n est égal à 0, 1 ou 2 ;
t est égal à 1, 2 ou 3 ; B représente

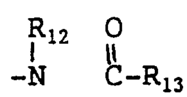

ou $N_3$ ;
$R_{12}$ représente H, un radical alkyl en $C_1$ à $C_{10}$ ou cycloalkyl en $C_3$ à $C_8$ ;
$R_{13}$ représente H, un radical alkyl en $C_1$ à $C_4$ éventuellement substitué par un ou plusieurs atomes d,halogène ; un groupe alkényl en $C_2$ à $C_4$ ; un groupe cycloalkyl en $C_3$ à $C_4$ ; phényl ; $-CH_2OR_{15}$ ; $-CH(OR_{16})OR_{17}$ ; $CH_2S(O)_vR_{14}$ ; $-OR_{18}$ ; $-SR_{14}$ ; $-CH_2N_3$ ; $-NR_{19}R_{20}$ ; ou $C(NH_2)R_{21}R_{22}$ ;
$R_{14}$ représente un radical alkyl en $C_1$ à $C_4$, éventuellement substitué par un ou plusieurs atomes

d'halogène ;
$R_{15}$ représente un radical alkyle en $C_1$ à $C_4$ ; $R_{16}$ et $R_{17}$ représentent indépendamment un radical alkyl en $C_1$ à $C_4$ ;
$R_{18}$ représente un radical alkyle en $C_1$ à $C_4$ ;
$R_{19}$ et $R_{20}$ représentent indépendamment H ou un radical alkyl en $C_1$ à $C_4$ ;
$R_{21}$ et $R_{22}$ représentent indépendamment un atome de H ou un radical phényl ;
u est égal à 1 ou 2 ;
v est égal à 0, 1 ou 2 ;
ou un de leurs sels pharmaceutiquement convenable pourvu que :

1) lorsque A représente $CH_3S$-, alors B ne représente pas

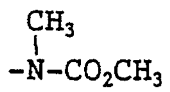 ;

2) lorsque A représente $CH_3SO_2$, alors B ne représente pas

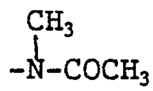 ou 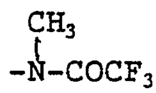 ;

3) quand A est $H_2NSO_2$- et B est

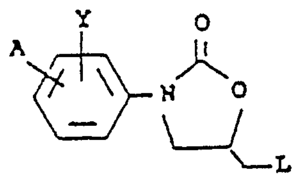

alors $R_{12}$ représente H ;
4) lorsque A représente -$CN_1$ B ne représente pas -$N_3$ ;
5) lorsque A représente$(CH_3)_2CH$, B ne représente pas $NHCOCH_2Cl$. qui comprend les étapes suivantes :

(a) mise en contact d'un dérivé de formule :

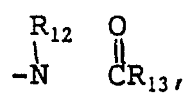

dans laquelle A et Y sont tels que définis dans la revendication 1 originale et L représente n'importe quel groupe partant convenable avec l'ion azoture pour préparer un dérivé répondant à la formule :

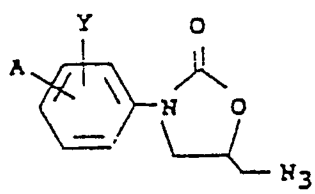

(b) réduction éventuelle du produit d'étape (a) pour préparer un dérivé répondant à la formule :

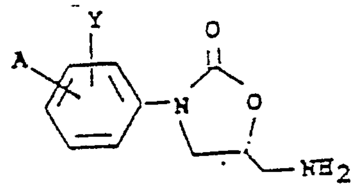

(c) éventuellement mise en réaction de l'amine obtenue dans l'étape (b) avec un chlorure d'acide, un anhydride d'acide ou du chlorure de sulfonyle ou avec un acide carboxylique en présence d'un réactif de couplage approprié pour préparer un dérivé répondant à la formule

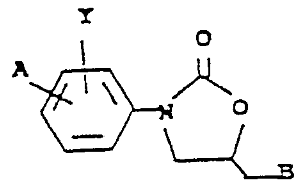

dans laquelle B est égal à

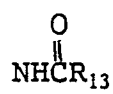

ou $NHS(O)_uR_{14}$ ;

(d) lorsque A représente $SR_1$, on oxyde éventuellement le produit d'étape (c) pour préparer un dérivé répondant à la formule

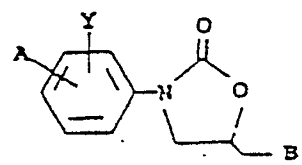

dans laquelle A représente $R_1S(O)_n$ et n est égal à 1 ou 2 ;

(e) lorsque A représente H, on fait éventuellement réagir le produit d'étape (c) avec de l'acide chlorosulfonique et ensuite avec une amine, hydrazine, hydroxylamine ou un ion azoture pour préparer un dérivé de formule

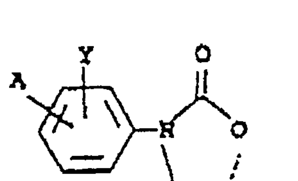

dans laquelle A représente $R_1SO_2$, dans lequel $R_1$ représente $R_{10}R_9$, N ou $N_3$.

2. Un dérivé selon la revendication 1, caractérisé en ce que pour les stéréoisomères ℓ et les mélanges stéréoisomères d et ℓ du dérivé,
Y représente un atome d'H ;
A, substitué en position para, représente $-NO_2$, $-S(O)_nR_1$ ;
$R_1$ représente un radical alkyl en $C_1$ à $C_4$ éventuellement substitué par un ou plusieurs atomes d'halogène ou $-NR_9R_{10}$, $-N_3$ ;
$R_9$ représente H, un radical alkyle en $C_1$ à $C_4$ ou cycloalkyl en $C_3$ à $C_8$ ;

$R_{10}$ représente H, $-OR_8$, ou $-NR_{11}R_{11a}$ ;
$R_8$ représente H ou un radical alkyle en $C_1$ à $C_4$ ;
$R_{11}$ et $R_{11a}$ représentent indépendamment H, ou un radical alkyl en $C_1$ à $C_4$ ;
n est égal à 0, 1 ou 2 ;
B représente

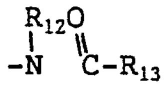 ;

ou $N_3$ ;
$R_{12}$ représente H, un radical alkyl en $C_1$ à $C_{10}$ ou cycloalkyl en $C_3$ à $C_8$ ;
$R_{13}$ représente H un radical alkyl en $C_1$ à $C_4$ éventuellement substitué par un ou plusieurs atomes d'halogène ; un radical alcényl en $C_2$ à $C_4$, cycloalkyl en $C_3$ à $C_4$, phényl, $CH_2OR_{15}$, $-CH(OR_{16})OR_{17}$ ; $-CH_2S(O)_vR_{14}$ ; $-OR_{18}$ ; $-SR_{14}$ ; ou $-NR_{19}R_{20}$ ;
$R_{14}$ représente un radical alkyl en $C_1$ à $C_4$ ; et éventuellement substitué par un ou plusieurs atomes d'halogène ;
$R_{15}$ représente un radical alkyl en $C_1$ à $C_4$ ; et $R_{16}$ et $R_{17}$ représentent indépendamment un radical alkyl en $C_1$ à $C_4$ ;
$R_{18}$ représente un radical alkyl en $C_1$ à $C_4$.
$R_{19}$ représente H ou un radical alkyl en $C_1$ à $C_4$ ;
$R_{20}$ représente H ou un radical alkyl en $C_1$ à $C_4$ ;
u est 1 ou 2 ;
v est 0, 1 ou 2 ;
ou un de leurs sels pharmaceutiquement acceptable pourvu que :

1) lorsque A représente $CH_3S$-, alors B représente

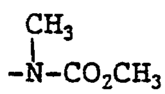 ;

2) lorsque A représente $CH_3SO_2$, alors B ne représente pas

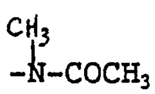 ou 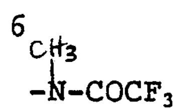 ;

3) quand A est $H_2NSO_2$- et B est

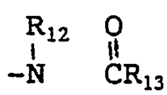,

alors $R_{12}$ représente H ;

**3.** Un procédé selon la revendication 1, caractérisé en ce que :
Y représente un atome d'H ;
A, substitué en position para, représente $-S(O)_nR_1$, $NO_2$,

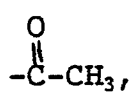,

ou $-CH(CH_3)_2$ ;
$R_1$ représente un radical alkyl en $C_1$ à $C_2$ éventuellement substitué par un ou plusieurs atomes

d'halogène ou $-NR_9R_{10}$ ;
n est égal à 0, 1 ou 2 lorsque $R_1$ est un radical alkyl ou alkyl substitué.

**4.** Un procédé selon la revendication 1, caractérisé en ce que : B représente

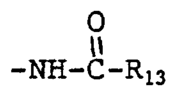 ;

$R_{13}$ représente H, $CH_3$, $OR_{18}$, $CHCl_2$, $CH_2Cl$ ou $CH_2OR_{15}$ ;
$R_{15}$ représente un radical alkyl en $C_1$ à $C_4$ ; et
$R_{18}$ représente un radical alkyl en $C_1$ à $C_4$.

**5.** Un procédé selon la revendication 1, de préparation d'un dérivé présentant la configuration stéréochimique :

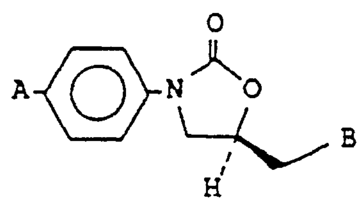

dans laquelle
A représente $S(O)CH_3$, $-S-CH_3$, $-S(O)_2CH_3$, $SO_2NH_2$, $COCH_3$ ou $CH(CH_3)_2$.

**6.** Un procédé selon la revendication 1, de préparation d'un dérivé présentant la formule stéréochimique :

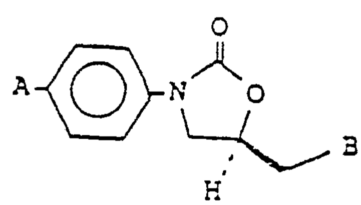

dans laquelle B représente

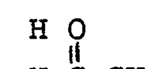 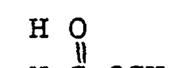 ou 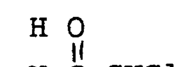

**7.** Un dérivé selon la revendication 5, dans lequel B représente

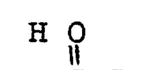 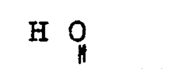 ou 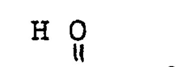

**8.** Un procédé selon la revendication 1, dans lequel les dérivés sont choisis parmi :
- ester méthylique d'acide (ℓ)-N-[3-[4-(méthylsulfonyl)-phényl]-2-oxooxazolidin-5-ylméthyl]-carbamique ;

- ester méthylique d'acide (ℓ)-N-[3-[4-(méthylthio)-phényl]-2-oxooxazolidin-5-ylméthyl]carbamique ;
- (ℓ)-N-[3-[4-(méthylsulfonyl)phényl]-2-oxooxazolidin-5-ylméthyl]formamide ;
- (ℓ)-N-[3-[4-(méthylsulfonyl)phényl]-2-oxooxazolidin-5-ylméthyl]acétamide ;
- (ℓ)-N-[3-[4-(méthylthio)phényl]-2-oxooxazolidin-5-ylméthyl]acétamide ;
- (ℓ)-N-[3-[4-(aminosulfonyl)phényl]-2-oxooxazolidin-5-ylméthyl]acétamide ;
- (ℓ)-N-[3-[4-(méthylsulfinyl)phényl]-2-oxooxazolidin-5-ylméthyl]acétamide ;
- (ℓ)-2,2-dichloro-N-[3-[4-(méthylsulfonyl)phényl]-2-oxooxazolidin-5-ylméthyl]acétamide ;
- (ℓ)-N-[3-(4-(isopropylphényl)-2-oxooxazolidin-5-ylméthyl]acétamide ; et
- (ℓ)-N-[3-(4-acétylphényl)-2-oxooxazolidin-5-ylméthyl]acétamide ;

**9.** Un procédé de préparation d'un dérivé répondant à la formule :

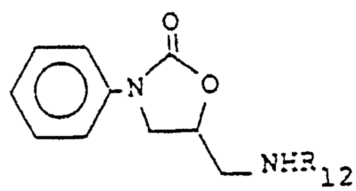

dans laquelle, pour le stéréoisomère ℓ et les mélanges stéréoisomères d et ℓ du dérivé,
$R_{12}$ représente H, un radical alkyl en $C_1$ à $C_{10}$ ou cycloalkyl en $C_3$ à $C_8$,
comprenant les étapes suivantes :
mise en contact d'un dérivé de formule :

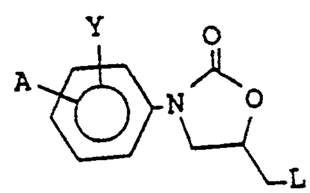

dans laquelle A et Y sont un atome d'H et L représente n'importe quel groupe partant convenable quelconque avec une amine primaire ou de l'ammoniac pour préparer un dérivé de formule :

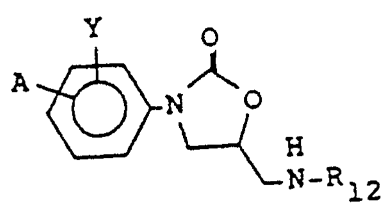

dans laquelle A et Y représentent un atome d'H.

**10.** Un procédé de préparation d'un dérivé de formule :

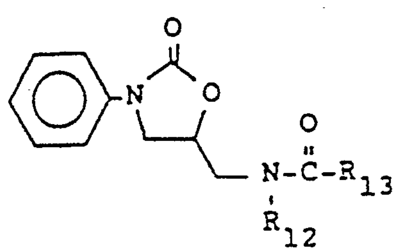

dans laquelle pour les stéréoisomères ℓ et les mélanges stéréoisomères d et ℓ du dérivé,
$R_{12}$ représente H, un radical alkyl en $C_1$ à $C_{10}$ ou cycloalkyl en $C_3$ à $C_8$ ;
$R_{13}$ représente H, un radical alkyl en $C_1$ à $C_4$ éventuellement substitué par un ou plusieurs atomes d'halogène ; un radical alcényl en $C_2$ à $C_4$, cycloalkyl en $C_3$ à $C_4$, phényl, $CH_2OR_{15}$, $-CH(OR_{16})OR_{17}$ ; $-CH_2S(O)_vR_{14}$ ; $-OR_{18}$ ; $-SR_{14}$ ;
$-NR_{19}R_{20}$ ; ou $C(NH_2)R_{21}R_{22}$ ;
$R_{14}$ représente un radical alkyl en $C_1$ à $C_4$ ; et éventuellement substitué par un ou plusieurs atomes d'halogène ;
$R_{15}$ représente un radical alkyl en $C_1$ à $C_4$ ; et $R_{16}$ et $R_{17}$ représentent indépendamment un radical alkyl en $C_1$ à $C_4$ ;
$R_{18}$ représente un radical alkyl en $C_1$ à $C_4$ ;
$R_{19}$ et $R_{20}$ représentent indépendamment H ou un radical alkyl en $C_1$ à $C_4$ ;
$R_{21}$ et $R_{22}$ représentent indépendamment H ou un radical phényle ;
v est égal à 0, 1 ou 2,
comprenant les étapes suivantes :

On met en contact un dérivé de formule :

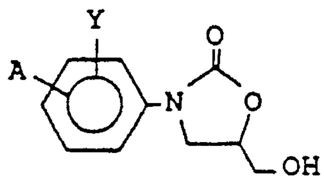

avec l'halogénure de sulfonyle ou l'anhydride sulfonique approprié pour préparer un dérivé

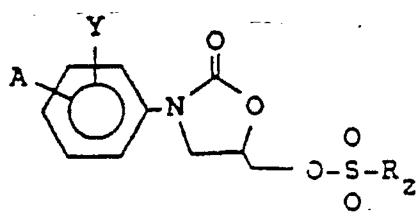

ensuite, on fait réagir ce dérivé avec l'amide appropriée et un catalyseur, A et Y dans les deux formules représentant H.

**11.** Un procédé de préparation du dérivé selon la revendication 1, comprenant le étapes suivantes :

(a) mise en contact d'un dérivé de formule :

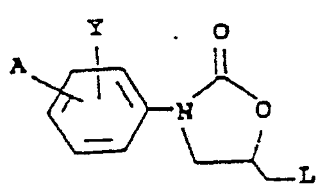

dans laquelle A et Y sont tels que définis dans la revendication 1 originale et L représente un groupe partant convenable quelconque avec une amine primaire ou de l'ammoniac pour préparer un dérivé de formule :

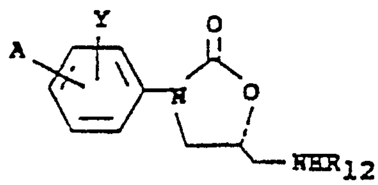

(b) éventuellement, traitement de l'amine obtenue à l'étape (a) selon les étapes (c), (d) ou (e) ci-dessus.

**12.** Une composition pharmaceutique comprenant un support pharmaceutique convenable et une quantité efficace antibactérienne d'au moins un dérivé selon les revendications 1 à 8.

**Patentansprüche**

**Patentansprüche für folgende Vertragsstaaten: BE, CH, DE, FR, GN, IT, LI, LU, NL, SE**

**1.** Verbindung der Formel

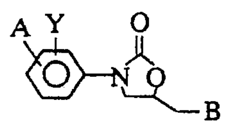 ,

(I)

in der für die ℓ- und Gemische der d- und ℓ-Stereoisomeren der Verbindung

| | |
|---|---|
| A | $-NO_2$, $-S(O)_nR_1$, -SH, |
| | 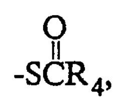 |
| | $-COR_5$, |
| | 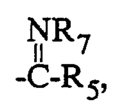 |
| | -CN, $-OR_5$, $-NR_5R_6$, |
| | 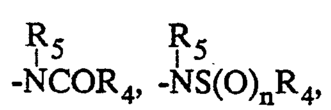 |
| | Alkyl mit 1 bis 5 Kohlenstoff-Atomen, das gegebenenfalls mit einem oder mehreren Halogen-Atomen substituiert ist, oder Alkenyl mit 2 bis 5 Kohlenstoff-Atomen ist; |
| $R_1$ | $C_1$-$C_4$-Alkyl, das gegebenenfalls mit einem oder mehreren Halogen-Atomen substituiert ist, CN, $-NR_9R_{10}$, $-N_3$ ist; |
| $R_4$ | Alkyl mit 1 bis 4 Kohlenstoff-Atomen ist; |
| $R_5$ und $R_6$ | unabhängig voneinander H, Alkyl mit 1 bis 4 Kohlenstoff-Atomen sind; |
| $R_7$ | $-OR_5$ ist; |
| $R_8$ | H oder Alkyl mit 1 bis 4 Kohlenstoff-Atomen ist; |
| $R_9$ | H, $C_1$-$C_4$-Alkyl oder $C_3$-$C_8$-Cycloalkyl ist; |
| $R_{10}$ | H, $-OR_8$ oder $-NR_{11}R_{11a}$ ist; |
| $R_{11}$ und $R_{11a}$ | unabhängig voneinander H oder $C_1$-$C_4$-Alkyl sind; |

| | |
|---|---|
| Y | H oder $NO_2$ ist, oder A und Y zusammen genommen $-O(CH_2)_tO-$ sein können; |
| n | 0, 1 oder 2 ist; |
| t | 1, 2 oder 3 ist; |
| B | 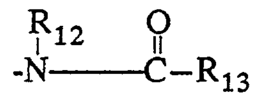 oder $N_3$ ist; |
| $R_{12}$ | H, $C_1$-$C_{10}$-Alkyl oder $C_3$-$C_8$-Cycloalkyl ist; |
| $R_{13}$ | H, $C_1$-$C_4$-Alkyl, das gegebenenfalls mit einem oder mehreren Halogen-Atomen substituiert ist, $C_2$-$C_4$-Alkenyl, $C_3$-$C_4$-Cycloalkyl, Phenyl, $-CH_2OR_{15}$, $-CH(OR_{16})$-$OR_{17}$, $-CH_2S(O)_vR_{14}$, $-OR_{18}$, $-SR_{14}$, $-CH_2N_3$, $-NR_{19}R_{20}$ oder $C(NH_2)R_{21}R_{22}$ ist; |
| $R_{14}$ | $C_1$-$C_4$-Alkyl ist, das gegebenenfalls mit einem oder mehreren Halogen-Atomen substituiert ist; |
| $R_{15}$ | $C_1$-$C_4$-Alkyl ist, |
| $R_{16}$ und $R_{17}$ | unabhängig voneinander $C_1$-$C_4$-Alkyl sind, |
| $R_{18}$ | $C_1$-$C_4$-Alkyl ist, |
| $R_{19}$ und $R_{20}$ | unabhängig voneinander H oder $C_1$-$C_4$-Alkyl sind, |
| $R_{21}$ und $R_{22}$ | unabhängig voneinander H, Phenyl sind; |
| u | 1 oder 2 ist; |
| v | 0, 1 oder 2 ist; |

oder ein pharmazeutisch geeignetes Salz derselben, mit der Maßgabe, daß

1) wenn A $CH_3S$- ist, dann B nicht

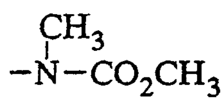

ist:

2) wenn A $CH_3SO_2$- ist, dann B nicht

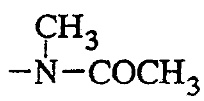 oder 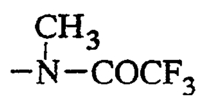

ist;

3) wenn A $H_2NSO_2$- ist und B

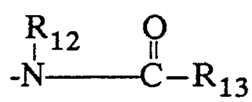

ist, dann $R_{12}$ H ist;

4) wenn A -CN ist, dann B nicht $-N_3$ ist;

5) wenn A $(CH_3)_2CH$ ist, dann B nicht $NHCOCH_2Cl$ ist.

**2.** Verbindung nach Anspruch 1, worin für die ℓ- und Gemische der d- und ℓ-Stereoisomeren der Verbindung

| | |
|---|---|
| Y | H ist; |
| A, | substituiert in der para-Stellung, $-NO_2$, $-S(O)_nR_1$ ist; |
| $R_1$ | $C_1$-$C_4$-Alkyl, das gegebenenfalls mit einem oder mehreren Halogen-Atomen substituiert ist, $-NR_9R_{10}$, $-N_3$ ist; |
| $R_9$ | H, $C_1$-$C_4$-Alkyl oder $C_3$-$C_8$-Cycloalkyl ist; |
| $R_{10}$ | H, $-OR_8$ oder $-NR_{11}R_{11a}$ ist; |

$R_8$ H oder $C_1$-$C_4$-Alkyl ist;
$R_{11}$ und $R_{11a}$ unabhängig voneinander H oder $C_1$-$C_4$-Alkyl sind;
n 0, 1 oder 2 ist;
B

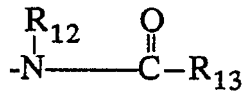

oder $N_3$ ist;
$R_{12}$ H, $C_1$-$C_{10}$-Alkyl oder $C_3$-$C_8$-Cycloalkyl ist;
$R_{13}$ H, $C_1$-$C_4$-Alkyl, das gegebenenfalls mit einem oder mehreren Halogen-Atomen substituiert ist, $C_2$-$C_4$-Alkenyl, $C_3$-$C_4$-Cycloalkyl, Phenyl, $-CH_2OR_{15}$, $-CH(OR_{16})$-$OR_{17}$, $-CH_2S(O)_vR_{14}$, $-OR_{18}$, $-SR_{14}$ oder $-NR_{19}R_{20}$ ist;
$R_{14}$ $C_1$-$C_4$-Alkyl ist, das gegebenenfalls mit einem oder mehreren Halogen-Atomen substituiert ist;
$R_{15}$ $C_1$-$C_4$-Alkyl ist,
$R_{16}$ und $R_{17}$ unabhängig voneinander $C_1$-$C_4$-Alkyl sind,
$R_{18}$ $C_1$-$C_4$-Alkyl ist,
$R_{19}$ H oder $C_1$-$C_4$-Alkyl ist;
$R_{20}$ H oder $C_1$-$C_4$-Alkyl ist;
u 1 oder 2 ist;
v 0, 1 oder 2 ist;

oder ein pharmazeutisch geeignetes Salz derselben, mit der Maßgabe, daß

1) wenn A $CH_3S$- ist, dann B nicht

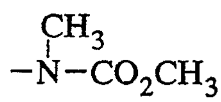

ist;

2) wenn A $CH_3SO_2$- ist, dann B nicht

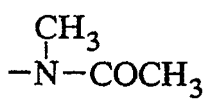 oder 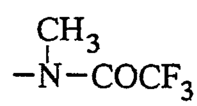

ist;

3) wenn A $H_2NSO_2$- ist und B

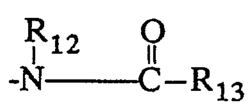

ist, dann $R_{12}$ H ist.

**3.** Verbindung nach Anspruch 1, worin

Y H ist;
A, substituiert in der para-Stellung, $-S(O)_nR_1$, $-NO_2$,

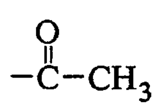

oder $-CH(CH_3)_2$ ist;

R $C_1$-$C_2$-Alkyl, das gegebenenfalls mit einem oder mehreren Halogen-Atomen substituiert ist, oder -$NR_9R_{10}$ ist;

n 0, 1 oder 2 ist, wenn $R_1$ Alkyl oder substituiertes Alkyl ist.

**4.** Verbindung nach Anspruch 1, worin B

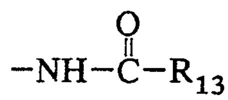

ist;

$R_{13}$ H, $CH_3$, $OR_{18}$, $CHCl_2$, $CH_2Cl$ oder $CH_2OR_{15}$ ist;

$R_{15}$ $C_1$-$C_4$-Alkyl ist; und

$R_{18}$ $C_1$-$C_4$-Alkyl ist.

**5.** Verbindung nach Anspruch 1 mit der stereochemischen Konfiguration

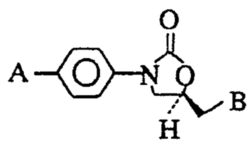

,

worin

A -S(O)$CH_3$, -S-$CH_3$, -S(O)$_2CH_3$, $SO_2NH_2$, -$COCH_3$ oder -CH($CH_3$)$_2$ ist.

**6.** Verbindung nach Anspruch 1 mit der stereochemischen Konfiguration

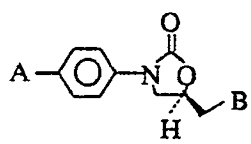

,

worin

B

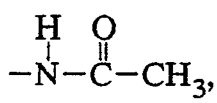, 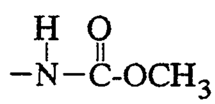 oder 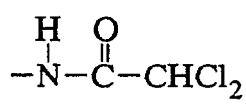

ist.

**7.** Verbindung nach Anspruch 5, worin

B

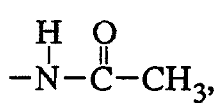, 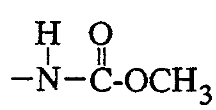 oder 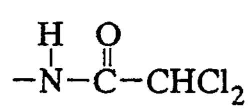

ist.

**8.** Verbindung nach Anspruch 1, ausgewählt aus

(ℓ)-N-[3-[4-(Methylsulfonyl)phenyl]-2-oxooxazolidin-5-ylmethyl]carbaminsäuremethylester,

(ℓ)-N-[3-[4(Methylthio)phenyl]-2-oxooxazolidin-5-ylmethyl]carbaminsäuremethylester,

(ℓ)-N-[3-[4-(Methylsulfonyl)phenyl]-2-oxooxazolidin-5-ylmethyl]formamid,
(ℓ)-N-[3-[4(Methylsulfonyl)phenyl]-2-oxooxazolidin-5-ylmethyl]acetamid,
(ℓ)-N-[3-[4-(Methylthio)phenyl]-2-oxooxazolidin-5-ylmethyl]acetamid,
(ℓ)-N-[3-[4-(Aminosulfonyl)phenyl]-2-oxooxazolidin-5-ylmethyl]acetamid,
(ℓ)-N-[3-[4-(Methylsulfinyl)phenyl]-2-oxooxazolidin-5-ylmethyl]acetamid,
(ℓ)-2,2-Dichloro-N-[3-[4-(methylsulfonyl)phenyl]-2-oxooxazolidin-5-ylmethyl]acetamid,
(ℓ)-N-[3(4-Isopropylphenyl)-2-oxooxazolidin-5-ylmethyl]acetamid und
(ℓ)-N-[3-(4-Acetylphenyl)-2-oxooxazolidin-5-ylmethyl]acetamid.

**9.** Verbindung der Formel

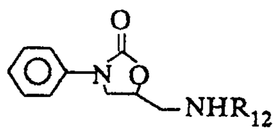

(Ia)

worin
für die ℓ- und Gemische der d- und ℓ-Stereoisomeren der Verbindung
$R_{12}$ H, $C_1$-$C_{10}$-Alkyl oder $C_3$-$C_8$-Cycloalkyl ist.

**10.** Verbindung der Formel

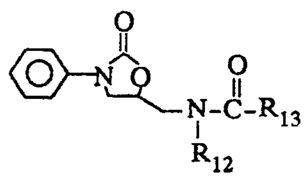

(Ib)

worin
für die ℓ- und Gemische der d- und ℓ-Stereoisomeren der Verbindung

| | |
|---|---|
| $R_{12}$ | H, $C_1$-$C_{10}$-Alkyl oder $C_3$-$C_8$-Cycloalkyl ist, |
| $R_{13}$ | H, $C_1$-$C_4$-Alkyl, das gegebenenfalls mit einem oder mehreren Halogen-Atomen substituiert ist, $C_2$-$C_4$-Alkenyl, $C_3$-$C_4$-Cycloalkyl, Phenyl, $-CH_2OR_{15}$, $-CH(OR_{16})$-$OR_{17}$, $-CH_2S(O)_vR_{14}$, - $OR_{18}$, $-SR_{14}$, $-NR_{19}R_{20}$ oder $C(NH_2)R_{21}R_{22}$ ist; |
| $R_{14}$ | $C_1$-$C_4$-Alkyl ist, das gegebenenfalls mit einem oder mehreren Halogen-Atomen substituiert ist; |
| $R_{15}$ | $C_1$-$C_4$-Alkyl ist, |
| $R_{16}$ und $R_{17}$ | unabhängig voneinander $C_1$-$C_4$-Alkyl sind, $R_{18}$ $C_1$-$C_4$-Alkyl ist, |
| $R_{19}$ und $R_{20}$ | unabhängig voneinander H oder $C_1$-$C_4$-Alkyl sind, |
| $R_{21}$ und $R_{22}$ | unabhängig voneinander H, Phenyl sind; |
| v | 0, 1 oder 2 ist. |

**11.** Pharmazeutische Zusammensetzung, umfassend einen geeigneten pharmazeutischen Träger und eine antibakteriell wirksame Menge wenigstens einer Verbindung der Ansprüche 1 bis 8.

**12.** Verfahren zur Herstellung der Verbindungen nach Anspruch 1, umfassend
(a) das In-Berührung-Bringen einer Verbindung der Formel

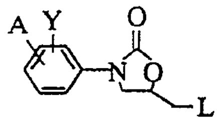

,

in der A und Y die im ursprünglichen Anspruch 1 angegebenen Bedeutungen haben und L irgendeine beliebige austretende Gruppe ist, mit Azid-Ionen zur Herstellung einer Verbindung der Formel

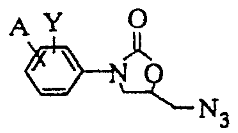

;

(b) gegebenenfalls das Reduzieren des Produkts aus Schritt (a) zur Herstellung einer Verbindung der Formel

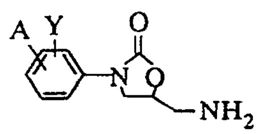

;

(c) gegebenenfalls die Umsetzung des in Schritt (b) erhaltenen Amins mit einem Säurechlorid, -anhydrid oder Sulfonylchlorid oder mit einer Carbonsäure in Gegenwart eines geeigneten Kupplungs-Reagens zur Herstellung einer Verbindung der Formel

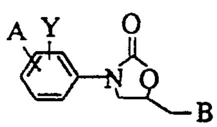

,

in der B

$$NH\overset{\overset{O}{\|}}{C}R_{13} \text{ oder } NHS(O)_uR_{14}$$

ist;

(d) wenn A $SR_1$ ist, gegebenenfalls die Oxidation des Produkts aus Schritt (c) zur Herstellung einer Verbindung der Formel

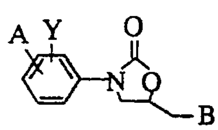

,

in der A $R_1S(O)_n$ ist und n 1 oder 2 ist;

(e) wenn A H ist, gegebenenfalls die Umsetzung des Produkts aus Schritt (c) mit Chlorsulfonsäure und anschließend mit einem Amin, Hydrazin, Hydroxylamin oder Azid-Ion zur Herstellung einer Verbindung der Formel

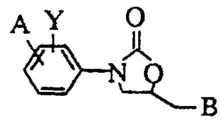

,

in der A $R_1SO_2$ ist, worin $R_1$ $R_{10}R_9N$ oder $N_3$ ist.

**13.** Verfahren zur Herstellung der Verbindungen nach Anspruch 1, umfassend

(a) das In-Berührung-Bringen einer Verbindung der Formel

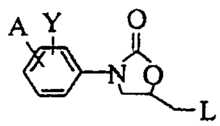

,

in der A und Y die im ursprünglichen Anspruch 1 angegebenen Bedeutungen haben und L irgendeine beliebige austretende Gruppe ist, mit einem primären Amin oder mit Ammoniak zur Herstellung einer Verbindung der Formel

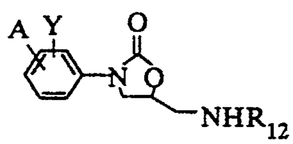

;

(b) das Behandeln des in Schritt (a) erhaltenen Amins gemäß den im vorstehenden beschriebenen Schritten (c), (d) oder (e).

**Patentansprüche für folgenden Vertragsstaat: AT**

**1.** Verfahren zur Herstellung einer Verbindung der Formel

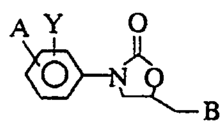

,

(I)

in der für die ℓ- und Gemische der d- und ℓ-Stereoisomeren der Verbindung

A -$NO_2$, -$S(O)_nR_1$, -SH,

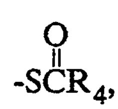

-$COR_5$,

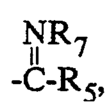

-CN, -$OR_5$, -$NR_5R_6$,

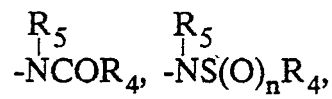

Alkyl mit 1 bis 5 Kohlenstoff-Atomen, das gegebenenfalls mit einem oder mehreren Halogen-Atomen substituiert ist, oder Alkenyl mit 2 bis 5 Kohlenstoff-Atomen ist;

| | |
|---|---|
| $R_1$ | $C_1$-$C_4$-Alkyl, das gegebenenfalls mit einem oder mehreren. Halogen-Atomen substituiert ist, CN, $-NR_9R_{10}$, $-N_3$ ist; |
| $R_4$ | Alkyl mit 1 bis 4 Kohlenstoff-Atomen ist; |
| $R_5$ und $R_6$ | unabhängig voneinander H, Alkyl mit 1 bis 4 Kohlenstoff-Atomen sind; |
| $R_7$ | $-OR_5$ ist; |
| $R_8$ | H oder Alkyl mit 1 bis 4 Kohlenstoff-Atomen ist; |
| $R_9$ | H, $C_1$-$C_4$-Alkyl oder $C_3$-$C_8$-Cycloalkyl ist; |
| $R_{10}$ | H, $-OR_8$ oder $-NR_{11}R_{11a}$ ist; |
| $R_{11}$ und $R_{11a}$ | unabhängig voneinander H oder $C_1$-$C_4$-Alkyl sind; |
| Y | H oder $NO_2$ ist, oder A und Y zusammen genommen $-O(CH_2)_tO-$ sein können; |
| n | 0, 1 oder 2 ist; |
| t | 1, 2 oder 3 ist; |
| B | 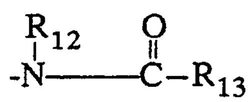 oder $N_3$ ist; |
| $R_{12}$ | H, $C_1$-$C_{10}$-Alkyl oder $C_3$-$C_8$-Cycloalkyl ist; |
| $R_{13}$ | H, $C_1$-$C_4$-Alkyl, das gegebenenfalls mit einem oder mehreren Halogen-Atomen substituiert ist, $C_2$-$C_4$-Alkenyl, $C_3$-$C_4$-Cycloalkyl, Phenyl, $-CH_2OR_{15}$, $-CH(OR_{16})-OR_{17}$, $-CH_2S(O)_vR_{14}$, $-OR_{18}$, $-SR_{14}$, $-CH_2N_3$, $-NR_{19}R_{20}$ oder $C(NH_2)R_{21}R_{22}$ ist; |
| $R_{14}$ | $C_1$-$C_4$-Alkyl ist, das gegebenenfalls mit einem oder mehreren Halogen-Atomen substituiert ist; |
| $R_{15}$ | $C_1$-$C_4$-Alkyl ist, |
| $R_{16}$ und $R_{17}$ | unabhängig voneinander $C_1$-$C_4$-Alkyl sind, |
| $R_{18}$ | $C_1$-$C_4$-Alkyl ist, |
| $R_{19}$ und $R_{20}$ | unabhängig voneinander H oder $C_1$-$C_4$-Alkyl sind, |
| $R_{21}$ und $R_{22}$ | unabhängig voneinander H, Phenyl sind; |
| u | 1 oder 2 ist; |
| v | 0, 1 oder 2 ist; |

oder eines pharmazeutisch geeigneten Salzes derselben, mit der Maßgabe, daß

1) wenn A $CH_3S$- ist, dann B nicht

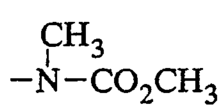

ist;

2) wenn A $CH_3SO_2$- ist, dann B nicht

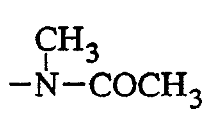 oder 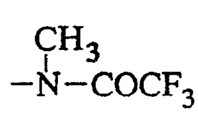

ist;

3) wenn A $H_2NSO_2$- ist und B

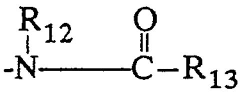

ist, dann $R_{12}$ H ist;
4) wenn A -CN ist, dann B nicht $-N_3$ ist;
5) wenn A $(CH_3)_2CH$ ist, dann B nicht $NHCOCH_2Cl$ ist, umfassend
(a) das In-Berührung-Bringen einer Verbindung der Formel

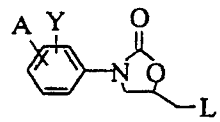

,

in der A und Y die im ursprünglichen Anspruch 1 angegebenen Bedeutungen haben und L irgendeine beliebige austretende Gruppe ist, mit Azid-Ionen zur Herstellung einer Verbindung der Formel

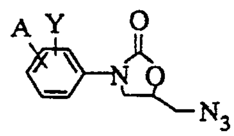

;

(b) gegebenenfalls das Reduzieren des Produkts aus Schritt (a) zur Herstellung einer Verbindung der Formel

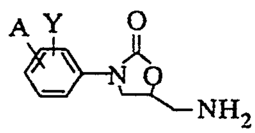

;

(c) gegebenenfalls die Umsetzung des in Schritt (b) erhaltenen Amins mit einem Säurechlorid, -anhydrid oder Sulfonylchlorid oder mit einer Carbonsäure in Gegenwart eines geeigneten Kupplungs-Reagens zur Herstellung einer Verbindung der Formel

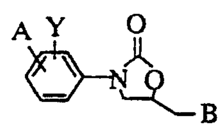

,

in der B

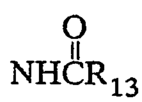 oder $NHS(O)_uR_{14}$

ist;
(d) wenn A $SR_1$ ist, gegebenenfalls die Oxidation des Produkts aus Schritt (c) zur Herstellung einer Verbindung der Formel

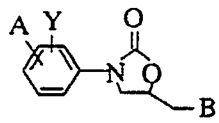

,

in der A $R_1S(O)_n$ ist und n 1 oder 2 ist;

(e) wenn A H ist, gegebenenfalls die Umsetzung des Produkts aus Schritt (c) mit Chlorsulfonsäure und anschließend mit einem Amin, Hydrazin, Hydroxylamin oder Azid-Ion zur Herstellung einer Verbindung der Formel

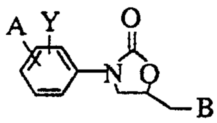

,

in der A $R_1SO_2$ ist, worin $R_1$ $R_{10}R_9N$ oder $N_3$ ist.

2. Verfahren nach Anspruch 1, worin für die ℓ- und Gemische der d- und ℓ-Stereoisomeren der Verbindung

| | |
|---|---|
| Y | H ist; |
| A, | substituiert in der para-Stellung, $-NO_2$, $S(O)_nR_1$ ist; |
| $R_1$ | $C_1$-$C_4$-Alkyl, das gegebenenfalls mit einem oder mehreren Halogen-Atomen substituiert ist, $-NR_9R_{10}$, $-N_3$ ist; |
| $R_9$ | H, $C_1$-$C_4$-Alkyl oder $C_3$-$C_8$-Cycloalkyl ist; |
| $R_{10}$ | H, $-OR_8$ oder $NR_{11}R_{11a}$ ist; |
| $R_8$ | H oder $C_1$-$C_4$-Alkyl ist; |
| $R_{11}$ und $R_{11a}$ | unabhängig voneinander H oder $C_1$-$C_4$-Alkyl sind; |
| n | 0, 1 oder 2 ist; |
| B | 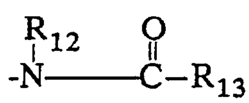 oder $N_3$ ist; |
| $R_{12}$ | H, $C_1$-$C_{10}$-Alkyl oder $C_3$-$C_8$-Cycloalkyl ist; |
| $R_{13}$ | H, $C_1$-$C_4$-Alkyl, das gegebenenfalls mit einem oder mehreren Halogen-Atomen substituiert ist, $C_2$-$C_4$-Alkenyl, $C_3$-$C_4$-Cycloalkyl, Phenyl, $-CH_2OR_{15}$, $-CH(OR_{16})$-$OR_{17}$, $-CH_2S(O)_vR_{14}$, $-OR_{18}$, $-SR_{14}$ oder $-NR_{19}R_{20}$ ist; |
| $R_{14}$ | $C_1$-$C_4$-Alkyl ist, das gegebenenfalls mit einem oder mehreren Halogen-Atomen substituiert ist; |
| $R_{15}$ | $C_1$-$C_4$-Alkyl ist, |
| $R_{16}$ und $R_{17}$ | unabhängig voneinander $C_1$-$C_4$-Alkyl sind, |
| $R_{18}$ | $C_1$-$C_4$-Alkyl ist, |
| $R_{19}$ | H oder $C_1$-$C_4$-Alkyl ist; |
| $R_{20}$ | H oder $C_1$-$C_4$-Alkyl ist; |
| u | 1 oder 2 ist; |
| v | 0, 1 oder 2 ist; |

oder eines pharmazeutisch geeigneten Salzes derselben, mit der Maßgabe, daß

1) wenn A $CH_3S$- ist, dann B nicht

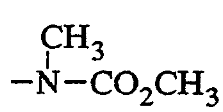

ist;
2) wenn A $CH_3SO_2$- ist, dann B nicht

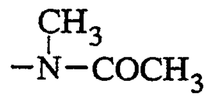 oder 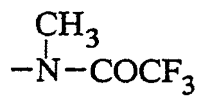

ist;
3) wenn A $H_2NSO_2$- ist und B

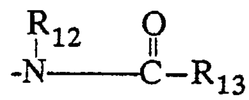

ist, dann $R_{12}$ H ist.

**3.** Verfahren nach Anspruch 1, worin
Y H ist;
A, substituiert in der para-Stellung, -$S(O)_nR_1$, -$NO_2$,

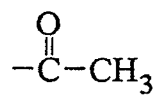

oder -$CH(CH_3)_2$ ist;
R $C_1$-$C_2$-Alkyl, das gegebenenfalls mit einem oder mehreren Halogen-Atomen substituiert ist, oder -$NR_9R_{10}$ ist;
n 0, 1 oder 2 ist, wenn $R_1$ Alkyl oder substituiertes Alkyl ist.

**4.** Verfahren nach Anspruch 1, worin B

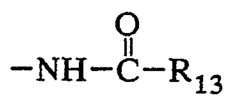

ist;
$R_{13}$ H, $CH_3$, $OR_{18}$, $CHCl_2$, $CH_2Cl$ oder $CH_2OR_{15}$ ist;
$R_{15}$ $C_1$-$C_4$-Alkyl ist; und
$R_{18}$ $C_1$-$C_4$-Alkyl ist.

**5.** Verfahren nach Anspruch 1 zur Herstellung einer Verbindung mit der stereochemischen Konfiguration

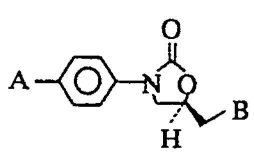 ,

worin
A -$S(O)CH_3$, -$S$-$CH_3$, -$S(O)_2CH_3$, $SO_2NH_2$, -$COCH_3$ oder -$CH(CH_3)_2$ ist.

**6.** Verfahren nach Anspruch 1 zur Herstellung einer Verbindung mit der stereochemischen Konfiguration

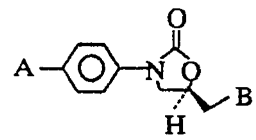,

worin
B

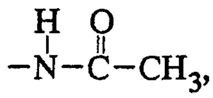, 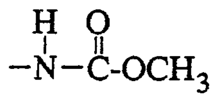 oder 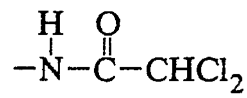

ist.

7. Verfahren nach Anspruch 5, worin
B

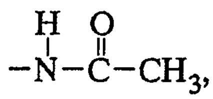, 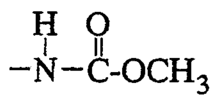 oder 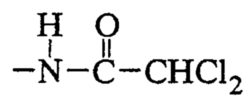

ist.

8. Verfahren nach Anspruch 1, worin die Verbindungen ausgewählt sind aus
(ℓ)-N-[3-[4(Methylsulfonyl)phenyl]-2-oxooxazolidin-5-ylmethyl]carbaminsäuremethylester,
(ℓ)-N-[3-[4-(Methylthio)phenyl]-2-oxooxazolidin-5-ylmethyl]carbaminsäuremethylester,
(ℓ)-N-[3-[4-(Methylsulfonyl)phenyl]-2-oxooxazolidin-5-ylmethyl]formamid,
(ℓ)-N-[3-[4-(Methylsulfonyl)phenyl]-2-oxooxazolidin-5-ylmethyl]acetamid,
(ℓ)-N-[3-[4-(Methylthio)phenyl]-2-oxooxazolidin-5-ylmethyl]acetamid,
(ℓ)-N-[3-[4-(Aminosulfonyl)phenyl]-2-oxooxazolidin-5-ylmethyl]acetamid,
(ℓ)-N-[3-[4(Methylsulfinyl)phenyl]-2-oxooxazolidin-5-ylmethyl]acetamid,
(ℓ)-2,2-Dichloro-N-[3-[4-(methylsulfonyl)phenyl]-2-oxooxazolidin-5-ylmethyl]acetamid,
(ℓ)-N-[3-(4-Isopropylphenyl)-2-oxooxazolidin-5-ylmethyl]acetamid und
(ℓ)-N[3-(4-Acetylphenyl)-2-oxooxazolidin-5-ylmethyl]acetamid.

9. Verfahren zur Herstellung einer Verbindung der Formel

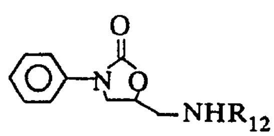

(Ia)

worin
für die ℓ- und Gemische der d- und ℓ-Stereoisomeren der Verbindung
$R_{12}$ H, $C_1$-$C_{10}$-Alkyl oder $C_3$-$C_8$-Cycloalkyl ist,
umfassend
das In-Berührung-Bringen einer Verbindung der Formel

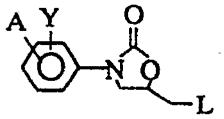,

in der A und Y H sind und L irgendeine beliebige austretende Gruppe ist, mit einem primären Amin oder mit Ammoniak zur Herstellung einer Verbindung der Formel

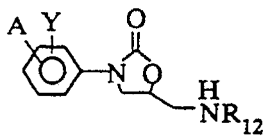

,

in der A und Y H sind.

**10.** Verfahren zur Herstellung einer Verbindung der Formel

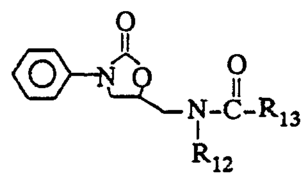

(Ib)

worin
für die ℓ- und Gemische der d- und ℓ-Stereoisomeren der Verbindung

$R_{12}$ H, $C_1$-$C_{10}$-Alkyl oder $C_3$-$C_8$-Cycloalkyl ist,
$R_{13}$ H, $C_1$-$C_4$-Alkyl, das gegebenenfalls mit einem oder mehreren Halogen-Atomen substituiert ist, $C_2$-$C_4$-Alkenyl, $C_3$-$C_4$-Cycloalkyl, Phenyl, $-CH_2OR_{15}$, $-CH(OR_{16})$-$OR_{17}$, $-CH_2S(O)_vR_{14}$, - $OR_{18}$, $-SR_{14}$, $-NR_{19}R_{20}$ oder $C(NH_2)R_{21}R_{22}$ ist;
$R_{14}$ $C_1$-$C_4$-Alkyl ist, das gegebenenfalls mit einem oder mehreren Halogen-Atomen substituiert ist;
$R_{15}$ $C_1$-$C_4$-Alkyl ist,
$R_{16}$ und $R_{17}$ unabhängig voneinander $C_1$-$C_4$-Alkyl sind,
$R_{18}$ $C_1$-$C_4$-Alkyl ist,
$R_{19}$ und $R_{20}$ unabhängig voneinander H oder $C_1$-$C_4$-Alkyl sind,
$R_{21}$ und $R_{22}$ unabhängig voneinander H, Phenyl sind;
v 0, 1 oder 2 ist,

umfassend das in-Berührung-Bringen einer Verbindung der Formel

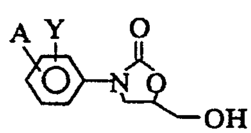

mit einem geeigneten Sulfonylhalogenid oder Sulfonsäureanhydrid zur Herstellung einer Verbindung der Formel

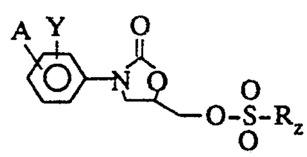

und Umsetzung dieser Verbindung mit dem geeigneten Amid und einem Katalsyator, wobei A und Y in beiden Formeln H sind.

**11.** Verfahren zur Herstellung der Verbindungen nach Anspruch 1, umfassend
(a) das In-Berührung-Bringen einer Verbindung der Formel

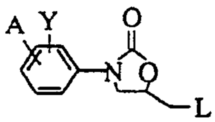

,

in der A und Y die im ursprünglichen Anspruch 1 angegebenen Bedeutungen haben und L irgendeine geeignete austretende Gruppe ist, mit einem primären Amin oder mit Ammoniak zur Herstellung einer Verbindung der Formel

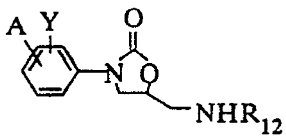

;

(b) das Behandeln des in Schritt (a) erhaltenen Amins gemäß den im vorstehenden beschriebenen Schritten (c), (d) oder (e).

**12.** Pharmazeutische Zusammensetzung, umfassend einen geeigneten pharmazeutischen Träger und eine antibakteriell wirksame Menge wenigstens einer Verbindung der Ansprüche 1 bis 8.